(19) Europäisches Patentamt
European Patent Office
Office européen des brevets

(11) EP 2 041 142 B1

(12) EUROPÄISCHE PATENTSCHRIFT

(45) Veröffentlichungstag und Bekanntmachung des Hinweises auf die Patenterteilung:
**09.10.2013 Patentblatt 2013/41**

(21) Anmeldenummer: **07786123.5**

(22) Anmeldetag: **17.07.2007**

(51) Int Cl.:
***C07D 491/14*** *(2006.01)* ***A61K 31/438*** *(2006.01)*
***A61P 25/04*** *(2006.01)*

(86) Internationale Anmeldenummer:
**PCT/EP2007/006326**

(87) Internationale Veröffentlichungsnummer:
**WO 2008/009416 (24.01.2008 Gazette 2008/04)**

(54) **SPIROCYCLISCHE AZAINDOL-DERIVATE**
SPIROCYCLIC AZAINDOLE DERIVATIVES
DÉRIVÉS AZAINDOLIQUES SPIROCYCLIQUES

(84) Benannte Vertragsstaaten:
**AT BE BG CH CY CZ DE DK EE ES FI FR GB GR HU IE IS IT LI LT LU LV MC MT NL PL PT RO SE SI SK TR**
Benannte Erstreckungsstaaten:
**HR**

(30) Priorität: **18.07.2006 DE 102006033114**

(43) Veröffentlichungstag der Anmeldung:
**01.04.2009 Patentblatt 2009/14**

(73) Patentinhaber: **Grünenthal GmbH**
**52078 Aachen (DE)**

(72) Erfinder:
- **ZEMOLKA, Saskia**
  **52066 Aachen (DE)**
- **SCHUNK, Stefan**
  **52080 Aachen (DE)**
- **BERGRATH, Ellen**
  **52146 Würselen (DE)**
- **KÖGEL, Babette-Yvonne**
  **52379 Langerwehe-Hamich (DE)**
- **ENGLBERGER, Werner**
  **52223 Stolberg (DE)**
- **LINZ, Klaus**
  **53343 Wachtberg (DE)**
- **SCHICK, Hans**
  **13086 Berlin (DE)**

(74) Vertreter: **Bülle, Jan et al**
**Kutzenberger Wolff & Partner**
**Theodor-Heuss-Ring 23**
**50668 Köln (DE)**

(56) Entgegenhaltungen:
**EP-A- 1 142 587**
**WO-A-2004/043967**
**WO-A-2005/063769**
**WO-A-2005/066183**
**WO-A-2006/108565**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft substituierte spirocyclische Azaindol-Derivate, Verfahren zu deren Herstellung, Arzneimittel enthaltend diese Verbindungen und die Verwendung von substituierten spirocyclische Azaindol-Derivaten zur Herstellung von Arzneimitteln.

Die Behandlung chronischer und nichtchronischer Schmerzzustände hat in der Medizin eine große Bedeutung. Es besteht ein weltweiter Bedarf an gut wirksamen Schmerztherapien. Der dringende Handlungsbedarf für eine patientengerechte und zielorientierte Behandlung chronischer und nicht chronischer Schmerzzustände, wobei hierunter die erfolgreiche und zufriedenstellende Schmerzbehandlung für den Patienten zu verstehen ist, dokumentiert sich in der großen Anzahl von wissenschaftlichen Arbeiten, die auf dem Gebiet der angewandten Analgetik bzw. der Grundlagenforschung zur Nociception in letzter Zeit erschienen sind.

Klassische μ- Opioide wie Morphin sind bei der Therapie starker bis stärkster Schmerzen gut wirksam und von größter Bedeutung für die Schmerztherapie. Es kann jedoch von Vorteil sein, wenn neben dem μ- Opioid- Rezeptor auch andere Opioid- Rezeptoren, insbesondere der ORL- 1- Rezeptor, beeinflusst werden, da die reinen μ- Opioide auch unerwünschte Nebenwirkungen wie Obstipation und Atemdepression aufweisen, aber auch zu Abhängigkeit führen können. Auch die Opioid- Rezeptoren δ, κ und ORL- 1 sind am Schmerzgeschehen beteiligt (Opioids: Introduction, S. 127- 150, Further Opioid Receptors, 455- 476 in: Analgesics- From Chemistry and Pharmacology to Clinical Application, Wiley VCH, 2002) .

Der ORL1- Rezeptor ist außerdem noch an der Regulation weiterer physiologischer und pathophysiologischer Prozesse beteiligt. Hierzu gehören unter anderem Lernen und Gedächtnisbildung (Manabe et al., Nature, 394, 1997, S. 577- 581), Hörvermögen (Nishi et al., EMBO J., 16, 1997, S. 1858- 1864) sowie zahlreiche weitere Prozesse. In einem Übersichtsartikel von Calo et al. (Br.J. Pharmacol., 129, 2000, 1261- 1283) wird ein Überblick über die Indikationen oder biologischen Vorgänge gegeben, in denen der ORL1- Rezeptor eine Rolle spielt oder mit hoher Wahrscheinlichkeit spielen könnte. Genannt werden u.a.: Analgesie, Stimulation und Regulation der Nahrungsaufnahme, Einfluß auf μ-Agonisten wie Morphin, Behandlung von Entzugserscheinungen, Reduzierung des Suchtpotentials von Opioiden, Anxiolyse, Modulation der Bewegungsaktivität, Gedächtnis- Störungen, Epilepsie; Modulation der Neurotransmitter- Ausschüttung, insbesondere von Glutamat, Serotonin und Dopamin, und damit neurodegenerative Erkrankungen; Beeinflußung des cardiovaskulären Systems, Auslösung einer Erektion, Diurese, Antinatriurese, Elektrolyt- Haushalt, arterieller Blutdruck, Wasserspeicher- Krankheiten, intestinale Motilität (Diarrhoe), relaxierende Effekte auf die Atemwege, Mikturations Reflex (Harninkontinenz) . Weiter wird die Verwendung von Agonisten und Antagonisten als Anoretika, Analgetika (auch in Coadministration mit Opioiden) oder Nootropika diskutiert.

Aus dem Stand der Technik (WO 04043967) sind strukturell verwandte Verbindungen bekannt, die eine hohe Affinität zum ORL-1-Rezeptor und zum μ-Opioid-Rezeptor besitzen. Bei diesen Verbindungen ist jedoch der aromatische Heterocyclus ein Indolring, bei dem kein Kohlenstoff-Atom durch ein Stickstoff-Atom ersetzt sein kann.

Aufgabe der vorliegenden Erfindung war es, weitere Arzneimittel zur Verfügung zu stellen, die auf das Opioid-Rezeptor-System wirken und damit für Arzneimittel insbesondere zur Behandlung der verschiedenen mit diesem System in Verbindung stehenden Krankeiten bzw. zum Einsatz in den damit zusammenhängenden Indikationen geeignet sind.

Gegenstand der Erfindung sind daher substituierte spirocyclische Azaindol-Derivate der allgemeinen Formel I,

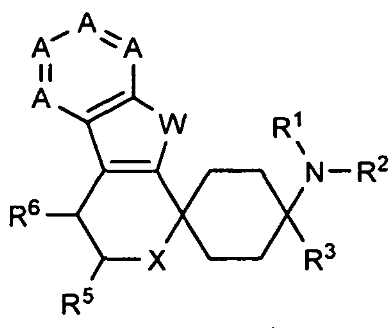

I

worin

A für N oder $CR^{7-10}$ steht, wobei A mindestens einmal und höchstens zweimal für N steht

W für $NR^4$ steht

X für $NR^{17}$, O oder S steht

$R^1$ und $R^2$, unabhängig voneinander für H; $C_{1-5}$- Alkyl jeweils gesättigt oder ungesättigt, verzweigt oder unverzweigt, einfach oder mehrfach substituiert oder unsubstituiert; $C_{3-8}$- Cycloalkyl, jeweils einfach oder mehrfach substituiert oder unsubstituiert; oder über $C_{1-3}$- Alkyl gebundenes Aryl, $C_{3-8}$- Cycloalkyl oder Heteroaryl, jeweils einfach oder mehrfach substituiert oder unsubstituiert, stehen;

oder die Reste $R^1$ und $R^2$ zusammen für $CH_2CH_2OCH_2CH_2$, $CH_2CH_2NR^{11}CH_2CH_2$ oder $(CH_2)_{3-6}$ stehen,

wobei $R^{11}$ H; $C_{1-5}$- Alkyl, jeweils gesättigt oder ungesättigt, verzweigt oder unverzweigt, einfach oder mehrfach substituiert oder unsubstituiert; $C_{3-8}$- Cycloalkyl, jeweils einfach oder mehrfach substituiert oder unsubstituiert; Aryl-, oder Heteroaryl, jeweils einfach oder mehrfach substituiert oder unsubstituiert; oder über $C_{1-3}$- Alkyl gebundenes Aryl, $C_{3-8}$- Cycloalkyl oder Heteroaryl, jeweils einfach oder mehrfach substituiert oder unsubstituiert; C (O) Phenyl, C (O) Heteroaryl, C (O) $C_{1-5}$- Alkyl, jeweils substituiert oder unsubstituiert, bedeutet;

$R^3$ für $C_{1-8}$- Alkyl, jeweils gesättigt oder ungesättigt, verzweigt oder unverzweigt, einfach oder mehrfach substituiert oder unsubstituiert; $C_{3-8}$- Cycloalkyl, jeweils einfach oder mehrfach substituiert oder unsubstituiert; Aryl oder Heteroaryl, jeweils unsubstituiert oder einfach oder mehrfach substituiert; über $C_{1-3}$- Alkyl- Gruppe gebundenes Aryl, Heteroaryl oder $C_{3-8}$- Cycloalkyl, jeweils unsubstituiert oder einfach oder mehrfach substituiert, steht;

$R^4$ für H; $C_{1-5}$- Alkyl, gesättigt oder ungesättigt, verzweigt oder unverzweigt, unsubstituiert oder einfach oder mehrfach substituiert; Aryl, oder Heteroaryl, jeweils substituiert oder unsubstituiert; über eine $C_{1-3}$- Alkyl- Gruppe gebundenes Aryl, Heteroaryl oder Cycloalkyl, jeweils einfach oder mehrfach substituiert oder unsubstituiert; $COR^{12}$ ; $SO_2R^{12}$ steht,

wobei $R^{12}$ H; $C_{1-5}$- Alkyl, jeweils gesättigt oder ungesättigt, verzweigt oder unverzweigt, einfach oder mehrfach substituiert oder unsubstituiert; $C_{3-8}$- Cycloalkyl, jeweils gesättigt oder ungesättigt, einfach oder mehrfach substituiert oder unsubstituiert; Aryl-, oder Heteroaryl, jeweils einfach oder mehrfach substituiert oder unsubstituiert; oder über $C_{1-3}$- Alkyl gebundenes Aryl, $C_{3-8}$- Cycloalkyl oder Heteroaryl, jeweils einfach oder mehrfach substituiert oder unsubstituiert; $OR^{13}$; $NR^{14}R^{15}$ bedeutet;

$R^5$ für =O; H; $COOR^{13}$, $CONR^{13}$, $OR^{13}$; $C_{1-5}$- Alkyl, gesättigt oder ungesättigt, verzweigt oder unverzweigt, unsubstituiert oder einfach oder mehrfach substituiert; $C_{3-8}$- Cycloalkyl, gesättigt oder ungesättigt, unsubstituiert oder einfach oder mehrfach substituiert; Aryl-, oder Heteroaryl, unsubstituiert oder einfach oder mehrfach substituiert; oder über $C_{1-3}$- Alkyl gebundenes Aryl, $C_{3-8}$- Cycloalkyl oder Heteroaryl, unsubstituiert oder einfach oder mehrfach substituiert, steht;

$R^6$ für H; F, Cl, $NO_2$, $CF_3$, $OR^{13}$, $SR^{13}$, $SO_2R^{13}$, $SO_2OR^{13}$, CN, $COOR^{13}$, $NR^{14}R^{15}$; $C_{1-5}$- Alkyl, gesättigt oder ungesättigt, verzweigt oder unverzweigt, unsubstituiert oder einfach oder mehrfach substituiert; $C_{3-8}$- Cycloalkyl, gesättigt oder ungesättigt, unsubstituiert oder einfach oder mehrfach substituiert; Aryl-, oder Heteroaryl, unsubstituiert oder einfach oder mehrfach substituiert; oder über $C_{1-3}$- Alkyl gebundenes Aryl, $C_{3-8}$- Cycloalkyl oder Heteroaryl, unsubstituiert oder einfach oder mehrfach substituiert, steht;

oder $R^5$ und $R^6$ gemeinsam $(CH_2)$n mit n = 2, 3, 4, 5 oder 6 bedeuten, wobei einzelne Wasserstoffatome auch durch F, Cl, Br, I, $NO_2$, $CF_3$, $OR^{13}$, CN oder $C_{1-5}$-Alkyl ersetzt sein können;

$R^7$, $R^8$, $R^9$ und $R^{10}$ unabhängig voneinander für
H, F, Cl, Br, I, $NO_2$, $CF_3$, $OR^{13}$, $SR^{13}$, $SO_2R^{13}$, $SO_2OR^{13}$, CN, $COOR^{13}$, $NR^{14}R^{15}$; $C_{1-5}$- Alkyl, $C_{3-8}$- Cycloalkyl, unsubstituiert oder einfach oder mehrfach substituiert; Aryl-, oder Heteroaryl, unsubstituiert oder einfach oder mehrfach substituiert; oder über $C_{1-3}$- Alkyl gebundenes Aryl, $C_{3-8}$- Cycloalkyl oder Heteroaryl, unsubstituiert oder einfach oder mehrfach substituiert, stehen;

wobei $R^{13}$ H; $C_{1-5}$- Alkyl jeweils gesättigt oder ungesättigt, verzweigt oder unverzweigt, unsubstituiert oder einfach oder mehrfach substituiert; $C_{3-8}$- Cycloalkyl, jeweils gesättigt oder ungesättigt, unsubstituiert oder einfach oder mehrfach substituiert; Aryl-, oder Heteroaryl, unsubstituiert oder einfach oder mehrfach substituiert; oder über $C_{1-3}$- Alkyl gebundenes Aryl, $C_{3-8}$- Cycloalkyl oder Heteroaryl, unsubstituiert oder einfach oder mehrfach substituiert, bedeutet;

$R^{14}$ und $R^{15}$ unabhängig voneinander H; $C_{1-5}$- Alkyl, jeweils gesättigt oder ungesättigt, verzweigt oder unverzweigt, unsubstituiert oder einfach oder mehrfach substituiert; oder $C_{3-8}$- Cycloalkyl, jeweils gesättigt oder ungesättigt, unsubstituiert oder einfach oder mehrfach substituiert; Aryl-, oder Heteroaryl, unsubstituiert oder einfach oder mehrfach substituiert; oder über $C_{1-3}$- Alkyl gebundenes Aryl, $C_{3-8}$- Cycloalkyl oder Heteroaryl, unsubstituiert oder einfach oder mehrfach substituiert, bedeuten;

oder $R^{14}$ und $R^{15}$ zusammen $CH_2CH_2OCH_2CH_2$, $CH_2CH_2NR^{16}CH_2CH_2$ oder $(CH_2)_{3-6}$ bilden,

wobei $R^{16}$ H; $C_{1-5}$-Alkyl gesättigt oder ungesättigt, verzweigt oder unverzweigt, unsubstituiert oder einfach oder mehrfach substituiert, bedeutet;

$R^{17}$ für H; $C_{1-8}$- Alkyl, gesättigt oder ungesättigt, verzweigt oder unverzweigt; $COR^{12}$ oder $SO_2R^{12}$ steht

in Form des Razemats; der Enantiomere, Diastereomere, Mischungen der Enantiomere oder Diastereomere oder eines einzelnen Enantiomers oder Diastereomers; der Basen und/oder Salze physiologisch verträglicher Säuren.

Die erfindungsgemäßen Verbindungen zeigen gute Bindung an den μ-Opioid-Rezeptor und den ORL-1-Rezeptor.

Die Ausdrücke "$C_{1-8}$- Alkyl", "$C_{1-3}$- Alkyl", " und "$C_{1-5}$- Alkyl" umfassen im Sinne dieser Erfindung acyclische gesättigte oder ungesättigte Kohlenwasserstoffreste, die verzweigt- oder geradkettig sowie unsubstituiert oder ein- oder mehrfach substituiert sein können, mit 1 bis 8 bzw. 1 bis 3 C- Atomen bzw. 1- 5 C- Atomen, d.h. $C_{1-8}$- Alkanyle, $C_{2-8}$- Alkenyle und $C_{2-8}$- Alkinyle bzw. $C_{1-3}$- Alkanyle, $C_{2-3}$- Alkenyle und $C_{2-3}$- Alkinyle bzw $C_{1-5}$- Alkanyle, $C_{2-5}$- Alkenyle und $C_{2-5}$- Alkinyle. Dabei weisen Alkenyle mindestens eine C- C- Doppelbindung und Alkinyle mindestens eine C- C- Dreifachbindung auf. Vorteilhaft ist Alkyl aus der Gruppe ausgewählt, die Methyl, Ethyl, n- Propyl, 2- Propyl, n- Butyl, iso-Butyl, sec.- Butyl, tert.- Butyl, n- Pentyl, isoPentyl, neo- Pentyl, n- Hexyl, 2- Hexyl, n- Heptyl, n- Octyl, 1, 1, 3, 3- Tetramethylbutyl; Ethylenyl (Vinyl), Ethinyl, Propenyl (- $CH_2CH=CH_2$, -$CH=CH$- $CH_3$, -C (=$CH_2$)- $CH_3$), Propinyl (- CH- C≡CH, -C≡C- $CH_3$), Butenyl, Butinyl, Pentenyl, Pentinyl, Hexenyl, Hexinyl, Heptenyl, Heptinyl, Octenyl und Octinyl umfasst. Besonders vorteihaft sind Methyl, Ethyl, n- Propyl, n- Butyl, sec- Butyl, iso- Butyl.

Der Ausdruck "Cycloalkyl" oder "$C_{3-8}$- Cycloalkyl" bedeutet für die Zwecke dieser Erfindung cyclische Kohlenwasserstoffe mit 3, 4, 5, 6, 7 oder 8 Kohlenstoffatomen, wobei die Kohlenwasserstoffe gesättigt oder ungesättigt (aber nicht aromatisch), unsubstituiert oder ein- oder mehrfach substituiert sein können. In Bezug auf Cycloalkyl umfasst der Begriff auch gesättigte oder ungesättigte (aber nicht aromatische) Cycloalkyle, in denen ein oder zwei Kohlenstoffatome durch ein Heteroatom S, N oder O ersetzt sind. Vorteilhaft ist $C_{3-8}$- Cycloalkyl aus der Gruppe, die Cyclopropyl, Cyclobutyl, Cyclopentyl, Cyclohexyl, Cycloheptyl, Cyclooctyl, Cyclopentenyl, Cyclohexenyl, Cycloheptenyl und Cyclooctenyl, aber auch Tetrahydropyranyl, Dioxanyl, Dioxolanyl, Morpholinyl, Piperidinyl, Piperazinyl, Pyrazolinonyl und Pyrrolidinyl enthält. Besonders bevorzugt ist Cyclopropyl, Cyclobutyl, Cyclopentyl und Cyclohexyl.

Der Ausdruck "Aryl" bedeutet im Sinne dieser Erfindung aromatische Kohlenwasserstoffe, u.a. Phenyle und Naphthyle. Die Aryl- Reste können auch mit weiteren gesättigten, (partiell) ungesättigten oder aromatischen Ringsystemen kondensiert sein, sodass der Arylrest ein aromatisches Ringsystem von höchstens 20 C- Atomen bildet. Jeder dieser $C_{6-20}$- Aryl- Reste kann unsubstituiert oder einfach oder mehrfach substituiert vorliegen, wobei die ArylSubstituenten gleich oder verschieden und in jeder beliebigen und möglichen Position des Aryls sein können. Vorteilhafterweise ist Aryl aus der Gruppe ausgewählt, die Phenyl, 1- Naphthyl, 2- Naphthyl, welche jeweils unsubstituiert oder ein- oder mehrfach substituiert sein können, enthält. Besonders vorteilhaft ist der Phenyl- Rest.

Der Ausdruch Heteroaryl" steht für einen 5-, 6- oder 7- gliedrigen cyclischen aromatischen Rest, der mindestens 1, ggf. auch 2, 3, 4 oder 5 Heteroatome, enthält, wobei die Heteroatome gleich oder verschieden sind und der Heterocyclus unsubstituiert oder ein- oder mehrfach substituiert sein kann; im Falle der Substitution am Heterocyclus können die Substituenten gleich oder verschieden sein und in jeder beliebigen und möglichen Position des Heteroaryls sein. Der Heterocyclus kann auch Teil eines bicyclischen oder polycyclischen Systems mit insgesamt bis zu 20 Ringgliedern sein. Bevorzugte Heteroatome sind Stickstoff, Sauerstoff und Schwefel. Es ist bevorzugt, daß der Heteroaryl- Rest ausgewählt ist aus der Gruppe, die Pyrrolyl, Indolyl, Furyl (Furanyl), Benzofuranyl, Thienyl (Thiophenyl), Benzothienyl, Benzothiadiazolyl, Benzothiazolyl, Benzotriazolyl, Benzodioxolanyl, Benzodioxanyl, Phtalazinyl, Pyrazolyl, Imidazolyl, Thiazolyl, Oxazolyl, Isoxazoyl, Pyridyl, Pyridazinyl, Pyrimidinyl, Pyrazinyl, Pyranyl, Indazolyl, Purinyl, Indolizinyl, Chinolinyl, Isochinolinyl, Chinazolinyl, Carbazolyl, Phenazinyl, Phenothiazinyl, Triazolyl oder Oxadiazolyl enthält, wobei die Bindung an die Verbindungen der allgemeinen Struktur I über jedes beliebige und mögliche Ringglied des Heteroaryl- Restes erfolgen kann. Bevorzugt sind Pyridyl, Furyl, Thienyl, Indolyl, Benzothienyl, Pyrrolyl, Triazolyl und Isoxazolyl, besonders bevorzugt Pyridyl, Thienyl, Benzothienyl und Triazolyl.

Der Ausdruck "über $C_{1-3}$- Alkyl gebundenes Aryl oder Heteroaryl" bedeuten für die Zwecke der vorliegenden

Erfindung, daß $C_{1-3}$- Alkyl und Aryl bzw. Heteroaryl die oben definierten Bedeutungen haben und der Aryl- bzw. Heteroaryl- Rest über eine $C_{1-3}$- Alkyl- Gruppe an die Verbindung der allgemeinen Struktur I gebunden ist. Besonders vorteilhaft im Sinne dieser Erfindung sind Benzyl und Phenethyl.

Im Zusammenhang mit "Alkyl" oder Cycloalkyl" versteht man unter dem Begriff "substituiert" im Sinne dieser Erfindung die Substitution eines Wasserstoffrestes durch F, Cl, Br, I, -CN, $NH_2$, NH- $C_{1-6}$- Alkyl, NH- $C_{1-6}$- Alkyl- OH, N ($C_{1-6}$- Alkyl)$_2$, N ($C_{1-6}$- Alkyl- OH)$_2$, $NO_2$, SH, S- $C_{1-6}$- Alkyl, S- Benzyl, $OCF_3$, O- $C_{1-6}$- Alkyl, OH, O- $C_{1-6}$- Alkyl- OH, =O, $C_{1-6}$- Alkyl, Benzyl, O- Benzyl, O- Phenyl, C (=O) $C_{1-6}$- Alkyl, $CO_2H$, $CO_2$- $C_{1-6}$- Alkyl, wobei unter mehrfach substituierten Resten solche Reste zu verstehen sind, die entweder an verschiedenen oder an gleichen Atomen mehrfach, z.B. zwei- oder dreifach, substituiert sind, beispielsweise dreifach am gleichen C- Atom wie im Falle von $CF_3$ oder- $CH_2CF_3$ oder an verschiedenen Stellen wie im Falle von- CH (OH)- CH=CH- $CHCl_2$. Die Mehrfachsubstitution kann mit dem gleichen oder mit verschiedenen Substituenten erfolgen. Bevorzugt für die Zwecke der vorliegenden Erfindung bedeutet " einfach oder mehrfach substituiert" im Zusammenhang mit Alkyl die Substitution mit $COOCH_3$, $OCH_3$, OH, $COOC_2H_5$, F oder Cl.

In Bezug auf "Aryl" und "Heteroaryl" versteht man im Sinne dieser Erfindung unter "ein- oder mehrfach substituiert" die ein- oder mehrfache, z.B. zwei-, drei- oder vierfache, Substitution eines oder mehrerer Wasserstoffatome des Ringsystems durch F, Cl, Br, I, CN, $NH_2$, NH- $C_{1-6}$- Alkyl, NH- $C_{1-6}$- Alkyl- OH, N ($C_{1-6}$Alkyl)$_2$, N ($C_{1-6}$- Alkyl- OH)$_2$, $NO_2$, SH, S- $C_{1-6}$- Alkyl, OH, O- $C_{1-6}$- Alkyl, O- $C_{1-6}$Alkyl- OH, C (=O) $C_{1-6}$- Alkyl,

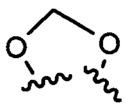 ; 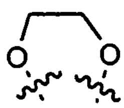

$CO_2H$, $CO_2$- $C_{1-6}$- Alkyl, $CF_3$, $OCF_3$, $C_{1-6}$- Alkyl; an einem oder ggf. verschiedenen Atomen (wobei ein Substituent ggf. seinerseits substituiert sein kann) . Die Mehrfachsubstitution erfolgt dabei mit dem gleichen oder mit unterschiedlichen Substituenten. Für "Aryl" und "Heteroaryl" sind dabei bevorzugte Substituenten- F, -Cl, -$CF_3$, -O- $CH_3$, OH, Methyl, Ethyl, n- Propyl, Nitro, tert.- Butyl,

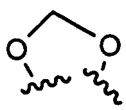

und- CN. Besonders bevorzugt sind- F und- Cl.

Unter dem Begriff des mit einer physiologisch verträglichen Säure gebildeten Salzes versteht man im Sinne dieser Erfindung Salze des jeweiligen Wirkstoffes mit anorganischen bzw. organischen Säuren, die physiologisch- insbesondere bei Anwendung im Menschen und/ oder Säugetier- verträglich sind. Bevorzugt ist das Hydrochlorid, das Citrat, das Hemicitrat und das Methansulfonat. Besonders bevorzugt ist das Methansulfonat. Beispiele für physiologisch verträgliche Säuren sind: Salzsäure, Bromwasserstoffsäure, Schwefelsäure, Methansulfonsäure, Ameisensäure, Essigsäure, Oxalsäure, Bernsteinsäure, Weinsäure, Mandelsäure, Fumarsäure, Milchsäure, Äpfelsäure, Maleinsäure, Zitronensäure, Glutaminsäure, 1, 1- Dioxo- 1, 2- dihydro1$\lambda^6$- benzo [d] isothiazol- 3- on (Saccharinsäure), Monomethylsebacinsäure, 5- Oxo- prolin, Hexan- 1- sulfonsäure, Nicotinsäure, 2-, 3- oder 4- Aminobenzoesäure, 2, 4, 6- Trimethylbenzoesäure, $\alpha$- Liponsäure, Acetylglycin, Hippursäure, Phosphorsäure und/ oder Asparaginsäure. Bevorzugt sind Citronensäure, Methansulfonsäure und Salzsäure. Besonders bevorzugt ist die Methansulfonsäure.

Unter dem Begriff $(CH_2)_{3-6}$ bzw $(CH_2)_{4-5}$ ist- $CH_2$- $CH_2$- $CH_2$-, - $CH_2$- $CH_2$- $CH_2$- $CH_2$-, - $CH_2$- $CH_2$- $CH_2$- $CH_2$- $CH_2$- und $CH_2$- $CH_2$- $CH_2$- $CH_2$- $CH_2$- $CH_2$- bzw.- $CH_2$- $CH_2$- $CH_2$- $CH_2$- und- $CH_2$- $CH_2$- $CH_2$- $CH_2$- $CH_2$- zu verstehen.

Bevorzugt sind Verbindungen der allgemeinen Formel I,

worin die Reste A, W, X und $R^{1-17}$ die oben angegebene Bedeutung haben

wobei

die vorstehend genannten $C_{1-8}$- Alkyle, $C_{1-5}$- Alkyle, $C_{1-3}$- Alkyle bzw. $C_{1-3}$- Alkylene bzw. $C_{3-8}$- Cycloalkylreste jeweils einfach oder mehrfach mit F, Cl, Br, I, CN, $NH_2$, NH- $C_{1-6}$- Alkyl, NH- $C_{1-6}$- Alkyl- OH, N ($C_{1-6}$Alkyl)$_2$, N ($C_{1-6}$- Alkyl- OH)$_2$, $NO_2$, SH, S- $C_{1-6}$- Alkyl, OH, O- $C_{1-6}$- Alkyl, O- $C_{1-6}$Alkyl- OH, C (=O) $C_{1-6}$- Alkyl, $CO_2H$, $CO_2$- $C_{1-6}$- Alkyl, $CF_3$, $OCF_3$, $C_{1-6}$- Alkyl substituiert sein können,

die vorstehend genannten Aryl- oder Heteroaryl- Reste jeweils einfach oder mehrfach mit F, Cl, Br, I, CN, $NH_2$, NH-

$C_{1-6}$- Alkyl, NH- $C_{1-6}$- Alkyl- OH, N ($C_{1-6}$Alkyl)$_2$, N ($C_{1-6}$- Alkyl- OH)$_2$, $NO_2$, SH, S- $C_{1-6}$- Alkyl, OH, O- $C_{1-6}$- Alkyl, O- $C_{1-6}$Alkyl- OH, C (=O) $C_{1-6}$- Alkyl, $CO_2H$, $CO_2$- $C_{1-6}$- Alkyl, $CF_3$, $OCF_3$, $C_{1-6}$- Alkyl,

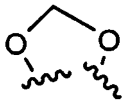 ; 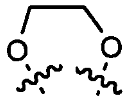

oder Phenoxy substituiert sein können,

in Form des Razemats; der Enantiomere, Diastereomere, Mischungen der Enantiomere oder Diastereomere oder eines einzelnen Enantiomers oder Diastereomers; der Basen und/oder Salze physiologisch verträglicher Säuren.

Die nachfolgend als bevorzugt beschriebenen Reste und Gruppen bzw. Substituenten können in den erfindungsgemäßen Verbindungen mit der breitesten Bedeutung der übrigen Reste, aber auch mit bevorzugten Bedeutungen anderer Reste und Gruppen bzw. Substituenten kombiniert werden.

Für eine bevorzugte Ausführungsform der erfindungsgemäßen spirocyclischen Azaindol-Derivate gilt, dass

$R^1$ und $R^2$ unabhängig voneinander für H; $C_{1-5}$- Alkyl, gesättigt oder ungesättigt, verzweigt oder unverzweigt, einfach oder mehrfach substituiert oder unsubstituiert, stehen;

oder die Reste $R^1$ und $R^2$ zusammen einen Ring bilden und $CH_2CH_2OCH_2CH_2$, $CH_2CH_2NR^{11}CH_2CH_2$ oder $(CH_2)_{3-6}$ bedeuten,

wobei $R^{11}$ H; $C_{1-5}$- Alkyl, gesättigt oder ungesättigt, verzweigt oder unverzweigt, einfach oder mehrfach substituiert oder unsubstituiert, bedeutet.

Besonders bevorzugt sind spirocyclische Azaindol-Derivate, worin $R^1$ und $R^2$ unabhängig voneinander für $CH_3$ oder H stehen, wobei $R^1$ und $R^2$ nicht gleichzeitig H bedeuten
oder $R^1$ und $R^2$ für $(CH_2)_3$ stehen.

Ganz besonders bevorzugt sind spirocyclische Azaindol-Derivate, worin $R^1$ und $R^2$ für $CH_3$ stehen.

Weiterhin bevorzugt sind spirocyclische Azaindol-Derivate, worin

$R^3$ für Ethyl, Propyl, Butyl, Pentyl, Hexyl, Heptyl, Cyclopentyl, Cyclohexyl, Phenyl, Benzyl, Naphthyl, Anthracenyl, Thiophenyl, Benzothiophenyl, Furyl, Benzofuranyl, Benzodioxolanyl, Indolyl, Indanyl, Benzodioxanyl, Pyrrolyl, Pyridyl, Pyrimidyl oder Pyrazinyl, jeweils unsubstituiert oder einfach oder mehrfach substituiert; über eine gesättigte, unverzweigte $C_{1-3}$- Alkyl- Gruppe gebundenen $C_5$- oder $C_6$- Cycloalkyl, Phenyl, Naphthyl, Anthracenyl, Thiophenyl, Benzothiophenyl, Pyridyl, Furyl, Benzofuranyl, Benzodioxolanyl, Indolyl, Indanyl, Benzodioxanyl, Pyrrolyl, Pyrimidyl, Triazolyl oder Pyrazinyl, jeweils unsubstituiert oder einfach oder mehrfach substituiert, steht;

insbesondere

$R^3$ Propyl, Butyl, Pentyl, Hexyl, Phenyl, Furyl, Thiophenyl, Naphthyl, Benzyl, Benzofuranyl, Indolyl, Indanyl, Benzodioxanyl, Benzodioxolanyl, Pyridyl, Pyrimidyl, Pyrazinyl, Triazolyl oder Benzothiophenyl, jeweils unsubstituiert oder einfach oder mehrfach substituiert; über eine gesättigte, unverzweigte $C_{1-3}$- AlkylGruppe gebundenen Phenyl, Furyl oder Thiophenyl, jeweils unsubstituiert oder einfach oder mehrfach substituiert, bedeutet.

Besonders bevorzugt sind spirocyclische Azaindol- Derivate, worin $R^3$ für Propyl, Butyl, Pentyl, Hexyl, Phenyl, Phenethyl, Thiophenyl, Pyridyl, Triazolyl, Benzothiophenyl oder Benzyl, jeweils substituiert oder unsubstituiert, steht, besonders bevorzugt für Propyl, 3- Methoxypropyl, Butyl, Pentyl, Hexyl, Phenyl, 3- Methylphenyl, 3- Fluorphenyl, Benzo [1, 3]- dioxolyl, Thienyl, Benzothiophenyl, 4- Chlorbenzyl, Benzyl, 3- Chlorbenzyl, 4- Methylbenzyl, 2- Chlorbenzyl, 4- Fluorbenzyl, 3- Methylbenzyl, 2- Methylbenzyl, 3- Fluorbenzyl, 2- Fluorbenzyl, 1- Methyl- 1, 2, 4- triazolyl oder Phenethyl.

Ganz besonders bevorzugt sind spirocyclische Azaindol- Derivate, worin $R^3$ Butyl, Ethyl, 3- Methoxypropyl, Benzothiophenyl, Phenyl, 3- Methylphenyl, 3- Fluorphenyl, Benzo [1, 3]- dioxolyl, Benzyl, 1- Methyl- 1, 2, 4- triazolyl, Thienyl oder Phenethyl bedeutet.

$R^4$ steht vorzugsweise für H.

Außerdem bevorzugt sind spirocyclische Azaindol- Derivate, worin $R^5$ für H, $C_{1-5}$- Alkyl, verzweigt oder unverzweigt, unsubstituiert oder einfach oder mehrfach substituiert oder $COOR^{13}$ steht.

Besonders bevorzugt sind spirocyclische Azaindol-Derivate, worin $R^5$ für $CH_3$, $CH_2OH$, COOH oder $COOCH_3$ steht.

Ganz besonders bevorzugt sind spirocyclische Azaindol-Derivate, worin $R^5$ H bedeutet.

Bevorzugt sind auch spirocyclische Azaindol- Derivate, worin $R^6$ für H, $C_{1-5}$- Alkyl, Aryl oder über eine $C_{1-3}$-Alkylgruppe verknüpftes Aryl steht.

Besonders bevorzugt sind spirocyclische Azaindol-Derivate, worin $R^6$ für H, $CH_3$, Phenyl oder Benzyl steht.

Ganz besonders bevorzugt sind spirocyclische Azaindol-Derivate, worin $R^6$ für H steht.

Darüber hinaus sind auch spirocyclische Azaindol-Derivate bevorzugt, worin $R^7$, $R^8$, $R^9$ und $R^{10}$ unabhängig voneinander für H; Methyl, Ethyl, Propyl, Butyl; Pyridyl, O-Benzyl, F, Cl, Br, I, $CF_3$, OH, $OCH_3$, $NH_2$, COOH, $COOCH_3$, $NHCH_3$ oder $N(CH_3)_2$ oder $NO_2$ stehen.

Besonders bevorzugt sind spirocyclische Azaindol-Derivate, worin $R^7$, $R^8$, $R^9$ und $R^{10}$ unabhängig voneinander für H, F, OH, $CH_3$, Cl, $OCH_3$, Br oder $NO_2$ stehen.

Ganz besonders bevorzugt sind spirocyclische Azaindol-Derivate, worin $R^7$, $R^8$, $R^9$ und $R^{10}$ für H stehen.

Bevorzugt sind auch substituierte Azaindol-Derivate der allgemeinen Formel I, worin A einmal N bedeutet und die übrigen Reste A die Bedeutung $CR^{7-9}$ oder $CR^{8-10}$ oder $CR^7$ und $CR^{9-10}$ oder $CR^{7-8}$ und $CR^{10}$ annehmen.

Besonders bevorzugt sind Verbindungen der allgemeinen Formeln Ia und Ib:

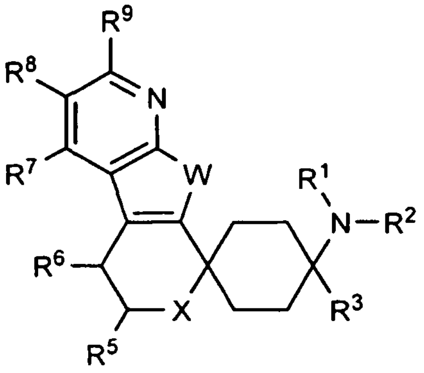

**Ia**

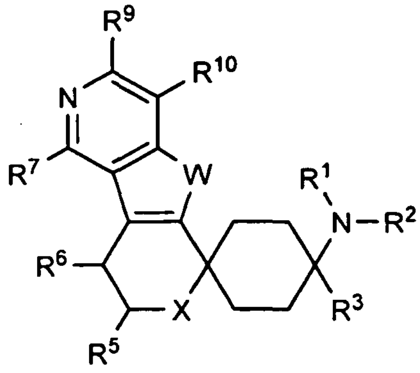

**Ib**

worin X, W und die Reste $R^1$-$R^{10}$ die in der breitesten Definition aufgeführten sowie die in den als bevorzugt bezeichneten Definitionen beschriebenen Bedeutungen annehmen können.

Ganz besonders bevorzugt sind substituierte Azaindol-Derivate aus der Gruppe

(1) 4- (Dimethylamino)- 4- phenyl- spiro [cyclohexan- 1, 8'- (5, 6, 8, 9- Tetrahydro- pyrano [3, 4- b]- 7- aza- indol) ]

(2) 4- (Dimethylamino)- 4- phenyl- spiro [cyclohexan- 1, 8'- (5, 6, 8, 9- Tetrahydro- pyrano [3, 4- b]- 7- aza- indol) ] ; Methansulfonat

(3) 4- (Dimethylamino)- 4- phenyl- spiro [cyclohexan- 1, 6'- (5, 6, 8, 9- Tetrahydro- pyrano [3, 4- b]- 5- aza- indol) ]

(4) 4- (Dimethylamino)- 4- thiophen- 2- yl- spiro [cyclohexan- 1, 8'- (5, 6, 8, 9- Tetrahydro- pyrano [3, 4- b]- 7- aza-indol) ] ; Methansulfonat

(5) 4- (Dimethylamino)- 4- thiophen- 2- yl- spiro [cyclohexan- 1, 6'- (5, 6, 8, 9- Tetrahydro- pyrano [3, 4- b]- 5- aza-indol) ]

(6) 4- (Dimethylamino)- 4- phenyl- spiro [cyclohexan- 1, 8'- (5, 6, 8, 9- Tetrahydro- pyrano [3, 4- b]- 7- aza- indol) ] ; Citrat (4: 3)

(7) 4- (Methylamino)- 4- phenyl- spiro [cyclohexan- 1, 8'- (5, 6, 8, 9- Tetrahydro- pyrano [3, 4- b]- 7- aza- indol) ] ; Citrat (1: 1)

(8) 4- (Methylamino)- 4- tiophen- 2- yl- spiro [cyclohexan- 1, 8'- (5, 6, 8, 9- Tetrahydro- pyrano [3, 4- b]- 7- aza- indol) ] ; Citrat (4: 3)

(9) 4- (Dimethylamino)- 4- benzo [1, 3- dioxol]- spiro [cyclohexan- 1, 8'- (5, 6, 8, 9- Tetrahydro- pyrano [3, 4- b]- 7- aza- indol) ] ; Citrat (1: 1)

(10) 4- (Dimethylamino)- 4- (Benzothiophen- 2- yl)- spiro [cyclohexan- 1, 8'- (5, 6, 8, 9- Tetrahydro- pyrano [3, 4- b]- 7- aza- indol) ] ; Citrat (1: 1)

(12) 4- (Dimethylamino)- 4- (3- fluorophenyl)- spiro [cyclohexan- 1, 8'- (5, 6, 8, 9- Tetrahydro- pyrano [3, 4- b]- 7- aza- indol) ] ; Citrat (4: 3)

(13) 4- (Dimethylamino)- 4- (3- methylphenyl)- spiro [cyclohexan- 1, 8'- (5, 6, 8, 9- Tetrahydro- pyrano [3, 4- b]- 7- aza- indol) ] ; Citrat (1: 1)

(14) 4- (Dimethylamino)- 4- (but- 1- yl)- spiro [cyclohexan- 1, 8'- (5, 6, 8, 9- Tetrahydro- pyrano [3, 4- b]- 7- aza- indol) ], Citrat (1: 1)

(15) 4- (Dimethylamino)- 4- Phenylethyl- spiro [cyclohexan- 1, 8'- (3- trifluoromethyl- 5, 6, 8, 9- Tetrahydro- pyrano [3, 4- b]- 7- aza- indol) ] ; Citrat (1: 1)

(17) 4- (Dimethylamino)- 4- Ethyl- spiro [cyclohexan- 1, 8'- (5, 6, 8, 9- Tetrahydro- pyrano [3, 4- b]- 7- aza- indol) ] ; Citrat (2: 3)

(18) 4- (Dimethylamino)- 4- Phenylethyl- spiro [cyclohexan- 1, 8'- (5, 6, 8, 9- Tetrahydro- pyrano [3, 4- b]- 7- aza- indol) ] ; Citrat (2: 3)

(19) 4- Benzyl- 4- morpholino- spiro [cyclohexan- 1, 8'- (5, 6, 8, 9- Tetrahydro- pyrano [3, 4- b]- 7- aza- indol) ] ; Citrat (1: 1)

(20) 4- (Dimethylamino)- 4- (3- methoxypropyl)- spiro [cyclohexan- 1, 8'- (5, 6, 8, 9- Tetrahydro- pyrano [3, 4- b]- 7- aza- indol) ] ; Citrat (1: 1)

(21) 4- (Dimethylamino)- 4- phenyl- spiro [cyclohexan- 1, 8'- (5, 6, 8, 9- Tetrahydro- pyrano [3, 4- b]- 7- aza- indol) ] ; Citrat (1: 1)

(22) 4- (Dimethylamino)- 4- thiophen- 2- yl- spiro [cyclohexan- 1, 8'- (5, 6, 8, 9- Tetrahydro- pyrano [3, 4- b]- 7- aza- indol) ] ; Citrat (1: 1)

(23) 4- (Dimethylamino)- 4- phenyl- spiro [cyclohexan- 1, 6'- (5, 6, 8, 9- Tetrahydro- pyrano [3, 4- b]- 5- aza- indol) ], Citrat (1: 1)

(24) 4- (Dimethylamino)- 4- phenyl- spiro [cyclohexan- 1, 6'- (5, 6, 8, 9- Tetrahydro- pyrano [3, 4- b]- 5- aza- indol) ], Citrat (1: 1)

(25) 4- Butyl- 4- (dimethylamino)- spiro [cyclohexan- 1, 8'- (5, 6, 8, 9- Tetrahydro- pyrano [3, 4- b]- 7- aza- indol) ] ; Citrat (1: 1)

(26) 4- Butyl- 4- (dimethylamino)- spiro [cyclohexan- 1, 8'- (5, 6, 8, 9- Tetrahydro- pyrano [3, 4- b]- 7- aza- indol) ] ; Citrat (1: 1)

(27) 4- Benzyl- 4- morpholino- spiro [cyclohexan- 1, 8'- (3- trifluoromethyl- 5, 6, 8, 9- Tetrahydro- pyrano [3, 4- b]- 7- aza- indol) ] ; Citrat (1: 1)

(28) 4- (Dimethylamino)- 4- phenyl- spiro [cyclohexan- 1, 8'- (3- trifluoromethyl- 5, 6, 8, 9- Tetrahydro- pyrano [3, 4- b]- 7- aza- indol) ] ; Citrat (1: 1)

(29) 4- (Azetidin- 1- yl)- 4- phenyl- spiro [cyclohexan- 1, 8'- (5, 6, 8, 9- Tetrahydro- pyrano [3, 4- b]- 7- aza- indol) ],

Citrat (1: 1)

(30) 4- Butyl- 4- (pyrrolidin- 1- yl)- spiro [cyclohexan- 1, 8'- (5, 6, 8, 9- Tetrahydro- pyrano [3, 4- b]- 7- aza- indol) ] ; Citrat (2: 1)

(31) 4- (Dimethylamino)- 4- phenyl- spiro [cyclohexan- 1, 8'- (5, 6, 8, 9- Tetrahydro- thiopyrano [3, 4- b]- 7- aza-indol) ] ; Citrat (1: 1)

(32) 4- (Dimethylamino)- 4- phenyl- spiro [cyclohexan- 1, 6'- (5, 6, 8, 9- Tetrahydro- thiopyrano [3, 4- b]- 5- aza-indol) ], Citrat (1: 1)

(33) 4- (1- Methyl- 1H- 1, 2, 4- triazol- 5- yl)- 4- (methylamino)- spiro [cyclohexan- 1, 8'- (5, 6, 8, 9- Tetrahydro-pyrano [3, 4- b]- 7- aza- indol) ], Citrat (1: 1)

(34) 4- (1- Methyl- 1H- 1, 2, 4- triazol- 5- yl)- 4- (methylamino)- spiro [cyclohexan- 1, 8'- (5, 6, 8, 9- Tetrahydro-pyrano [3, 4- b]- 7- aza- indol) ], Citrat (1: 1)

in Form des Razemats; der Enantiomere, Diastereomere, Mischungen der Enantiomere oder Diastereomere oder eines einzelnen Enantiomers oder Diastereomers; der Basen und/oder Salze physiologisch verträglicher Säuren.

Die erfindungsgemäßen Substanzen wirken beispielsweise auf den im Zusammenhang mit verschiedenen Erkrankungen relevanten μ- Opioid- Rezeptor, sodass sie sich als pharmazeutischer Wirkstoff in einem Arzneimittel eignen. Ein weiterer Gegenstand der Erfindung sind daher Arzneimittel enthaltend wenigstens ein erfindungsgemäßes spirocyclisches Azaindol- Derivat, sowie gegebenenfalls geeignete Zusatz- und/ oder Hilfsstoffe und/ oder gegebenenfalls weitere Wirkstoffe.

Die erfindungsgemäßen Arzneimittel enthalten neben mindestens einem erfindungsgemäßen spirocyclischen Azaindol-Derivat gegebenenfalls geeignete Zusatz- und/oder Hilfsstoffe, so auch Trägermaterialien, Füllstoffe, Lösungsmittel, Verdünnungsmittel, Farbstoffe und/oder Bindemittel und können als flüssige Arzneiformen in Form von Injektionslösungen, Tropfen oder Säfte, als halbfeste Arzneiformen in Form von Granulaten, Tabletten, Pellets, Patches, Kapseln, Pflaster/Sprühpflaster oder Aerosolen verabreicht werden. Die Auswahl der Hilfsstoffe etc. sowie die einzusetzenden Mengen derselben hängen davon ab, ob das Arzneimittel oral, peroral, parenteral, intravenös, intraperitoneal, intradermal, intramuskulär, intranasal, buccal, rektal oder örtlich, zum Beispiel auf die Haut, die Schleimhäute oder in die Augen, appliziert werden soll. Für die orale Applikation eignen sich Zubereitungen in Form von Tabletten, Dragees, Kapseln, Granulaten, Tropfen, Säften und Sirupen, für die parenterale, topische und inhalative Applikation Lösungen, Suspensionen, leicht rekonstituierbare Trockenzubereitungen sowie Sprays. Erfindungsgemäße spirocyclische Azaindol-Derivate in einem Depot, in gelöster Form oder in einem Pflaster, gegebenenfalls unter Zusatz von die Hautpenetration fördernden Mitteln, sind geeignete perkutane Applikationszubereitungen. Oral oder perkutan anwendbare Zubereitungsformen können die erfindungsgemäßen spirocyclische Azaindol-Derivate verzögert freisetzen. Die erfindungsgemäßen spirocyclischen Azaindol-Derivate können auch in parenteralen Langzeitdepotformen wie z. B. Implantaten oder implantierten Pumpen angewendet werden. Prinzipiell können den erfindungsgemäßen Arzneimitteln andere dem Fachmann bekannte weitere Wirkstoffe zugesetzt werden.

Die an den Patienten zu verabreichende Wirkstoffmenge variiert in Abhängigkeit vom Gewicht des Patienten, von der Applikationsart, der Indikation und dem Schweregrad der Erkrankung. Üblicherweise werden 0,00005 bis 50 mg/kg, bevorzugt 0,01 bis 5 mg/kg wenigstens eines erfindungsgemäßen spirocyclischen Azaindol-Derivats appliziert.

Für alle vorstehenden Formen der erfindungsgemäßen Arzneimittel ist es besonders bevorzugt, wenn das Arzneimittel neben wenigstens einem spirocyclischen Azaindol-Derivat noch einen weiteren Wirkstoff, insbesondere ein Opioid, vorzugsweise ein starkes Opioid, insbesondere Morphin, oder ein Anesthetikum, vorzugsweise Hexobarbital oder Halothan, enthält.

In einer bevorzugten Form des Arzneimittels liegt ein enthaltenes erfindungsgemäßes spirocyclisches Azaindol-Derivat als reines Diastereomer und/oder Enantiomer, als Razemat oder als nicht-äquimolare oder äquimolare Mischung der Diastereomere und/oder Enantiomere vor.

Der ORL-1-Rezeptor und der μ-Opioid-Rezeptor wurden insbesondere im Schmerzgeschehen identifiziert. Entsprechend können erfindungsgemäße spirocyclische Azaindol-Derivate zur Herstellung eines Arzneimittels zur Behandlung von Schmerz, insbesondere von akutem, neuropathischem oder chronischem Schmerz, verwendet werden.

Ein weiterer Gegenstand der Erfindung ist daher die Verwendung eines erfindungsgemäßen spirocyclischen Azaindol-Derivats zur Herstellung eines Arzneimittels zur Behandlung von Schmerz, insbesondere von akutem, viszeralem, neuropathischem oder chronischem Schmerz.

Ein weiterer Gegenstand der Erfindung ist die Verwendung eines erfindungsgemäßen spirocyclischen Azaindol-Derivats zur Herstellung eines Arzneimittels zur Behandlung von Angstzuständen, von Stress und mit Stress verbundenen Syndromen, Depressionen, Epilepsie, Alzheimer Erkrankung, seniler Demenz, Katalepsie, allgemeinen kognitiven Dysfunktionen, Lern- und Gedächtnis-Störungen (als Nootropikum). Entzugserscheinungen, Alkohol- und/oder Drogen- und/oder Medikamentenmißbrauch und/oder -abhängigkeit. sexuellen Dysfunktionen, cardiovaskulären Erkrankungen, Hypotension, Hypertension, Tinitus, Pruritus, Migräne, Schwerhörigkeit, mangelnder Darmmotilität, gestörter Nahrungsaufnahme, Anorexie, Fettsucht, lokomotorischen Störungen, Diarrhoe, Kachexie, Harninkontinenz bzw. als Muskelrelaxanz, Antikonvulsivum oder Anesthetikum bzw. zur Coadministration bei Behandlung mit einem opioiden Analgetikum oder mit einem Anesthetikum, zur Diurese oder Antinatriurese, Anxiolyse, zur Behandlung von mit der Modulation der Neurotransmitter-Ausschüttung verbundenen neurodegenerativen Erkrankungen, zur Behandlung von Entzugserscheinungen und/oder zur Reduzierung des Suchtpotentials von Opioiden.

Dabei kann es in einer der vorstehenden Verwendungen bevorzugt sein, wenn ein verwendetes spirocyclisches Azaindol-Derivat als reines Diastereomer und/oder Enantiomer, als Razemat oder als nicht-äquimolare oder äquimolare Mischung der Diastereomere und/oder Enantiomere vorliegt.

Ein weiterer Gegenstand der Erfindung ist ein Verfahren zur Behandlung, insbesondere in einer der vorgenannten Indikationen, eines nichthumanen Säugetieres oder Menschen, das oder der eine Behandlung von Schmerzen, insbesondere chronischer Schmerzen, benötigt, durch Verabreichung einer therapeutisch wiksamen Dosis eines erfindungsgemäßen spirocyclischen Azaindol-Derivats, oder eines erfindungsgemäßen Arzneimittels.

Ein weiterer Gegenstand der Erfindung ist ein Verfahren zur Herstellung der erfindungsgemäßen spirocyclischen Azaindol-Derivats wie in der folgenden Beschreibung und Beispielen ausgeführt.

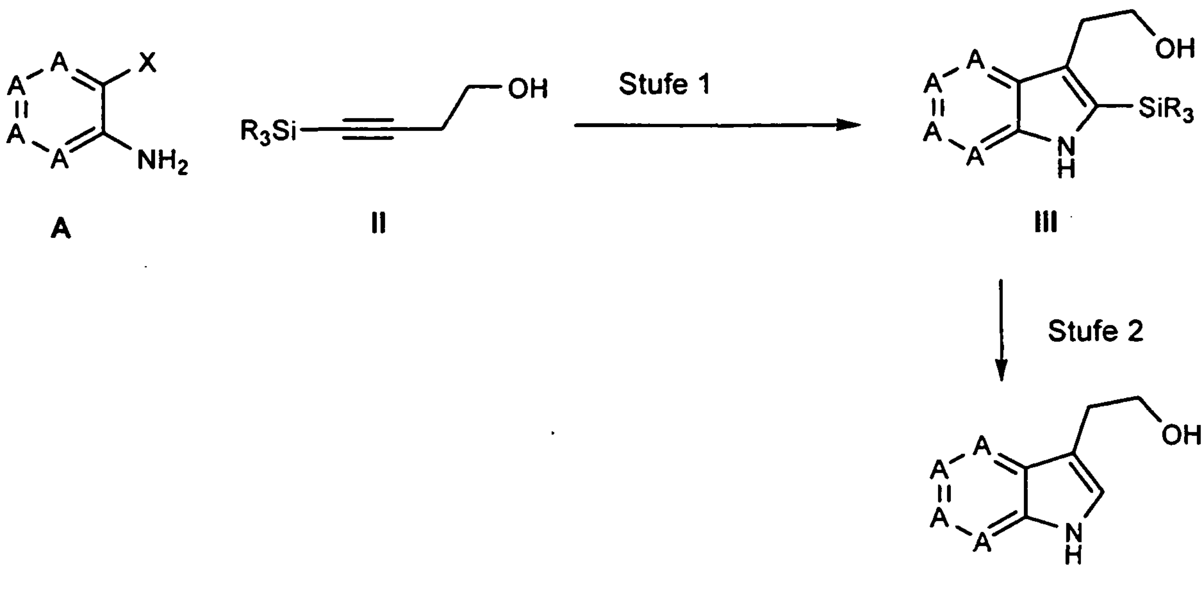


A II III IV

In Stufe 1 werden Verbindungen der vorstehend angegebenen allgemeinen Formel A, worin X für einen Halogen-Rest oder einen Sulfonsäureester, besonders bevorzugt für Iod, Brom oder Trifluormethansulfonat, steht, im Sinne einer Indolsynthese nach Larock mit Alkinen der allgemeinen Formel II in einem Reaktionsmedium, vorzugsweise ausgewählt aus der Gruppe bestehend aus Methanol, Ethylacetat, Ethanol, Isopropanol, n- Butanol, Dioxan, Chloroform, Dichlormethan, Pyridin, Dimethylsulfoxid, Toluol, Tetrahydrofuran, Dimethylformamid, Acetonitril, Diethylether, Wasser und entsprechenden Mischungen, besonders bevorzugt ausgewählt aus der Gruppe bestehend aus Dimethylformamid, Ethylacetat, Tetrahydrofuran, Wasser und entsprechenden Mischungen, vorzugsweise unter Zusatz wenigstens eines Palladiumkatalysators, vorzugsweise ausgewählt aus der Gruppe bestehend aus Palladium (II)- dichlorid [$PdCl_2$], Bis (triphenylphosphin)- palladium (II)- acetat [Pd $(PPh_3)_2$ $(OAc)_2$], Bis (triphenylphosphin)- palladium (II)- chlorid [$PdCl_2$ $(PPh_3)_2$], Palladium (II)- acetat [Pd $(OAc)_2$; Ac = Acetat], Bis (acetonitril)- palladium (II)- chlorid [$(CH_3CN)_2$) $PdCl_2$], Bis (benzonitril)- palladium (II)- chlorid [$(PhCN)_2PdCl_2$] und Tetrakis (triphenylphosphin) palladium [$(PPh_3)_4Pd$], besonders bevorzugt ausgewählt aus der Gruppe bestehend aus Pd $(PPh_3)_2$ $(OAc)_2$, $(PPh_3)_4Pd$ und $PdCl_2$ $(PPh_3)_2$, ggf. in Gegenwart wenigstens eines Phosphins, vorzugsweise eines Phosphins ausgewählt aus der Gruppe bestehend aus Triphenylphosphin, Tri- (tert- butyl)- phosphin, Triphenylarsin und Tri- (ortho- toluyl)- phosphin, besonders bevorzugt in Gegenwart von Triphenylphosphin, ggf. unter Zusatz wenigstens eines anorganischen Salzes, bevorzugt unter Zusatz von Lithiumchlorid oder Tetrabutylammoniumchlorid, ggf. unter Zusatz wenigstens einer anorganischen Base, vorzugsweise ausgewählt aus der Gruppe bestehend aus Kaliumcarbonat, Natriumcarbonat, Kaliumacetat, Natriumhydrogencarbonat und Cäsiumcarbonat, und/ oder Zusatz wenigstens einer organischen Base ausgewählt aus der Gruppe bestehend aus Triethylamin, Diisopropylamin, Diisopropylethylamin und [1, 4] .Diazabicydo- [2.2.2] octan bei Temperaturen von vorzugsweise- 70 °C bis 300 °C, besonders bevorzugt von- 70°C bis 150°C, zu Verbindungen der allgemeinen Formel III umgesetzt.

Verbindungen der allgemeinen Formel A sind kommerziell erhältlich oder aus der Literatur bekannt. Exemplarisch sind Synthesen zu Verbindungen der allgemeinen Formel A im Beispielteil beschrieben.

In Stufe 2 werden Verbindungen der allgemeinen Formel III in einem Reaktionsmedium vorzugsweise ausgewählt aus der Gruppe bestehend aus Methanol, Ethylacetat, Ethanol, Isopropanol, n- Butanol, Dioxan, Chloroform, Dichlormethan, Pyridin, Dimethylsulfoxid, Toluol, Tetrahydrofuran, Dimethylformamid, Acetonitril, Diethylether, Wasser und entsprechenden Mischungen, besonders bevorzugt ausgewählt aus der Gruppe bestehend aus Acetonitril, Tetrahydrofuran, Methanol, Ethanol, Ethylacetat, Pyridin, Wasser und entsprechenden Mischungen, in Gegenwart von Fluorid, ausgewählt aus der Gruppe bestehend aus Tetra- n- butylammoniumfluorid, Flusssäure (HF, HF- Pyridin), Kalium- und/ oder Natriumfluorid, Cäsiumfluorid oder in Gegenwart einer organischen oder anorganischen Säure, vorzugsweise HCl, Essigsäure, Trifluoressigsäure, Bortrifluorid bei Temperaturen von vorzugsweise- 70 °C bis 300 °C, besonders bevorzugt von- 70°C bis 150°C, zu Verbindungen der allgemeinen Formel IV umgesetzt.

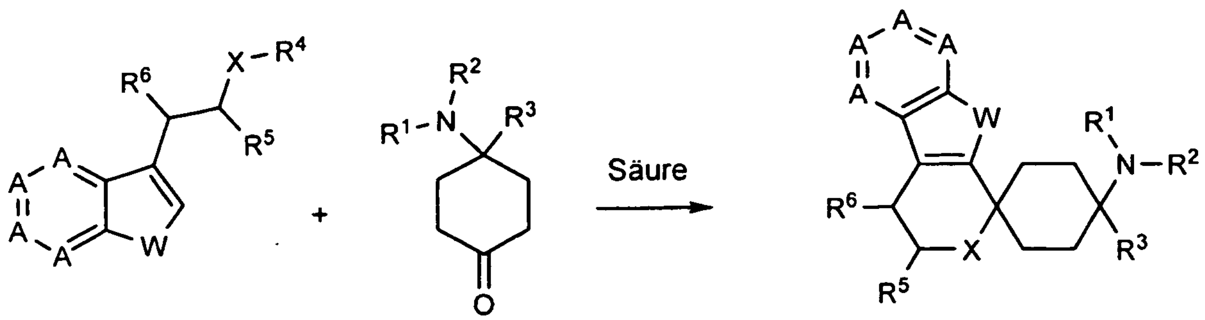


IV VII I

Zur Herstellung der spirocyclischen Verbindungen der allgemeinen Formel VIII werden Ketone der allgemeinen Formel VII mit Heteroaromaten der allgemeinen Formel IV unter Zusatz wenigstens einer organischen Säure oder deren Trimethylsilylester vorzugsweise ausgewählt aus der Gruppe bestehend aus Trifluormethansulfonsäuretrimethylsilylester, Trifluormethansulfonsäure, Methansulfonsäure, Trifluoressigsäure, Essigsäure, Phosphorsäure, p- Toluolsulfonsäure oder einer anorganischen Säure, ausgewählt aus der Gruppe bestehend aus Bortrifluorid, Indium (III) chlorid, Titantetrachlorid, Aluminium (III) chlorid oder unter Zusatz wenigstens eines Übergangsmetallsalzes, vorzugsweise unter Zusatz wenigstens eines Übergangsmetalltriflats (Übergangsmetalltrifluormethansulfonats), besonders bevorzugt unter Zusatz wenigstens eines Übergangsmetalltrifluormethansulfonats ausgewählt aus der Gruppe bestehend aus Scandium (III) trifluormethansulfonat, Ytterbium (III) trifluormethansulfonat und Indium (III) trifluormethansulfonat, in einem geeigneten Lösungsmittel oder Lösungsmittelgemisch wie beispielsweise, Dichlormethan, Dichlorethan, Chloroform, Acetonitril, Diethylether oder Nitroethan, bei Temperaturen von 0 bis 150 °C ggf. unter Verwendung von Mikrowellen umgesetzt.

Die Synthesen der Cyclohexanonderivate mit der allgemeinen Formel VII sind in der Literatur bekannt (WO04043967, WO0290317, US 4065573, Lednicer et al., J.Med.Chem., 23, 1980, 424-430).

Beispiele

Die folgenden Beispiele dienen zur näheren Erläuterung der Erfindung, schränken aber den allgemeinen Erfindungsgedanken nicht ein.

Die Ausbeuten der hergestellten Verbindungen sind nicht optimiert.

Alle Temperaturen sind unkorrigiert.

**Abkürzungen**:

| | |
|---|---|
| d | Tage |
| DCM | Dichlormethan |
| DMF | N, N- Dimethylformamid |

| | |
|---|---|
| Ether | Diethylether |
| EtOAc | Essigsäureethylester |
| $H_2O$ | Wasser |
| MeOH | Methanol |
| $NEt_3$ | Triethylamin |
| RT | Raumtemperatur |
| TBAF | Tetrabutylammoniumfluorid |
| THF | Tetrahydrofuran |
| TMEDA | N, N, N', N'- Tetramethylethylendiamin |
| Bsp | Beispiel |

Mikrowelle: Biotage Initiator, 2, 45 GHz.

**Ketonbausteine**

**Allgemeine Bausteine**

**8-Dimethylamino-1,4-dioxa-spiro[4.5]decan-8-carbonitril:**

*Variante 1:*

**8-Dimethylamino-1,4-dioxa-spiro[4.5]decan-8-carbonitril**

Zu einem Gemisch aus 4N Salzsäure (50 ml) und Methanol (30 ml) wurde unter Eiskühlung 40- proz. wäßrige Dimethylaminlösung (116 ml, 0, 92 mol), Cyclohexan- 1, 4- dion- monoethylenketal (30 g, 0, 192 mol) und Kaliumcyanid (30 g, 0, 46 mol) hinzugefügt. Die Mischung wurde 74 h bei Raumtemperatur gerührt und anschließend nach der Zugabe von Wasser (80 ml) mit Diethylether (4 × 100 ml) extrahiert. Nach dem Einengen wurde der Rückstand in Dichlormethan (200 ml) aufgenommen und mit Magnesiumsulfat über Nacht getrocknet. Die organische Phase wurde eingeengt und das Ketal als weißer Feststoff mit einem Schmelzpunkt von 86- 88 °C in einer Ausbeute von 97 % (40 g) erhalten.

*Variante* **2: 8-Dimethylamino-1,4-dioxa-spiro[4.5]decan-8-Carbonitril**

Zu einem Gemisch aus 4N Salzsäure (50 ml) und Methanol (30 ml) wurde unter Eiskühlung 40- proz. wässrige Dimethylamin- Lösung (116 ml, 0.92 mol), Cyclohexan- 1, 4- dion- monoethylenketal (30.0 g, 0.192 mol) und Kaliumcyanid (30.0 g, 0.46 mol) hinzugefügt. Die Mischung wurde 72 h bei Raumtemperatur gerührt und anschließend nach der Zugabe von Wasser (80 ml) mit Ether (4 x 100 ml) extrahiert. Nach dem Einengen der Lösung wurde der Rückstand in Dichlormethan (200 ml) aufgenommen und mit Magnesiumsulfat über Nacht getrocknet. Die organische Phase wurde eingeengt und das Ketal als weißer Feststoff erhalten.
*Ausbeute:* 38.9 g (96 %)
*Schmelzpunkt:* 86- 88 °C
*$^1$H- NMR (DMSO- $d_6$) :* 1.57 (2 H, m) ; 1.72 (2 H; m) ; 1.85 (2 H, m) ; 1.99 (2 H, m) ; 2.25 (6 H, s) ; 3.87 (4 H, m) .
*$^{13}$C- NMR (DMSO- $d_6$) :* 30.02; 31.32; 60.66; 63.77; 106.31; 118.40.

**8-Methylamino-1,4-dioxa-spiro[4.5]decan-8-carbonitril**

Zu einem Gemisch aus 4N Salzsäure (12.5 ml) und Methanol (7.5 ml) wurde unter Eiskühlung 40%- ige wässrige Methylamin- Lösung (29.0 ml, 0.23 mol), Cyclohexan- 1, 4- dion- monoethylenketal (7.50 g, 0.048 mol) und Kaliumcyanid (7.50 g) hinzugefügt. Die Mischung wurde bei Raumtemperatur 7 d gerührt. Nach Zugabe von Wasser (20 ml) wurde mit Ether (4 x 25 ml) extrahiert. Nach dem Einengen der Lösung wurde der Rückstand in Dichlormethan (50 ml) aufgenommen und mit $MgSO_4$ über Nacht getrocknet. Die organische Phase wurde eingeengt und das Ketal als Öl erhalten, welches durchkristallisierte.
Ausbeute: 7.05 g (80 %)
$^1$H- NMR (DMSO- $d_6$) : 1.54 (2 H, m) ; 1.71 (4 H, m) ; 1.95 (2 H, m) ; 2.30 (3 H, d) ; 2.72 (1 H, q) ; 3.86 (4 H, s) .

**Baustein Ket-1:**

**Dimethyl-(8-phenyl-1,4-dioxaspiro[4.5]dec-8-yl)amin-hydrochlorid**

Zu einer 1, 82 M Phenylmagnesiumchlorid- Lösung in THF (109 ml, 0, 198 mol) wurde unter Argon und Eiskühlung innerhalb von 15 min 8- Dimethylamino- 1, 4- dioxa- spiro [4.5] decan- 8- carbonitril (21 g, 0, 1 mol), gelöst in THF (210 ml), hinzugefügt und dann 16 h bei Raumtemperatur gerührt. Zur Aufarbeitung des Reaktionsgemisches wurde unter Eiskühlung gesättigte Ammoniumchloridlösung (150 ml) zugegeben und mit Diethylether (3 × 100 ml) extrahiert. Die organische Phase wurde mit Wasser (100 ml) und gesättigter NaCl- Lösung (100 ml) ausgeschüttelt und eingeengt. Es blieb ein gelbes Öl (25, 2 g) zurück. Das Rohprodukt wurde in Ethylmethylketon (280 ml) gelöst und unter Eiskühlung mit $ClSiMe_3$ (18, 8 ml, 0, 15 mol) versetzt. Nach einer Reaktionszeit von 6 h konnte Dimethyl- (8- phenyl- 1, 4- dioxaspiro [4.5] dec- 8- yl) amin- hydrochlorid in einer Ausbeute von 35 % (10, 5 g) als weißer Feststoff isoliert werden.

**4-Dimethylamino-4-phenylcyclohexanon (Ket-1)**

Dimethyl- (8- phenyl- 1, 4- dioxaspiro [4.5] dec- 8- yl) amin- hydrochlorid (10, 5 g, 35, 2 mmol) wurde in 7, 5N Salzsäure (36 ml) gelöst und 96 h bei Raumtemperatur gerührt. Nach beendeter Hydrolyse wurde die Reaktionsmischung mit Diethylether extrahiert (2 × 50 ml) . Die wäßrige Phase wurde unter Eiskühlung mit 5N Natronlauge alkalisch gemacht, mit Dichlormethan (3 × 50 ml) extrahiert und eingeengt. 4- Dimethylamino- 4- phenylcyclohexanon (Ket- 1) konnte so als gelber Feststoff mit einem Schmelzpunkt von 104- 108 °C in einer Ausbeute von 97 % (7, 4 g) isoliert werden.

**Baustein Ket-2:**

**Dimethyl-(8-thiophen-2-yl-1,4-dioxaspiro[4.5]dec-8-yl)amin-hydrochlorid**

2- Iodthiophen (22, 9 g, 109 mmol) wurde unter Argon in THF (80 ml) gelöst und innerhalb von 30 min bei 0 °C mit 2M Isopropylmagnesiumchlorid (35, 7 ml, 72 mmol) in THF versetzt. Nach einer Reaktionszeit von 1 h bei 3- 5 °C wurde 8- Dimethylamino- 1, 4- dioxa- spiro [4.5] decan- 8- carbonitril (10 g, 47, 6 mmol), gelöst in Tetrahydrofuran (20 ml), hinzugefügt und 20 h bei Raumtemperatur gerührt. Die Aufarbeitung des Ansatzes erfolgte durch Zugabe von gesättigter $NH_4Cl$- Lösung (85 ml) und Extraktion mit Diethylether (3x100 ml) . Die organische Phase wurde mit Wasser (50 ml) und gesättigter NaCl- Lösung (50 ml) ausgeschüttelt und eingeengt. Es konnte ein dunkelbraunes Öl (21, 3 g) erhalten werden. Das Rohprodukt wurde in Ethylmethylketon (140 ml) gelöst und mit $ClSiMe_3$ (9, 1 ml, 71, 4 mmol) versetzt. Nach einer Reaktionszeit von 6 h wurde Dimethyl- (8- thiophen- 2- yl- 1, 4- dioxaspiro [4.5] dec- 8- yl) amin-hydrochlorid als weiße, kristalline Verbindung in einer Ausbeute von 60 % (8, 74 g) isoliert.

**4-Dimethylamino-4-thiophen-2-ylcyclohexanon (Ket-2)**

Dimethyl- (8- thiophen- 2- yl- 1, 4- dioxaspiro [4.5] dec- 8- yl) amin- hydrochlorid (8, 68 g, 28, 6 h beendeter Hydrolyse wurde die Reaktionsmischung mit Diethylether extrahiert (2 × 50 ml) . Die wäßrige Phase wurde unter Eiskühlung mit 5N Natronlauge alkalisch gemacht, mit Dichlormethan (3 × 50 ml) extrahiert und eingeengt. Ket- 2 wurde so als gelber Feststoff mit einem Schmelzpunkt von 108- 110 °C in einer Ausbeute von 89 % (5, 66 g) erhalten.

**Baustein Ket-3:**

**4-Cyano-4-phenylheptandisäure-dimethylester**

Phenylacetonitril (11, 7 g, 0, 1 mol) und Methylacrylat (47 ml, 0, 5 mol) wurden in tert- Butanol (60 ml) vorgelegt und bis zum Sieden erhitzt. Anschließend wurde die Heizquelle entfernt. Das in tert- Butanol (23 ml) gelöste Triton B (Benzyltrimethylammoniumhydroxid, 40- proz. in Methanol, 15, 2 ml) wurde erst langsam, später zügig zugetropft. Nach dem Zutropfen wurde der Ansatz 4 h unter Rückfluß gekocht. Über Nacht wurde die Reaktionsmischung auf Raumtemperatur abgekühlt.- Zur Aufarbeitung wurde der Ansatz mit Toluol (100 ml) und Wasser (70 ml) versetzt. Die organische Phase wurde abgetrennt und mit Wasser (1 × 70 ml) und gesättigter Natriumchloridlösung (1 × 50 ml) gewaschen. Nach dem Trocknen mit $Na_2SO_4$ wurde das Lösungsmittel wegen der starken Geruchsbelästigung im Abzug destilliert. Die Reinigung erfolgte durch eine Kugelrohrdestillation bei einem Druck von 7, $8x10^{-2}$ mbar und einer Temperatur von 235 °C. Der gewünschte 4- Cyano- 4- phenylheptandisäure- dimethylester konnte in einer Ausbeute von 21, 45 g (72 %) als farblose, zähflüssige Substanz isoliert werden.

**5-Cyano-2-oxo-5-phenylcyclohexancarbonsäure-methylester**

4- Cyano- 4- phenylheptandisäure- dimethylester (14, 45 g, 0, 05 mol) wurde in trockenem Tetrahydrofuran (350 ml) gelöst. Anschließend wurde portionsweise Natrium- tert- butylat (9, 6 g, 0, 1 mol) zugegeben. Bei dieser Zugabe verfärbte sich die Reaktionsmischung orange. Danach wurde der Ansatz 5 h unter Rückfluß gekocht. Während des Kochens entstand eine beigefarbende, breiartige Suspension. Über Nacht wurde die Reaktionsmischung auf Raumtemperatur abgekühlt. Unter Eiskühlung wurde 2, 5N Essigsäure (170 ml) langsam zum Reaktionsgemisch zugetropft. Anschließend wurde der Ansatz mit Toluol (100 ml) versetzt. Die organische Phase wurde abgetrennt und mit gesättigter NatriumhydrogencarbonatLösung (3 × 70 ml), Wasser (3 × 50 ml) und Natriumchloridlösung (1 × 70 ml) gewaschen. Nach dem Trocknen mit $Na_2SO_4$ wurde das Lösungsmittel am Rotationsverdampfer abdestilliert und der Rückstand aus Methanol umkristallisiert. Das gewünschte Produkt konnte in einer Ausbeute von 10, 7 g (83 %) als gelber Feststoff mit einem Schmelzpunkt von 75- 80 °C gewonnen werden.

**4-Cyano-4-phenylcyclohexanon**

5- Cyano- 2- oxo- 5- phenylcyclohexancarbonsäure- methylester (7, 71 g, 0, 03 mol) wurde in 10- proz. $H_2SO_4$ und konzentrierter Essigsäure (240 ml) gelöst. Die Reaktionsmischung wurde 24 h bei 100 °C gerührt. Der Verlauf der Reaktion wurde durch DC verfolgt. Zur Aufarbeitung wurde der Ansatz unter Eiskühlung mit Wasser (400 ml) verdünnt und mit Ethylacetat (3 × 100 ml) extrahiert. Anschließend wurde die organische Phase gründlich mit Wasser (6 × 100 ml), gesättigter Natriumhydrogencarbonatlösung (10 × 100 ml) und gesättigter Natriumchloridlösung (1 × 100 ml) gewaschen. Nach dem Trocknen mit $Na_2SO_4$ wurde das Lösungsmittel am Rotationsverdampfer abdestilliert. Das gewünschte Produkt konnte in einer Ausbeute von 5, 46 g (92 %) mit einem Schmelzpunkt von 106- 107 °C isoliert werden.

**8-Cyano-8-phenyl-1,4-dioxaspiro[4.5]decan**

4- Cyano- 4- phenylcyclohexanon (5, 97 g, 30 mmol) wurde in Toluol (200 ml) aufgenommen und mit Ethylenglykol (4 ml, 71, 6 mmol) versetzt. Nach Zugabe von p- Toluolsulfonsäure (86 mg, 0, 5 mmol) wurde der Ansatz am Wasserabscheider zum Sieden erhitzt. Der Verlauf der Reaktion wurde DC- chromatographisch verfolgt. Nach 20 h war im DC kein Ausgangsprodukt mehr nachweisbar. Nach Abkühlung wurde die Toluol- Lösung mit Wasser (5 x 30 ml) und gesättigter wäßriger NaCl- Lösung (3 x 20 ml) ausgeschüttelt und über $Na_2SO_4$ getrocknet. Nach Entfernung des Lösungsmittels am Rotationsverdampfer fällt das gewünschte Ketal in einer Ausbeute von 6, 8 g (94 %) als weißer Feststoff mit einem Schmelzpunkt von 108- 110 °C an.

**8-Phenyl-1,4-dioxaspiro[4.5]decan-8-carbonsäure**

(Schneider, Woldemar; Krombholz, Gottfried; ARPMAS; Arch.Pharm. (Weinheim Ger.) ; 313; 6; 1980; 487- 498) 8- Cyano- 8- phenyl- 1, 4- dioxaspiro [4.5] decan (4, 86 g, 20 mmol) wurde in Ethylenglykol (40 ml) gelöst, mit NaOH (4 g, 100 mmol) versetzt und anschließend unter Rückfluß zum Sieden erhitzt. Der Reaktionsverlauf wurde mittels DC verfolgt. Nach 20 h war kein Nitril mehr nachweisbar. Zur Aufarbeitung wurde der Ansatz mit Eis (ca. 100 g) versetzt, mit Ether (40 ml) überschichtet und durch langsame Zugabe von halbkonzentrierter HCl (50 ml) angesäuert. Die wäßrige Phase wurde mit Ether extrahiert (3 x 30 ml) . Die vereinigten organischen Extrakte wurden mit gesättigter $NH_4Cl$-Lösung gewaschen (2 x 30 ml), über $Na_2SO_4$ getrocknet und am Rotationsverdampfer zur Trockne gebracht. Durch Umkristallisation des anfallenden Feststoffs aus Toluol wurde die gewünschte Carbonsäure als kristalliner Feststoff mit einem Schmelzpunkt von 134- 139 °C in einer Ausbeute von 3, 1 g (59 %) gewonnen.

**8-Isocyanato-8-phenyl-1,4-dioxaspiro[4.5]decan**

8- Phenyl- 1, 4- dioxaspiro [4.5] decan- 8- carbonsäure (3 g, 11, 5 mmol) wurde in Anisol (30 ml) vorgelegt. Die erhaltene Suspension wurde im Eis- Kochsalzbad auf eine Temperatur von 0 °C gekühlt und mit Triethylamin (2, 25 ml, 16 mmol) versetzt. Es entstand eine klare Lösung, die weitere 15 min bei 0 °C gerührt wurde. Anschließend wurde das Gemisch innerhalb von 5 min mit Phosphorsäurediphenylesterazid (2, 5 ml, 11, 5 mmol) versetzt. Das Reaktionsgemisch wurde 20 min bei 0 °C gerührt, innerhalb weiterer 20 min auf RT kommen gelassen und anschließend in einem Ölbad 2 h lang auf 100 °C (Badtemperatur) erhitzt. Zur Aufarbeitung wurde das Anisol im Ölpumpenvakuum abdestilliert. Die chromatographische Reinigung erfolgte an Kieselgel mit Toluol. Das gewünschte Produkt wurde als kristalliner Feststoff mit einem Schmelzpunkt von 38- 41 °C in einer Ausbeute von 2, 7 g (91 %) erhalten.

**Methyl-(8-phenyl-1,4-dioxaspiro[4.5]dec-8-yl)amin**

$LiAlH_4$ (535 mg, 14, 08 mmol) wurde unter Ausschluß von Luftfeuchtigkeit in trockenem THF (4 ml) suspendiert. Das 8- Isocyanato- 8- phenyl- 1, 4- dioxaspiro [4.5] decan (2, 29 g, 8, 8 mmol, gelöst in 40 ml trockenem THF) wurde innerhalb von 20 min zugetropft. Nach vollständiger Zugabe wurde das Reaktionsgemisch 4 h unter Rückfluß zum Sieden erhitzt. Nach Abkühlung wurde das Reaktionsgemisch unter Eiskühlung zunächst vorsichtig mit wäßrigem THF (1 ml $H_2O$ in 3 ml), dann mit 1, 7 ml 15- proz. Natronlauge und schließlich mit 5 ml $H_2O$ versetzt. Der Ansatz wurde 20 min gerührt und anschließend über Kieselgur filtriert. Das nach mehrmaligen Waschen des Filterkuchens mit Ethylacetat erhaltene Lösungsmittelgemisch wurde am Rotationsverdampfer zur Trockene eingeengt. Das gewünschte Produkt wurde in einer Ausbeute von 2, 1 g (97 %) als viskoses Öl erhalten.

**4-Methylamino-4-phenylcyclohexanon (Ket-3)**

(Upjohn_ Lednicer, US4065573A1, 1977)

Methyl- (8- phenyl- 1, 4- dioxaspiro [4.5] dec- 8- yl) amin (2, 1 g, 8, 4 mmol) wurde mit einem Gemisch aus konz. HCl (15 ml) und Wasser (8 ml) übergossen und 5 Tage bei RT gerührt. Zur Aufarbeitung wurde das Reaktionsgemisch mit Wasser (20 ml) verdünnt und mit Ether (3 × 30 ml) extrahiert. Die etherische Phase wurde verworfen. Die wäßrige Phase wurde anschließend mit 2N NaOH basisch gemacht und mit Dichlormethan (3 × 30 ml) extrahiert. Die so erhaltene organische Phase wurde mit $Na_2SO_4$ getrocknet und anschließend am Rotationsverdampfer eingeengt. Das Keton Ket- 3 konnte durch chromatographische Reinigung an Kieselgel mit Ethylacetat/ Ethanol (4 : 1) in einer Ausbeute von 1, 38 g (81 %) als Feststoff mit einem Schmelzpunkt von 32- 38°C erhalten werden.

**Baustein Ket-4: 2-(Chlormethyl)thiophen**

Thiophen (50 g) wurde mit conc. HCl-Lösung (25 ml) versetzt und das Gemisch auf 0-5 °C gekühlt. Unter einem ständigen HCl-Gasstrom wurde nun wässrige Formaldehyd-Lösung (54.8 ml, 40 %) tropfenweise über einen Zeitraum von 4 h bei 0-15 °C hinzugegeben. Das Reaktionsgemisch wurde für 10 min bei RT gerührt und dann mit Ethylacetat (500 ml) versetzt. Die organische Phase wurde mit gesättigter $NaHCO_3$-Lösung (3×250 ml) und Wasser extrahiert (1 x250 ml) und über $Na_2SO_4$ getrocknet. Vakuumdestillation bei 100 °C-110 °C (Ölbadtemperatur) lieferte (60 °C Kopftemperatur) das gewünschte Produt (8 mm Hg).
Ausbeute: 24 g (30%), farbloses Öl.

**2-(Thiophen-2-yl)acetonitril**

Eine Lösung von 2- (Chlormethyl) thiophen22 g) in DCM (60 ml) wurde mit einer Mischung aus Wasser (90 ml) und NaCN (12.2 g) versetzt. Das Reaktionsgemisch wurde bei 35- 40 °C für 18 h refluxiert. Das Gemisch wurde auf RT abgekühlt und die DCM- Phase abgetrennt. Es wurde mit DCM (2x100 ml) extrahiert und die vereinigten organischen Phasen mit gesättigter NaCl- Lösung gewaschen und über $Na_2SO_4$ getrocknet. Die Lösungsmittel wurden unter reduziertem Druck entfernt und der Rückstand bei 140- 150 °C (Ölbadtemperatur) destilliert (Kopftemperatur: 115- 120 °C) . Anschließende chromatographische Reinigung ($SiO_2$, 5 % EtOAc/n- Hexan) lieferte das gewünschte Produkt.
Ausbeute: 9.2 g (45 %), hellbraunes Öl.

**Ethyl 3-brompropionat**

Eine Lösung von Ethylacrylat (200 g) in Ether (400 ml) wurde auf 0-5 °C gekühlt. In einem separaten Reaktionsgefäß wurde über einen Zeitraum von 3 h Brom (278 ml) zu Tetralin (213 ml) getropft und das sich entwickelnde HBr-Gas zur Ethylacrylatlösung geleitet.
Das Reaktionsgemisch wurde für 12 h gerührt. Der Ether wurde unter reduziertem Druck entfernt und der Rückstand bei 70 °C destilliert (9 mm Hg).
Ausbeute: 360 g (99 %), farbloses Öl.

**Ethyl 5-cyano-2-oxo-5-(thiophen-2-yl)cyclohenxancarboxylat**

Eine Lösung von 2- (Thiophen- 2- yl) acetonitril (10 g) in Toluol (300 ml) wurde mit Ethyl- 3- brompropionat (33.8 g) versetzt und die Mischung auf- 10 °C gekühlt. $NaNH_2$ (27 g) wurde über einen Zeitraumvon 1 h portionsweise hinzugefügt (die Temperatur wurde unter 0°C gehalten) . Das Reaktionsgemisch wurde auf RT erwärmt und für 1 h refluxiert (111 °C) Schliesslich wurde das Gemisch auf 0- 5 °Cgekühlt und mit AcOH / water (50 ml/ 100 ml) versetzt. Die Toluolphase wurde abgetrennt und die wässrige Phase wurde mit Toluol extrahiert (3x200 ml) . Die vereinigten

organischen Extrakte wurden mit 5 % $Na_2CO_3$- Lösung (1×150 ml) und Wasser (1x00 ml) gewaschen und über $Na_2SO_4$ getrocknet. Das Lösungsmittel wurde unter reduziertem Druck entfernt.
Ausbeute: 12 g (55 %), dunkelbraunes Öl.

**4-Cyano-4-(thiophen-2-yl)cyclohexanon**

Ethyl 5- cyano- 2- oxo- 5- (thiophen- 2- yl) cyclohexancarboxylat (12 g) in Essigsäure (120 ml) wurde mit konz. HCl (60 ml) versetzt. Das Reaktionsgemisch wurde für 3 h refluxiert (110 °C- 120 °C) . Das Gemisch wurde auf 0 °C gekühlt und mit 2N NaOH- Lösung (200 ml) neutral (pH- 7) gestellt.

Die wässrige Phase wurde mit Ethylacetat (3x150 ml) extrahiert. Die vereinigten organischen Phasen wurden mit Wasser (1 x300 ml) und gesättigter $NaHCO_3$-Lösung (1 x300 ml) gewaschen und über $Na_2SO_4$ getrocknet. Die Lösungsmittel wurden unter reduziertem Druck entfernt und der Rückstand mittels Säulenchromatographie gereinigt ($SiO_2$, 15 % EtOAc/n-Hexan).
Ausbeute: 37 g(43 %), hellgelber Feststoff.

**8-Cyano-8-(thiophen-2-yl)-1,4-dioxaspiro[4.5]decan**

Eine Lösung von 4- Cyano- 4- (thiophen- 2- yl) cyctohexanon (15 g) in Benzol (120 ml) wurde mit Ethylenglycol (9.08 g) und p- Toluolsulfonsäure (0.0139 g) versetzt und das Reaktionsgemisch für 4 h bei 110 °C am Wasserabscheider (Dean- Stark Apparatur) unter Rückfluß gekocht. Das Reaktionsgemisch wurde auf RT gekühlt und die organische Phase mit wässriger Natriumbicarbonat- Lösung (1×150 ml), Wasser (1 x150 ml) und gesättigter NaCl- Lösung (1x150 ml) gewaschen. Nach Trocknung über $Na_2SO_4$ wurden die Lösungsmittel unter reduziertem Druck entfernt.
Ausbeute: 16.5 g (90 %), hellgleber Feststoff.

**8-(Thiophen-2-yl)-1,4-dioxaspiro[4.5]decan-8-carbonsäure**

8- Cyano- 8- (thiophen- 2- yl)- 1, 4- dioxaspiro [4.5] decan (20 g) in Ethylenglycol (240 ml) wurde mit KOH (22.48 g) versetzt und das Reaktionsgemisch für 16 h bei 140- 150 °C am Rückfluß gekocht.Die Mischung wurde auf RT und dann bei 0- 5°C mit Ether überschichtet (500 ml) . Eiswasser (250 ml) und HCl (30 ml) wurden hinzugefügt und der pH- Wert der wässrigen Phase auf pH~2 eingestellt.Die organische Phase wurde abgetrennt, mit Wasser (1×300 ml) und gesättigter NaCl- Lösung (1×300 ml) gewaschen and über $Na_2SO_4$ getrocknet. Das Lösungsmittel wurde unter reduziertem Druck entfernt.
Ausbeute: 20.5 g (95 %), gelber Feststoff.

**8-Isocyanato-8-(thiophen-2-yl)-1,4-dioxaspiro[4.5]decan**

Zu einer Lösung von 8- (Thiophen- 2- yl)- 1, 4- dioxaspiro [4.5] decan- 8- carbonsäure (26 g) in Toluol (221 ml) wurden TEA (14.1 ml) und DPPA (32.38 g) zugesetzt und das Reaktionsgemisch für 30 min auf 60- 70 °C erhitzt. Toluol wurde dann unter reduziertem Druck entfernt und der Rückstand mittels Säulenchromatographie gereingt (nasses $SiO_2$, 1. Lauf: 10 %EtOAc/n- Hexan, 2. Lauf: 10% EtOAc/ Hexan) .
Ausbeute: 14 g (54 %), hellgrünes Öl.

**N-Methyl-8-(thiophen-2-yl)-1,4-dioxaspiro[4.5]decan-8-amin**

8- Isocyanato- 8- (thiophen- 2- yl)- 1, 4- dioxaspiro [4.5] decan (4 g) wurde in trockenem THF (140 ml) gelöst und die Lösung auf 0- 10°C gekühlt. LAH (4 g) wurde über einen Zeitraum von 30 min portionsweise hinzugefügt und das Reaktionsgemisch für weitere 30 min auf 60°C erwärmt. Das Gemisch wurde auf 0- 10 °C gekühlt und mit gesättigter Ammoniumchlorid- Lösung (100 ml) versetzt. Das Gemisch wurde nun über Celite filtriert und mit Ethylacetat (3x150 ml) gewaschen. Nach Entfernung der Lösungsmittel wurde das Rohprodukt in Ethylacetat (200 ml) aufgenommen und für 3 min bei 0- 10 °C gerührt. Die Ethylacetat- Phase wurde abgetrennt, die wässrige Phase mit gesättigter NaOH- Lösung basisch (pH~10- 14) gestellt und mit Ethylacetat (3×200 ml) extrahiert. Die vereinigten organischen Phasen wurden über $Na_2SO_4$ getrocknet. Das Lösungsmittel wurde unter reduziertem Druck entfernt. Ausbeute: 7.5 g (56 %), farbloser Feststoff.

**4-Methylamino-4-thiophen-2-ylcyclohexanon (Ket-4)**

N- Methyl- 8- (thiophen- 2- yl)- 1, 4- dioxaspiro [4.5] decan- 8- amin (2 g, 7, 9 mmol) wurde mit einem Gemisch aus konz. HCl (15 ml) und Wasser (8 ml) versetzt und 5 Tage bei RT gerührt. Zur Aufarbeitung wurde das Reaktionsgemisch mit Wasser (30 ml) verdünnt und mit Ether extrahiert. Die etherische Phase wurde verworfen. Die wäßrige Phase wurde anschließend mit 2N NaOH basisch gemacht und mit Dichlormethan (3 × 30 ml) extrahiert. Die so erhaltene organische Phase wurde mit $Na_2SO_4$ getrocknet und anschließend am Rotationsverdampfer eingeengt. Der Rückstand wurde durch Säulenchromatographie [Kieselgel 60 (50 g) ; 500 ml Ethylacetat/ Ethanol (5: 1) ] gereinigt und **Ket- 4** in einer Ausbeute von 1, 4 g (85 %) als Feststoff mit einem Schmelzpunkt von 72- 74°C erhalten.

**Baustein Ket-5:**

**(8-Benzo[1,3]dioxol-5-yl-1,4-dioxa-spiro[4.5]dec-8-yl)-dimethyl-amin Hydrochlorid**

Zu einer Lösung von 8- Dimethylamino- 1, 4- dioxa- spiro [4.5] decan- 8- carbonitril (5.25 g, 25 mmol) in abs. THF (75 ml) wurde unter Argon und Eiskühlung innerhalb von 15 min 1 M 3, 4- (Methylendioxy) phenyl- magnesiumbromid-Lösung in Toluol/THF (1: 1) (62.5 ml, 62.5 mmol) bei 5- 10 °C getropft und anschließend bei RT 20 h gerührt. Zur Aufarbeitung des Reaktionsgemisches wurde unter Eiskühlung 20 %- ige Ammoniumchlorid- Lösung (20 ml) und Wasser (25 ml) zugegeben und das Gemisch mit Ether (3 x 50 ml) extrahiert. Die organische Phase wurde mit Wasser und gesättigter Natriumchlorid- Lösung gewaschen, über Natriumsulfat getrocknet und i. Vak. eingeengt. Es verblieb ein farbloses Öl (11.26 g), welches iin Ethylmethylketon (35 ml) gelöst und unter Eiskühlung mit Trimethylchlorsilan (4.75 ml, 37.5 mmol) versetzt wurde. Es wurde im offenen Kolben 5 h bei RT gerührt. Dabei fiel ein farbloser Feststoff aus, der abgesaugt und an der Luft getrocknet wurde.
Ausbeute : 2.7 g (32 %) .
1 H- NMR (DMSO- d6) : 1.71 (2 H, t) ; 1.72 (2 H; d) ; 2.09 (2 H, t) ; 2.43 (6 H, s) ; 2.84 (2 H, d) ; 3.82 (4 H, m) ; 6.11 (2 H, s) ; 7.07 (1 H, d) ; 7.15 (1 H, d) ; 7.32 (1 H, s) ; 10.74 (1 H, bs) .

**4-Benzo[1,3]dioxol-5-yl-4-dimethylamino-cyclohexanon (Ket-5)**

(8- Benzo [1, 3] dioxol- 5- yl- 1, 4- dioxa- spiro [4.5] dec- 8- yl)- dimethyl- amin Hydrochlorid (2.70 g, 7.91 mmol) wurde mit 6N Salzsäure (10 ml) versetzt und bei RT über Nacht gerührt. Nach beendeter Hydrolyse wurde die Reaktionsmischung mit Ether (2 x 20 ml) extrahiert, die wässrige Lösung unter Eiskühlung mit 5N Natronlauge alkalisch gestellt, die Reaktionsmischung mit Dichlormethan (3 x 50 ml) extrahiert, die organische Phase über Natriumsulfat getrocknet und i. Vak. eingeengt.
**Ausbeute (Ket- 5) :** 1.99 g (96 %), farblose Kristalle.
Schmelzpunkt: 122- 124 °C.
1 H- NMR (DMSO- d6) : 2.01 (6 H, s) ; 2.10 (4 H, m) ; 2.43 (6 H, m) ; 6.01 (2 H, s) ; 6.88 (2 H, m) ; 7.02 (1 H, s) .
13C- NMR (DMSO- d6) : 32.39; 36.68; 38.88; 58.82; 100.76; 107.12; 107.67; 120.46; 131.34; 145.69; 147.03; 210.27.

**Baustein Ket-6:**

**2-Iod-benzo[b]thiophen**

In einem 500 ml Dreihalskolben wurde unter Argonatmosphäre Butyllithium 1.6M in Hexan (112.5 ml, 180 mmol) und abs. Ether (70 ml) vorgelegt und im Eisbad auf 0 °C abgekühlt. Anschließend Benzothiophen (20.1 g, 150 mmol) in abs. Ether (40 ml) gelöst und tropfenweise unter Eiskühlung innerhalb von 30 Minuten zugetropft und 2.5 h im Eisbad nachgerührt. Der Reaktionsansatz stand über Nacht im Kühlschrank. In einem 500 ml Dreihalskolben wurden unter Argonatmosphäre Iod (75.0 g) und abs. Ether (50 ml) vorgelegt und Die Kösung der Lithium-Verbindung unter Eiskühlung zugetropft. Der Ansatz wurde langsam auf Raumtemperatur erwärmt, mit Wasser hydrolysiert, mit Natriumthiosulfat-Lösung gewaschen und die organische Phase über Natriumsulfat getrocknet. Anschließend wurde die Reaktionslösung i. Vak. eingeengt und mittels Flash-Chromatographie mit Cyclohexan gereinigt.
*Ausbeute:* 24.1 g (62 %), halbfeste, hellbraune Kristalle
*$^1$H-NMR (DMSO-$d_6$):* 7.32 (2 H, m); 7.75 (1 H, s); 7.81 (1 H, m); 7.93 (1 H, m).

**(8-Benzo[b]thiophen-2-yl-1,4-dioxa-spiro[4.5]dec-8-yl)-dimethyl-amin Hydrochlorid**

In einem 100 ml Dreihalskolben wurde Mg (238 mg) in abs. Ether (2 ml) unter Argon vorgelegt, dazu wurde langsam 2- Iod- benzo [b] thiophen (2.51 g, 9.6 mmol) in abs. Ether (8 ml) zugetropft. Nach Zugabe von abs. Ether (10 ml) wurde der Ansatz 5 h unter Rückfluss gekocht. Die Reaktionslösung wurde im Eisbad abgekühlt und bei 10 °C tropfenweise das 8- Dimethylamino- 1, 4- dioxa- spiro [4.5] decan- 8- carbonitril (1.03 g, 4.9 mmol) in THF (10 ml) zugegeben. Der Ansatz rührte bei Raumtemperatur über Nacht, das Reaktionsgemisch wurde unter Eiskühlung mit $NH_4Cl$- Lösung (5 ml) und Wasser (7 ml) versetzt und mit Ether (3 x 30 ml) extrahiert. Die org. Phase wurde mit Wasser

(30 ml) und anschließend mit gesättigter NaCl- Lösung (20 ml) gewaschen, über $Na_2SO_4$ getrocknet und i. Vak. eingeengt.
*Ausbeute:* 1.99 g (66 %)

Das Rohprodukt wurde in Ethylmethylketon (19 ml) gelöst, unter Eiskühlung mit Trimethylchlorsilan (1.63 ml, 12.8 mmol) versetzt und bei Raumtemperatur 5 h gerührt. Der entstandene Niederschlag wurde abgesaugt und i. Vak. getrocknet.
*Ausbeute:* 600 mg (35 %)
$^1$H-NMR (DMSO-$d_6$): 1.46 (2 H, m); 1.79 (2 H, m); 2.37 (2 H, m); 2.63 (6 H, s); 2.75 (2 H, m); 7.47 (2 H, m); 7.91 (1 H, s); 7.95 (1 H, m); 8.06 (1 H, m); 11.40 (1 H, s).
$^{13}$C-NMR (DMSO-$d_6$): 30.43; 31.13; 37.84; 63.88; 66.42; 105.84; 122.48; 124.55; 124.89; 125.71; 128.99; 135.00; 138.91; 139.58.

**4-Benzo[b]thiophen-2-yl-4-dimethylamino-cyclohexanon (Ket-6)**

Das (8- Benzo [b] thiophen- 2- yl- 1, 4- dioxa- spiro [4.5] dec- 8- yl)- dimethyl- amin Hydrochlorid (0.60 g, 1.7 mmol) wurde in Wasser (0.8 ml) gelöst, mit konzentrierter Salzsäure (1.04 ml, 151 mmol) versetzt und 3 d bei Raumtemperatur gerührt. Nach beendeter Hydrolyse wurde die Reaktionsmischung mit Diethylether (2 x 25 ml) extrahiert und die wässrige Phase mit 5N Natronlauge alkalisch gemacht, mit Dichlormethan (3 x 25 ml) extrahiert, über Natriumsulfat getrocknet und i. Vak. eingeengt.
**Ausbeute (Ket- 6) :** 0.44 g (95 %)
*$^1$H- NMR (DMSO- $d_6$) :* 2.19 (10 H, m) ; 2.52 (4 H, m) ; 7.35 (3 H, m) ; 7.84 (1 H, m) ; 7.91 (1 H, m) .
*$^{13}$C- NMR (DMSO- $d_6$) :* 33.74; 36.51; 38.05; 58.60; 121.87; 121.94; 123.35; 124.02; 124.16; 138.19; 139.17; 144.28; 209.50.

**Baustein Ket-7:**

*Variante 1:*

**[8-(3-Fluorphenyl)-1,4-dioxaspiro[4.5]dec-8-yl]dimethylamin-hydrochlorid**

Zu einer Lösung von 8- Dimethylamino- 1, 4- dioxa- spiro [4.5] decan- 8- carbonitril (19, 8 g, 94 mmol) in THF (100 ml) wurde unter Argon und Eiskühlung innerhalb von 15 min 0, 5M 3- Fluorphenylmagnesiumbromid- Lösung in THF (3, 750 ml, 375 mmol) hinzugefügt und dann 16 h bei Raumtemperatur gerührt. Zur Aufarbeitung des Reaktionsgemisches wurde unter Eiskühlung gesättigte Ammoniumchloridlösung (150 ml) und Wasser (60 ml) zugegeben und mit Diethylether (3 × 100 ml) extrahiert. Die organische Phase wurde mit Wasser (50 ml) und gesättigter NaCl- Lösung (50 ml) ausgeschüttelt und eingeengt. Es blieb ein braunes Öl (26, 5 g) zurück, das außer der Phenylverbindung **4** noch das Ketal 2 enthielt. Das Rohprodukt wurde in Ethylmethylketon (156 ml) gelöst und unter Eiskühlung mit ClSiMe3 (17, 8 ml, 141 mmol) versetzt. Nach einer Reaktionszeit von 6 h konnte das Hydrochlorid in einer Ausbeute von 55 % (16, 3 g) als weißer Feststoff mit einem Schmelzpunkt von 275- 278 °C isoliert werden.

***Variante 2:* [8-(3-Fluor-phenyl)-1,4-dioxa-spiro[4.5]dec-8-yl]-dimethyl-amin**

**Hydrochlorid**

Eine Lösung von 1- Brom- 3- fluorbenzol (5.00 g, 28.6 mmol) in abs. Ether (15 ml) wurde zu einer Suspension von Magnesium (694 mg, 28.6 mmol) in abs. Ether (10 ml) so getropft, dass der Ether siedete. Nach beendeter Zugabe wurde 10 min bei RT nachgerührt, das Magnesium war danach vollständig gelöst. Die Reaktionslösung wurde im Eisbad abgekühlt und bei 10°C tropfenweise 8- Dimethylamino- 1, 4- dioxa- spiro [4.5] decan- 8- carbonitril (3.00 g, 14.3 mmol) in abs. THF (30 ml) zugegeben. Der Ansatz rührte bei Raumtemperatur über Nacht, das Reaktionsgemisch wurde unter Eiskühlung mit 20 %iger $NH_4Cl$- Lösung (20 ml) und Wasser (30 ml) versetzt und mit Ether (3 x 50 ml) extrahiert. Die org. Phase wurde mit Wasser (50 ml) und anschließend mit gesättigter NaCl- Lösung (50 ml) gewaschen, über $Na_2SO_4$ getrocknet und i. Vak. eingeengt.
Das Rohprodukt wurde in Ethylmethylketon (25 ml) gelöst, unter Eiskühlung mit $ClSiMe_3$ (3.2 ml, 25 mmol) versetzt und bei Raumtemperatur 5 h gerührt. Der entstandene Niederschlag wurde abfiltriert und i. Vak. getrocknet.
Ausbeute: 2.8 g (62 %)
$^1$H- NMR (DMSO- $d_6$) : 1.91 (8 H, m) ; 2.54 (6 H, s) ; 3.91 (4 H, d) ; 7.37 (1 H, m) ; 7.61 (3 H, m) .

*Variante 1:* **4-Dimethylamino-4-(3-fluor-phenyl)-cyclohexanon (Ket-7)**

[8- (3- Fluor- phenyl)- 1, 4- dioxa- spiro [4.5] dec- 8- yl]- dimethyl- amin Hydrochlorid (7, 2 g, 22, 75 mmol) wurde in Wasser (9, 6 ml) gelöst, mit konzentrierter Salzsäure (14 ml, 455 mmol) versetzt und 4 d bei Raumtemperatur gerührt. Nach beendeter Hydrolyse wurde die Reaktionsmischung mit Diethylether extrahiert (2 × 50 ml), die wäßrige Phase unter Eiskühlung mit 5N Natronlauge alkalisch gemacht, wobei das Produkt ausfiel. Das Keton **Ket- 7** konnte als gelber Feststoff mit einem Schmelzpunkt von 83- 88 °C und einer Ausbeute von 50 % (6, 05 g) isoliert werden.

*Variante 2:*

**4-Dimethylamino-4-(3-fluor-phenyl)-cyclohexanon (Ket-7)**

[8- (3- Fluor- phenyl)- 1, 4- dioxa- spiro [4.5] dec- 8- yl]- dimethyl- amin Hydrochlorid (2.80 g, 8.86 mmol) wurde in Wasser (3.7 ml) gelöst, mit konzentrierter Salzsäure (5.5 ml) versetzt und bei RT 4 d gerührt. Nach beendeter Hydrolyse wurde die Reaktionsmischung mit Ether (2 x 10 ml) extrahiert, die wässrige Lösung unter Eiskühlung mit 5N Natronlauge alkalisch gestellt, die Reaktionsmischung mit Dichlormethan (3 x 50 ml) extrahiert, die organische Phase über Natriumsulfat getrocknet und i. Vak. eingeengt. Das Rohprodukt wurde durch Flash- Chromatographie mit $CHCl_3$/ MeOH (20: 1) gereinigt.
**Ausbeute (Ket- 7) :** 676 mg (32 %), farbloser Feststoff
Schmelzpunkt: 62- 67 °C
$^1$H- NMR (DMSO- $d_6$) : 2.02 (6 H, s) ; 2.12 (5 H, m) ; 2.45 (3 H, m) ; 7.24 (3 H, m) ; 7.43 (1 H, m) .

**Baustein Ket-8:**

**Dimethyl-(8-m-tolyl-1,4-dioxa-spiro[4.5]dec-8-yl)-amin Hydrochlorid**

In einem 500 ml Dreihalskolben wurde 8- Dimethylamino- 1, 4- dioxa- spiro [4.5] decan- 8- carbonitril (8.4 g, 40 mmol) in abs. THF (150 ml) unter Argon und Eiskühlung vorgelegt. Bei 0 °C wurde innerhalb von 15 min m- Tolylmagnesiumbromid, 1M Lösung in THF (100 ml, 100 mmol) zugetropft. Der Reaktionsansatz wurde anschließend 16 h bei Raumtemperatur gerührt.
Der Ansatz wurde unter Eiskühlung mit Ammoniumchlorid-Lösung (20 %ig, 37 ml) und Wasser (50 ml) versetzt und mit Ether (3 x 50 ml) extrahiert.
Die organische Phase wurde mit Wasser (50 ml) und gesättigter NaCl-Lösung gewaschen, über Natriumsulfat getrocknet und i. Vak. eingeengt. Die Rohausbeute betrug 11.25 g (braunes Öl).
Das Rohprodukt wurde in Ethylmethylketon (60 ml) gelöst und bei 0 °C mit Trimethylchlorsilan (7.6 ml, 60 mmol) versetzt. Nach 5 h Rühren bei Raumtemperatur wurde der ausgefallene Niederschlag abgesaugt und mit wenig kaltem Ethylmethylketon nachgewaschen.
Ausbeute: 5.64 g (45 %), weißer Feststoff.
Schmelzpunkt: 230-234 °C.
1 H-NMR (DMSO-d6): 1.19 (2 H, t); 1.67 (2 H; d); 2.13 (2 H, t); 2.44 (9 H, m); 2.89 (2 H, d); 3.87 (4 H, m); 7.43 (4 H, m); 10.82 (1 H, bs).

**4-Dimethylamino-4-m-tolyl-cyclohexanon (Ket-8)**

Dimethyl- (8- m- tolyl- 1, 4- dioxa- spiro [4.5] dec- 8- yl)- amin Hydrochlorid (2.76 g, 10 mmol) wurde in Wasser (4.2 ml) gelöst, mit konzentrierter Salzsäure (6.15 ml) versetzt und 76 h bei RT gerührt.
Die Lösung wurde mit Ether extrahiert (2 x 25 ml), die Etherphase wurde verworfen. Zu der wässrigen Lösung wurde 5N NaOH getropft bis sie alkalisch war. Anschließend wurde mit Dichlormethan (3 x 25 ml) extrahiert, die organische Phase mit Wasser nachgewaschen (25ml), über $Na_2SO_4$ getrocknet und eingeengt.
Ausbeute **Ket-8:** 1.69 g (73 %), gelbes Öl
1 H-NMR (DMSO-d6): 2.05 (10 H, m); 2.35 (3 H, s); 2.52 (2 H, m); 2.62 (2 H, m); 7.12 (1 H, m); 7.23 (3 H, m).

**Baustein Ket-9:**

*Variante 1:* **(8-Butyl-1,4-dioxa-spiro[4.5]dec-8-yl)-dimethyl-amin Hydrochlorid**

8- Dimethylamino- 1, 4- dioxa- spiro [4.5] decan- 8- carbonitril (10.5 g, 50 mmol) wurde in THF (150 ml) unter Eiskühlung und Argon vorgelegt. Innerhalb von 15 min wurde 2M Butylmagnesiumchlorid in THF (62.5 ml, 125 mmol)

zugetropft und 16 h bei RT gerührt.

Der Ansatz wurde unter Eiskühlung mit 20 %-iger Ammoniumchlorid-Lösung (37 ml) und Wasser (50 ml) versetzt und mit Ether (3 x 50 ml) extrahiert. Die org. Phase wurde mit Wasser (1 x 50 ml) und gesättigte Natriumchlorid-Lösung (1 x 50 ml) gewaschen, die organische Phase mit $Na_2SO_4$ getrocknet und i. Vak. eingeengt.

Das Rohprodukt (2.05 g) wurde in Ethylmethylketon (75 ml) gelöst, unter Eiskühlung mit $ClSiMe_3$ (9.5 ml, 75 mmol) versetzt und 6 h bei RT gerührt. Der ausgefallene weiße Niederschlag wurde abgesaugt und i. Vak. getrocknet.
*Ausbeute:* 3.1 g (22 %)
*$^1H$-NMR (DMSO-$d_6$):* 0.91 (3 H, t); 1.31 (4 H, m); 1.56 (2 H, m); 1.75 (8 H, m); 2.64 (6 H, s); 3.87 (4 H, s); 9.87 (1 H, s).

*Variante 1:*

**4-Butyl-4-dimethylamino-cyclohexanon (Ket-9)**

8- Butyl- 1, 4- dioxa- spiro [4.5] dec- 8- yl)- dimethyl- amin Hydrochlorid (3.10 g, 11.1 mmol) wurde in $H_2O$ (4.7 ml) und konz. HCl (7 ml) vorgelegt und 24 h bei RT gerührt. Der Ansatz wurde mit Ether (1 x 15 ml) extrahiert, die wässrige Phase unter Eiskühlung mit 5N NaOH alkalisch eingestellt und mit Dichlormethan (3 x 20 ml) extrahiert. Die org. Phase wurde über $Na_2SO_4$ getrocknet und i. Vak..eingeengt.
*Ausbeute:* 1.96 g (89 %), Öl
*$^1H$- NMR (DMSO- $d_6$) :* 0.88 (3 H, t) ; 1.23 (4 H, m) ; 1.40 (2 H, m) ; 1.68 (2 H, m) ; 1.91 (2 H, m) ; 2.31 (2 H, m) ; 2.22 (6 H, s) ; 2.42 (2 H, m) .
*$^{13}C$- NMR (DMSO- $d_6$) :* 13.91; 23.21; 26.06; 29.53; 31.07; 37.04; 38.88; 55.36; 210.37.

*Variante 2:*

**(8-Butyl-1,4-dioxa-spiro[4.5]dec-8-yl)-dimethyl-amin Hydrochlorid**

Zu einer Lösung von 8- Dimethylamino- 1, 4- dioxa- spiro [4.5] decan- 8- carbonitril (38, 3 g, 0, 182 mol) in abs. Tetrahydrofuran (420 ml) wurde unter Kühlung mit einer Eis- Kochsalzmischung langsam 2M *n*- Butylmagnesiumchlorid-Lösung in THF (228 ml, 0, 456 mol) unter Argon hinzugefügt. Dabei sollte die Reaktionstemperatur nicht über 10°C steigen. Anschließend wurde 16 h bei Raumtemperatur gerührt. Es entstand eine braune klare Lösung. Zur Aufarbeitung des Reaktionsgemisches wurde unter Eiskühlung (0 bis 10 °C) gesättigte Ammoniumchloridlösung (150 ml) zugetropft. Dabei entstand ein weißer Feststoff, der durch Zugabe von Wasser (ungefähr 250 ml) gelöst wurde. Die Reaktionsmischung wurde mit Diethylether (4 × 100 ml) extrahiert. Die organische Phase wurde mit Wasser (100 ml) und gesättigter NaCl- Lösung (100 ml) gewaschen, getrocknet und eingeengt. Es blieb ein gelbes Öl (44, 5 g) zurück, das außer der gewünschten Butylverbindung noch das Edukt Nitril enthielt. Das Rohprodukt wurde in Ethylmethylketon (275 ml) gelöst, unter Eiskühlung mit $ClSiMe_3$ (32 ml, 0, 245 mol) versetzt und im offenen Kolben bei Raumtemperatur gerührt. Das Hydrochlorid wurde durch mehrmalige Filtration im Abstand von 2 h abgetrennt. Nach einer Reaktionszeit von 6- 8 h konnte (8- Butyl- 1, 4- dioxa- spiro [4.5] dec- 8- yl)- dimethyl- amin Hydrochlorid in einer Ausbeute von 82 % (41, 8 g) als weißer Feststoff isoliert werden.

*Variante 2:*

**4-Butyl-4-dimethylamino-cyclohexanon (Ket-9)**

(8- Butyl- 1, 4- dioxa- spiro [4.5] dec- 8- yl)- dimethyl- amin Hydrochlorid (41, 8 g, 0, 15 mmol) wurde in Wasser (78 ml) gelöst und unter Rühren und Eiskühlung mit 37- proz. Salzsäure (100 ml, 1, 2 mol) versetzt. Die klare Reaktionsmischung wurde 7 Tage bei Raumtemperatur gerührt. Nach beendeter Hydrolyse wurde die Reaktionsmischung mit Diethylether extrahiert (2 × 70 ml) . Die organischen Extrakte wurden verworfen. Die wäßrige Phase wurde unter Eiskühlung mit 5N Natronlauge alkalisch gemacht (ungefähr 250 ml) und kräftig gerührt. Die Lösung wurde mit Diethylether extrahiert (3 × 100 ml) . Die vereinigten organischen Extrakte wurden mit Wasser (2 × 70 ml) gewaschen, getrocknet und eingeengt. 4- Butyl- 4- dimethylamino- cyclohexanon **(Ket- 9)** wurde als hellbraunes Öl in einer Ausbeute von 96 % (28, 4 g) gewonnen. Die Ausbeute an Keton- bezogen auf das in der ersten Stufe eingesetzte Ketal- betrug 75 %.

**Baustein Ket-10:**

**Dimethyl-(8-phenethyl-1,4-dioxa-spiro[4.5]dec-8-yl)amin-hydrochlorid**

Zu einer Lösung von 8- Dimethylamino- 1, 4- dioxa- spiro [4.5] decan- 8- carbonitril (39 g, 186 mmol) in THF

(300 ml) wurde unter Argon und Eiskühlung innerhalb von 15 min 1 M 2- Phenylethylmagnesiumchlorid- Lösung in THF (550 ml, 550 mmol) hinzugefügt und dann 16 h bei Raumtemperatur gerührt. Zur Aufarbeitung des Reaktionsgemisches wurde unter Eiskühlung gesättigte Ammoniumchloridlösung (295 ml) und Wasser (120 ml) zugegeben und mit Diethylether (3 × 150 ml) extrahiert. Die organische Phase wurde mit Wasser (100 ml) und gesättigter NaCl- Lösung (100 ml) gewaschen und anschließend eingeengt. Es blieb ein braunes Öl (60, 4 g) zurück. Das Rohprodukt wurde in Ethylmethylketon (310 ml) gelöst und unter Eiskühlung mit $ClSiMe_3$ (35, 6 ml, 282 mmol) versetzt. Nach 16 h bei RT wurde der entstandene Feststoff abgesaugt und mit Ethylmethylketon gewaschen.
Ausbeute : 50 g (83 %) .
Schmelzpunkt: 275- 278 °C.

**Dimethylamino-4-phenethylcyclohexanon (Ket-10)**

Dimethyl- (8- phenethyl- 1, 4- dioxa- spiro [4.5] dec- 8- yl) amin- hydrochlorid (50 g, 154 mmol) wurde in Wasser (60 ml) gelöst, mit konzentrierter Salzsäure (97, 2 ml, 3, 16 mol) versetzt und 4 d bei Raumtemperatur gerührt. Nach beendeter Hydrolyse wurde die Reaktionsmischung mit Diethylether extrahiert (2 × 100 ml) und die wäßrige Phase unter Eiskühlung mit 5N Natronlauge alkalisch gestellt, wobei ein Feststoff ausfiel. Dieser wurde abgesaugt, mit $H_2O$ (3 × 20 ml) gewaschen und anschließend getrocknet.
Ausbeute **Ket- 10**: 25, 3 g (67 %), gelber Feststoff.
Schmelzpunkt: 60 °C.

**Baustein Ket-12:**

*Dieser Baustein wurde unter den angegebenen Reaktionsbedingungen anstatt des gewünschten Zielproduktes erhalten. Es ist offensichtlich, dass (8- Ethyl- 1, 4- dioxa- spiro [4.5] dec- 8- yl)- dimethyl- aminhydrochlorid auch gezielt aus Ethylmagnesiumbromid und 8- Dimethylamino- 1, 4- dioxa- spiro [4.5] decan- 8- carbonitril hergestellt werden kann.*

**(8- Ethyl- 1, 4- dioxa- spiro [4.5] dec- 8- yl)- dimethyl- aminhydrochlorid** Eine Mischung von Ethylbromid (30.0 g, 0.3 mol) und 3- Brompyridin (16.0 g, 0.1 mol) wurde tropfenweise zu Magnesiumpulver (10.0 g) in Diethylether (50 ml) gegeben. Nach dem die Grignardbildung abgeschlossen war, wurde die graue Lösung bei 0 °C innerhalb von 15 min mit 8- Dimethylamino- 1, 4- dioxa- spiro [4.5] decan- 8- carbonitril (10.5 g, 47.6 mmol) in THF (80 ml) versetzt und die Reaktionslösung über Nacht bei RT gerührt. Anschließend wurde die Reaktionslösung unter Eiskühlung mit 20 %iger Ammoniumchlorid- Lösung (50 ml) und Wasser (50 ml) versetzt. Die Reaktionslösung wurde mit Diethylether (100 ml) verdünnt, die org. Phase abgetrennt und die wässr. Phase 2 x mit $Et_2O$ (100 ml) extrahiert. Die vereinten org. Phasen wurden mit Wasser (50 ml) und NaCl- Lösung (50 ml) gewaschen, über $Na_2SO_4$ getrocknet, filtriert und das Solvens i. Vak. entfernt. Der Rückstand wurde in 2- Butanon (200 ml) aufgenommen und bei 0 °C mit $Me_3SiCl$ (10 ml) versetzt. Die Reaktionslösung wurde unter Luftfeuchtigkeit 5 h gerührt und der ausgefallene Feststoff abgesaugt.
*Ausbeute:* 6.8 g (64 %), hellbrauner Feststoff
*$^1H$- NMR (DMSO- $d_6$) :* 0.94 (3 H, t) ; 1.51- 1.60 (2 H, m) ; 1.77- 1.86 (8 H, m) ; 2.64 (6 H, 2 s) ; 3.83- 3.89 (4 H, m) .

**4- Dimethylamino- 4- ethyl- cyclohexanon (Ket- 12)** (8- Ethyl- 1, 4- dioxa- spiro [4.5] dec- 8- yl)- dimethylamin hydrochlorid (6.67 g, 0.026 mmol) wurde in 6N HCl (40 ml) gelöst und bei Raumtemperatur über Nacht gerührt. Die Reaktionsmischung wurde zweimal mit Diethylether (100 ml) extrahiert. Anschließend wurde unter Eiskühlung mit 5N NaOH alkalisch gestellt und erneut dreimal mit $Et_2O$ (100 ml) extrahiert. Die vereinten org. Phasen wurden über $NaSO_4$ getrocknet, filtriert und das Solvens i. Vak. entfernt.
*Ausbeute:* 4.16 g (92 %), braunes Öl
*$^1H$- NMR (DMSO- $d_6$) :* 0.81 (3 H, t) ; 1.43- 1.50 (2 H, q) ; 1.67- 1.89 (2 H, m) ; 1.83- 1.89 (2 H, m) ; 1.99- 2.06 (2 H, m) ; 2.22 (6 H, 2 s) ; 2.39- 2.43 (4 H, m) .
*$^{13}C$- NMR (DMSO- $d_6$) :* 8.71; 21.99; 30.41; 36.17; 37.07; 38.66; 55.53; 210.57.

**Baustein Ket-13:**

**4-(8-Benzyl-1,4-dioxaspiro[4.5]dec-8-yl)morpholin**

In einem ausgeheizten Kolben wurde eine Lösung von Morpholin (958 mg, 0.96 ml, 11 mmol), 1, 4- Dioxaspiro [4.5] dec- 8- on (1.56 g, 10 mmol) und 1, 2, 3- Triazol (829 mg, 12 mmol) in Toluol (10 ml) 6 h unter Rückfluss am Wasserabscheider erhitzt. Anschließend wurde diese Lösung unter Argon zu einer 2 *M* Lösung von Benzylmagnesiumchlorid in Tetrahydrofuran (20 ml, 40 mmol) so getropft, dass die Innentemperatur unter 24°C blieb. Das Gemisch wurde 2 h bei Raumtemperatur gerührt und danach unter Eis- Wasser- Kühlung zu 20 % Ammoniumchlorid- Lösung (25 ml) getropft. Die organische Phase wurde abgetrennt und die wässrige Phase mit Diethylether (3 × 20 ml) extrahiert. Die vereinigten organischen Phasen wurden mit 2 *N* Natronlauge (40 ml) und Wasser (40 ml) gewaschen, mit Natriumsulfat

getrocknet und i. Vak. eingeengt. Das Rohprodukt (4 g) wurde durch Flashchromatographie (400 g, 20 × 7.5 cm) mit Ethylacetat / Cyclohexan (1: 3) gereinigt.
Ausbeute: 2.87 g (90 %), weiße Kristalle
Schmelzpunkt: 97- 101 °C
$^1$H- NMR ($CDCl_3$) : 1.35- 1.52 (m, 4H) ; 1.72- 1.96 (m, 4H) ; 2.61- 2.66 (m, 4H) ; 2.67 (s, 2H) ; 3.68- 3.75 (m, 4H) ; 3.78- 3.92 (m, 4H) ; 7.08- 7.34 (m, 5H) .

**4-Benzyl-4-morpholin-4-ylcyclohexanon (Ket-13)**

Zu einer Lösung von 4- (8- Benzyl- 1, 4- dioxaspiro [4.5] dec- 8- yl) morpholin (1.00 g, 3.15 mmol) in Aceton (5 ml) wurde 6 *M* Salzsäure (5 ml) gegeben. Nach 24 h wurde die Reaktionslösung mit weiterer 6 *M* Salzsäure (2.5 ml) versetzt, weitere 3 d bei Raumtemperatur gerührt, anschließend mit 25 % Kaliumcarbonat- Lösung alkalisch (pH~9) gestellt und mit Diethylether (3 × 20 ml) extrahiert. Die vereinigten organischen Phasen wurden mit Natriumsulfat getrocknet und i. Vak. eingeengt.
Ausbeute: 753 mg (87 %), weißer Feststoff **(Ket- 13)**
Schmelzpunkt: 124- 127 °C
$^1$H- NMR ($CDCl_3$) : 1.47- 1.72 (m, 2 H) ; 1.98- 2.14 (m, 4H) ; 2.48- 2.68 (m, 2H) ; 2.70- 2.77 (m, 2H) ; 2.78 (s, 4H) ; 3.72- 3.81 (s, 4H) ; 7.12- 7.36 (m, 5H) .

**<u>Baustein Ket-14:</u>**

**1-Chlor-3-methoxy-propan**

(Letsinger; Schnizer; J. Org. Chem.; 16; 1951; 704, 706)
3- Methoxypropan- 1- ol (47.1 g, 50 ml, 0.523 mol) wurde in Pyridin (41.3 g, 42.6 ml, 0.523 mol) gelöst, auf 10°C abgekühlt und bei 10- 30°C unter kräftigem Rühren tropfenweise mit Thionylchlorid (93.3 g, 56.9 ml, 0.784 mol) versetzt. Dabei fiel ein fester Niederschlag aus, die Mischung wurde dann 3 h bei 65 ° C nachgerührt.
Der Ansatz wurde auf ein Gemisch aus Eis (130 g) und konz. HCl (26 ml) gegossen. Die wässrige Lösung wurde mit Ether (2 x 20 ml) extrahiert und die vereinigten organischen Phasen mit $K_2CO_3$- Lösung gewaschen. Bei Zugabe des Trocknungsmittels $K_2CO_3$ war starke Gasbildung zu beobachten, daher wurde die Lösung über Nacht stehen gelassen.
Das Trockenmittel wurde abfiltriert und die organische Phase mit $K_2CO_3$- Lösung bis zur alkalischen Reaktion gewaschen. Die organische Phase wurde abgetrennt, mit Wasser gewaschen und über $K_2CO_3$ getrocknet, filtriert und bei Normaldruck destilliert.
*Siedepunkt:* 113°C
Ausbeute: 41.2 g (72 %), farblose Flüssigkeit
*$^1$H- NMR (DMSO- $d_6$)* : 1.93 (2 H, m) ; 3.23 (3 H; s) ; 3.44 (2 H, t) ; 3.66 (2 H, t) .

**[8-(3-Methoxy-propyl)-1,4-dioxa-spiro[4.5]dec-8-yl]-dimethyl-amin**

Magnesium (10.0 g, 92 mmol) und $I_2$ in abs. Diethylether (30 ml) wurden unter Argonatmosphäre und zeitweiliger Erwärmung tropfenweise mit einer Lösung von 1- Chlor- 3- methoxy- propan (10.0 g, 92 mmol) in abs. Ether (15 ml) versetzt. Anschließend wurde der Ansatz unter Rückfluss 60 min nachgerührt, das Magnesium war danach nicht vollständig aufgelöst.
Unter Eiskühlung wurde eine Lösung von 8- Dimethylamino- 1, 4- dioxa- spiro [4.5] decan- 8- carbonitril (9.68 g, 46 mmol) in abs. THF (30 ml) zugetropft. Dabei fiel ein zäher Niederschlag aus, zur besseren Durchmischung wurden 100 ml abs. THF nachgegeben. Die Mischung wurde bei Raumtemperatur 24 h gerührt.
Der Ansatz wurde unter Eiskühlung mit 20%-iger $NH_4Cl$-Lösung (100 ml) und Wasser (120 ml) versetzt, die organische Phase wurde abgetrennt und die wässrige Phase mit Ether (3 x 120 ml) extrahiert.
Die vereinigten organischen Phasen wurden mit gesättigter NaCl-Lösung (120 ml) und Wasser (120 ml) gewaschen, über $Na_2SO_4$ getrocknet und i. Vak. eingeengt. Die Rohausbeute betrug 10.8 g braunes Öl.
9.8 g Rohprodukt wurden durch Flash-Chromatographie mit $CHCl_3$/MeOH (50:1 ⇒ 20:1 ⇒ 9:1) gereinigt.
*Ausbeute:* 8.11 g (75 %), gelbes Öl
*$^1$H-NMR (DMSO-$d_6$):* 1.44 (8 H, m); 1.62 (4 H; m); 2.25 (6 H, s); 3.21 (3 H, s); 3.31 (2 H, m); 3.82 (4 H, s).
*$^{13}$C-NMR (DMSO-$d_6$):* 23.99; 26.52; 28.87; 29.88; 36.97; 55.24: 57.67; 63.40; 72.62; 108.07.

**4-Dimethylamino-4-(3-methoxy-propyl)-cyclohexanon (Ket-14)**

[8- (3- Methoxy- propyl)- 1, 4- dioxa- spiro [4.5] dec- 8- yl]- dimethyl- amin (8.11 g, 31.5 mmol) wurde in Wasser (12 ml) gelöst, unter Eiskühlung mit konz. HCl (19.5 ml) versetzt und 3 d bei Raumtemperatur gerührt. Die Reaktionsmischung wurde mit Ether (2 x 75 ml) gewaschen. Anschließend wurde die Lösung mit 5N NaOH alkalisch gestellt und mit Dichlormethan (3 x 75 ml) extrahiert. Die vereinigten organischen Phasen wurden mit Wasser (75 ml) gewaschen, über $Na_2SO_4$ getrocknet, filtriert und das Solvens i. Vak. entfernt.
*Ausbeute:* 6.03 g (90 %), gelbes Öl
*$^1$H- NMR (DMSO- $d_6$) :* 1.44 (4 H, m) ; 1.68 (2 H; m) ; 1.88 (2 H, m) ; 2.00 (1 H, m) ; 2.05 (1 H, m) ; 2.20 (6 H, s) ; 2.41 (2 H, m) ; 3.22 (3 H, s) ; 3.28 (2 H, m) .
*$^{13}$C- NMR (DMSO- $d_6$) :* 24.01; 26.34; 30.88; 36.15; 37.06; 55.26: 57.70; 72.55; 210.39.

**<u>Baustein Ket-15:</u>**

**8-Azetidin-1-yl-1,4-dioxaspiro[4.5]decan-8-carbonitril**

Zu einer Mischung von 4 *N* Salzsäure (8.1 ml), Methanol (4.9 ml) und Azetidin (8.5 g, 10 ml, 149 mmol) wurde unter Eiskühlung zuerst 1, 4- Dioxaspiro [4, 5] decan- 8- on (4.84 g, 31 mmol) und danach Kaliumcyanid (4.85 g, 74.4 mmol) in Wasser (15 ml) gegeben. Die Mischung wurde 5 d bei Raumtemperatur gerührt, dann mit Wasser (50 ml) versetzt und mit Diethylether (3 × 50 ml) extrahiert. Die vereinigten organischen Phasen wurden mit Natriumsulfat getrocknet und i. Vak. eingeengt.
Ausbeute: 6.77 g (98 %), Öl
$^1$H- NMR (DMSO- $d_6$) : 1.45- 1.63 (m, 4H) ; 1.67- 1.82 (m, 4H) ; 1.99 (q, 2H, J = 7.1 Hz) ; 3.21 (t, 4H, J = 7.1 Hz) ; 3.86 (s, 4H) .

**1-(8-Phenyl-1,4-dioxaspiro[4.5]dec-8-yl)azetidin**

Eine 2 *M* Lösung von Phenylmagnesiumchlorid in Tetrahydrofuran (12 ml, 24 mmol) wurde unter Argon und Eiskühlung tropfenweise mit einer Lösung des soeben hergestellten Nitrils (2.20 g, 9.9 mmol) in wasserfreiem Tetrahydrofuran (25 ml) versetzt und danach bei Raumtemperatur über Nacht gerührt. Nach Zugabe von gesättigter Ammoniumchloridlösung (5 ml) und Wasser (5 ml) wurden die Phasen getrennt und die wässrige mit Diethylether (3 × 50 ml) extrahiert. Die vereinigten organischen Phasen wurden mit Natriumsulfat getrocknet und i. Vak. eingeengt. Das Rohprodukt wurde durch Flashchromatographie (100 g, 20 × 4.0 cm) mit Ethylacetat / Cyclohexan (1:1) gereinigt.
Ausbeute: 670 mg (25 %), farbloses Öl
$^1$H-NMR (DMSO-$d_6$): 1.27-1.40 (m, 2H); 1.55-2.00 (m, 8H); 2.86 (t, 4H, J = 6.8 Hz); 3.76-3.89 (m, 4H); 7.24-7.45 (m, 5H).

**4-Azetidin-1-yl-4-phenylcyclohexanon (Ket-15)**

Eine Lösung des soeben hergestellten Acetals (370 mg, 1.3 mmol) in Aceton (30 ml) wurde mit 6 *N* Salzsäure (2 ml) versetzt und über Nacht bei Raumtemperatur gerührt. Durch Zugabe von 5 *N* Natronlauge wurde pH 10 eingestellt und die wässrige Phase mit Dichlormethan (3 × 20 ml) extrahiert. Die vereinigten organischen Phasen wurden mit Natriumsulfat getrocknet und i. Vak. eingeengt.
Ausbeute: 274 mg (92 %), weißer Feststoff **(Ket- 15)**
Schmelzpunkt: nicht bestimmbar
$^1$H- NMR (DMSO- $d_6$) : 1.67 (td, 2H, J = 13.8, 6.9 Hz) ; 1.95- 2.13 (m, 4H) ; 2.20- 2.33 (m, 2H) ; 2.40- 2.47 (m, 1 H) ; 2.52- 2.57 (m, 1H) ; 2.94 (t, 4H; J = 6.9 Hz) ; 7.28- 7.47 (m, 5H) .

**<u>Baustein Ket-16:</u>**

**1-(8-Pyrrolidin-1-yl-1,4-dioxaspiro[4.5]dec-8-yl)-1H-[1,2,3]triazol**

Zu einer Lösung von 1, 4 Dioxaspiro [4, 5] decan- 8.on (3.9 g, 25 mmol) in Toluol (40 ml) wurden Pyrrolidin (1.95 g, 2.29 ml, 27.5 mmol), 1, 2, 3- Triazol (2.07 g, 30 mmol) und Molsieb 4 Å (7.14 g) gegeben. Das Gemisch wurde 7 h bei 90 °C gerührt. Anschließend wurde die Lösung dekantiert und sofort weiter umgesetzt.

**1-(8-Butyl-1,4-dioxaspiro[4.5]dec-8-yl)pyrrolidin**

Zu einer 2 *M* Lösung von *n*-Butylmagnesiumchlorid (25 ml, 50 mmol) in Tetrahydrofuran wurde unter Eiskühlung und Argon die Reaktionslösung soeben hergestellten Triazol Derivates (ca. 6.9 g, 25 mmol) in Toluol (38 ml) getropft.

Das Reaktionsgemisch wurde über Nacht bei Raumtemperatur gerührt und anschließend in gesättigte Ammoniumchloridlösung (60 ml) gegossen. Die Phasen wurden getrennt und die wässrige mit Diethylether (3 × 70 ml) extrahiert. Die vereinigten organischen Phasen wurden mit Natriumsulfat getrocknet, i. Vak. eingeengt und der Rückstand (12 g) durch Flashchromatographie (400 g, 20 x 7.6 cm) mit Ethylacetat / Methanol (9:1) gereinigt.
Ausbeute: 2.70 g (40 % über zwei Stufen), braunes Öl
$^1$H-NMR (DMSO-$d_6$): 0.87 (t, 3H, J = 7.1 Hz); 1.12-1-29 (m, 4H); 1.30-1.45 (m, 4H); 1.46-1.60 (m, 4H); 1.61-1.75 (m, 6H); 1.93 (t, 1 H, J = 7.1 Hz); 2.36 (t, 1 H, J = 7.0 Hz), 2.58 (br s, 2H), 3.83 (s, 4H).

**4-Butyl-4-pyrrolidin-1-yl-cyclohexanon (Ket-16)**

Eine Lösung des soeben erhaltenen Acetals (2.70 g, 10.1 mmol) in Aceton (100 ml) wurde mit Wasser (10.0 ml) und 37 % Salzsäure (14.0 ml) versetzt und über Nacht bei Raumtemperatur gerührt. Zum Gemisch wurde anschließend langsam 4 *M* Natronlauge getropft, bis pH 10 erreicht war. Das Gemisch wurde mit Diethylether (4 × 40 ml) extrahiert, die vereinigten organischen Phasen wurden mit Natriumsulfat getrocknet und i. Vak. eingeengt. Das Rohprodukt (2.6 g) wurde durch Flashchromatographie (260 g, 30 × 5.6 cm) mit Ethylacetat / Methanol (9: 1) gereinigt.
Ausbeute: 1.06 g (47 %), braunes Öl (**Ket- 16**)
$^1$H- NMR (DMSO- $d_6$) : 0.88 (t, 3H, J = 6.7 Hz) ; 1.14- 1.34 (m, 4H) ; 1.40- 1.50 (m, 2H) ; 1.62- 1.88 (m, 8H) ; 2.04 (dt, 2H, J = 15.0, 3.9 Hz) ; 2.42 (ddd, 2H, J = 6.3, 11.8, 15.5 Hz) ; 2.63 (t, 4H, J = 6.0 Hz) .

**Baustein Ket-17:**

**Methyl-[8-(2-methyl-2*H*-[1,2,4]triazol-3-yl)-1,4-dioxa-spiro[4.5]dec-8-yl]-amin**

Unter Argonatmosphäre wurde Butyllithium (2.5 M in Hexan, 9.2 ml, 23.0 mmol) vorgelegt und auf- 78°C abgekühlt. 1- Methyl- 1, 2, 4- triazol (1.30 ml, 23 mmol) wurde in abs. Tetrahydrofuran (60.0 ml) gelöst und tropfenweise unter Eiskühlung bei- 78°C zugegeben. Anschließend wurde bei dieserTemperatur 10 min nachgerührt. Zu der dargestellten Lösung wurde 8- Methylamino- 1, 4- dioxa- spiro [4.5] decan- 8- carbonitril (2.12 g, 10.8 mmol) in abs. Tetrahydrofuran (15 ml) im Kältebad bei- 78°C schnell zugetropft. Nach der Zugabe wurde die Reaktionslösung 1 h im Kältebad nachgerührt und anschließend langsam auf 0 °C erwärmt. Bei Raumtemperatur wurde das Reaktionsgemisch über Nacht gerührt. Anschließend wurde bei 0 °C mit Wasser (10 ml) hydrolysiert, die wässrige Phase mit Chloroform (3 x 50 ml) extrahiert, die organische Phase mit Wasser (50 ml) und gesättigter NaCl- Lösung (50 ml) gewaschen, über $Na_2SO_4$ getrocknet und i. Vak. eingeengt.
Das Produkt wurde mittels Flash-Chromatographie mit Chloroform/Methanol (15:1) gereinigt. **Ausbeute**: 1.93 g (71 %)
$^1$H-NMR (DMSO-$d_6$): 1.54 (2 H, m); 1.72 (2 H, m); 1.91 (5 H, m); 2.10 (2 H, m); 3.84 (4 H, m); 4.01 (3 H, s); 7.74 (1 H, s).

**4-Methylamino-4-(2-methyl-2*H*-[1,2,4]triazol-3-yl)-cyclohexanon** (Ket-17)

Methyl- [8- (2- methyl- 2*H*- [1, 2, 4] triazol- 3- yl)- 1, 4- dioxaspiro [4.5] dec- 8- yl]- amin (4, 2 g, 16, 646 mmol) wurde bei Raumtemperatur mit 5- proz. Schwefelsäure (330 ml) versetzt und 3 d bei Raumtemperatur gerührt. Der Reaktionsansatz wurde mit Ether (120 ml) versetzt. Die Phasen wurden getrennt. Die wäßrige Phase wurde mit 5N NaOH alkalisch gemacht und mit Dichlormethan (4 × 50 ml) extrahiert. Die organische Phase wurde über Natriumsulfat getrocknet und im Vakuum zur Trockene eingeengt. Das Produkt konnte als farbloser kristalliner Feststoff in einer Ausbeute von 83 % (2, 89 g, 13, 862 mmol) erhalten werden.

**Indolbausteine**

**Bausteinsynthese:**

Die Synthesen der Iodopyridinamine sind literaturbekannt und durch Orthometallierung der entsprechenden Pivaloyl-geschützten Aminopyridine in einer dreistufigen Sequenz darstellbar: J. A. Turner, J. Org. Chem. 1983, 48, 3401; L. Estel, F. Marsais, G. Quéguiner, J. Org. Chem. 1988, 53, 2740; J. Malm, B. Rehn, A.-B. Hörnfeldt, S. Gronowitz, J. Het. Chem. 1994, 31, 11.
Die Synthese von 4- (Triethylsilyl) but- 3- in- 1- ol ist in der Literatur beschrieben und analog der folgenden Vorschriften durchgeführt worden: B. C. Bishop, I.F. Cottrell, D. Hands Synthesis, 1997, 1315; C. Cheng, D. R. Lieberman, R. D. Larsen, R. A. Reamer, T. R. Verhoeven, P. J. Reider Tet. Lett., 1994, 6981.
Die Azatryptophole wurden durch Palladium-vermittelte Larock-Heteroannelierung hergestellt, die Triethylsilylgeschützten Vorstufen sind literaturbekannt: F. Ujjainwalla, D. Warner Tet. Lett., 1998, 5355.

**Allgemeine Bausteine:**

**Triethyl(4-(triethylsilyl)but-3-inyloxy)silan**

3- Butin- 1- ol (34, 99 g, 0, 50 mol) wurde in THF (1, 2 l) unter Stickstoff gelöst und auf- 30°C gekühlt. Zu dieser Lösung wurde innerhalb von 15 min *n*- BuLi (640 ml, 1, 02 mol, 1, 6 M Lösung in *n*- Hexan) so hinzugetropft, dass die Temperatur- 20°C nicht überstieg. Nach 1 h bei- 20°C wurde eine Lösung von Triethylchlorsilan (171, 4 ml, 1, 02 mol) in THF (300 ml) innerhalb von 30 min tropfenweise zugegeben. Das Kältebad wurde entfernt und die Reaktionslösung für 15 h bei RT gerührt. Unter Eisbadkühlung wurde das Reaktionsgemisch mit wässriger $Na_2CO_3$- Lösung (1%iger) gequenscht und mit Hexan (2x 500 ml) extrahiert. Die vereinigten organischen Phasen wurden mit gesättigter NaCl-Lösung (300 ml) gewaschen und über $MgSO_4$ getrocknet. Nach Filtration des Trockenmittels wurden die Lösungsmittel am Rotationsverdampfer entfernt und der Rückstand destillativ (p=0, 05 mbar, Kopftemperatur=115- 110°C) gereinigt und Triethyl (4- (triethylsilyl) but- 3- inyloxy) silan (88, 9 g, 60 %) erhalten.

**4-(Triethylsilyl)but-3-in-1-ol**

Triethyl (4- (triethylsilyl) but- 3- inyloxy) silan (32, 99 g, 110, 66 mmol) wurde in MeOH (336 ml) gelöst, mit 2N HCl (62 ml) versetzt und das Gemisch 4 h bei RT gerührt. Anschließend wurde der Lösung Hexan (250 ml) und $H_2O$ (200 ml) hinzugefügt und die wässrige Phase mit Hexan (3x 100 ml) extrahiert. Die vereinigten organischen Phasen wurden mit $H_2O$ (100 ml) und anschließend mit gesättigter NaCl- Lösung (50 ml) gewaschen und über $MgSO_4$ getrocknet. Nach Filtration des Trockenmittels wurden die Lösungsmittel am Rotationsverdampfer entfernt. Säulenchromatographie des Rückstands (Hexan/ Ether=4: 1, dann Ether) lieferte 4- (Triethylsilyl) but- 3- in- 1- ol (19, 6 g, 96 %) als farbloses Öl.

**Baustein Ind-1**

**N-(Pyridin-2-yl)pivalamid**

Unter Stickstoff wurde 2- Aminopyridin (25, 00 g, 265, 6 mmol) in DCM (425 ml) aufgenommen und mit $NEt_3$ (46, 00 ml, 332 mmol) versetzt. Die Lösung wurde auf- 5°C gekühlt, mit einer Lösung von Trimethylacetylchlorid (35, 95 ml, 292, 20 mmol) in DCM (50 ml) tropfenweise versetzt und weitere 15 min bei- 5°C gerührt. Anschließend wurde das Gemisch 2 h bei RT gerührt. Die Suspension wurde mit $H_2O$ (200 ml), dann mit verdünnter $NaHCO_3$- Lösung gewaschen und die organische Phase über $MgSO_4$ getrocknet. Nach Filtration des Trockenmittels und Entfernung des Lösungsmittels am Rotationsverdampfer wurde der Rückstand (48, 30 g) aus Hexan (100 ml) in der Siedehitze umkristallisiert. Es wurde N- (Pyridin- 2- yl) pivalamid (42, 80 g, 91 %) in Form von farblosen Kristallen erhalten.

**N-(3-Iodpyridin-2-yl)pivalamid**

N- (Pyridin- 2- yl) pivalamid (14, 25 g, 80 mmol) und TMEDA (29, 80 ml, 200 mmol) wurden in THF (400 ml) unter Stickstoff gelöst und bei- 75°C tropfenweise mit n- BuLi (125 ml, 200 mmol; 1, 6 M Lösung in n- Hexan) versetzt. Das Gemisch wurde 15 min bei- 75°C, anschließend 2 h bei- 10°C gerührt. Nach erneuter Kühlung auf- 75°C wurde eine Lösung von Iod (50, 76 g, 200 mmol) in THF (200 ml) zugetropft und das Reaktionsgemisch für 2 h gerührt. Es wurde auf 0°C erwärmt und mit gesättigter wässriger Natriumthiosulfat- Lösung gequenscht. Die wässrige Phase wurde mit DCM (2x 150 ml) extrahiert und die vereinigten organischen Phasen über $MgSO_4$ getrocknet. Nach Filtration des Trockenmittels und Entfernung des Lösungsmittels am Rotationsverdampfer wurde der Rückstand mittels Säulenchromatographie (Ether: Cyclohexan= 3: 1) gereinigt und so N- (3- Iodpyridin- 2- yl) pivalamid (14, 80 g, 61 %) erhalten.

**3-Iodpyridin-2-amin**

N- (3- Iodpyridin- 2- yl) pivalamid (13, 80 g, 45, 36 mmol) wurde in $H_2SO_4$ (24 gew%, 394 ml) aufgenommen und das Gemisch 60 min am Rückfluss gerührt. Nach Abkühlen auf RT wurde mit 4 N NaOH und festem $NaHCO_3$ neutralisiert, die wässrige Phase mit DCM (3x 200 ml ) extrahiert und die vereinigten organischen Phasen über $MgSO_4$ getrocknet. Nach Filtration wurde das Lösungsmittel am Rotationsverdampfer entfernt. Es resultierte 3- Iodpyridin- 2-amin (9, 70 g, 97 %) als cremefarbener Feststoff.

**2-(2-(Triethylsilyl)-1H-pyrrolo[2,3-b]pyridin-3-yl)ethanol**

Unter Stickstoff wurde eine Mischung aus 3- Iodpyridin- 2- amin (0, 46 g, 2, 09 mmol), 4- (Triethylsilyl) but- 3-in- 1- ol (0, 58 g, 3, 14 mmol), 1, 1'- Bis (diphenylphosphino) ferrocen- palladium (II) dichlorid- dichloromethan (0, 083

g, 0, 105 mmol), Lithiumchlorid (0, 086 g, 2, 09 mmol) und Natriumcarbonat (0, 44 g, 4, 18 mmol) in DMF (21 ml) 15 h bei 100°C gerührt. Das Reaktionsgemisch wurde auf RT abgekühlt, mit EtOAc/ Ether (1: 1) versetzt und in $H_2O$ gegeben. Die zweiphasige Suspension wurde über Filtererde filtriert. Nach Trennung der Phasen wurde die wässrige Phase mit EtOAc (2x) extrahiert. Die organischen Phasen wurden vereinigt und mit $H_2O$ und mit gesättigter NaCl- Lösung gewaschen und über $MgSO_4$ getrocknet. Nach Filtration des Trockenmittels wurden die Lösungsmittel am Rotationsverdampfer entfernt. Säulenchromatographie des Rückstands (*n*- Hexan/ EtOAc= 2: 1, dann *n*- Hexan/ EtOAc= 1: 1) lieferte 2-(2- (Triethylsilyl)- 1H- pyrrolo [2, 3- b] pyridin- 3- yl) ethanol (0, 46 g, 80 %) .

**2-(1H-Pyrrolo[2,3-b]pyridin-3-yl)ethanol (Ind-1)**

2- (2- (Triethylsilyl)- 1H- pyrrolo [2, 3- b] pyridin- 3- yl) ethanol (1, 00 g, 3, 62 mmol) wurde mit TBAF (10, 86 ml, 10, 86 mmol; 1M- Lösung in THF) 6h bei 50°C und anschließend 10h bei RT gerührt. Das Lösungsmittel wurde am Rotationsverdampfer entfernt und der Rückstand säulenchromatographisch (DCM/ MeOH= 9: 1, dann 1: 1) gereinigt. Es resultierte 2- (1 H- Pyrrolo [2, 3- b] pyridin- 3- yl) ethanol (0, 46 g, 79 %) als weißer Feststoff.

**Baustein Ind-2**

**2-(1H-pyrrolo[2,3-c]pyridin-3-yl)ethanol (Ind-2)**

2- (2- (Triethylsilyl)- 1H- pyrrolo [2, 3- c] pyridin- 3- yl) ethanol (1, 99 g, 7, 24 mmol) wurde in THF (17 ml) gelöst und bei 0°C mit TBAF (7, 96 ml, 7, 96 mmol; 1 M Lösung in THF) versetzt. Das Gemisch wurde für 1 h bei 0 C gerührt und dann auf RT erwärmt. Es folgte eine erneute Zugabe von TBAF (7, 96 ml, 7, 96 mmol, 1 M Lösung in THF) und $H_2O$ (5 Tropfen) . Nach 2 d bei RT wurden mit 20 ml $H_2O$ zugesetzt und die wässrige Phase DCM (3 x 60 ml) extrahiert. Die organischen Phasen wurden vereinigt und mit gesättigter NaCl- Lösung (2x20 ml) gewaschen und über $MgSO_4$ getrocknet. Nach Filtration des Trockenmittels wurden die Lösungsmittel am Rotationsverdampfer entfernt und der Rückstand säulenchromatographisch (DCM/ MeOH= 4: 1) gereinigt. Es resultiert 2- (1H- pyrrolo [2, 3- c] pyridin- 3- yl) ethanol (0, 60 g, 51 %) als farbloses Öl, das mit der Zeit erstarrte.

**Baustein Ind-3**

**N-(5-(Trifluoromethyl)pyridin-2-yl)pivalamid**

Unter Stickstoff wurde 5- (Trifluoromethyl) pyridin- 2- amin (15 g, 92, 5 mmol) in DCM (190 ml) aufgenommen und mit $NEt_3$ (16 ml, 115, 7 mmol) versetzt. Die Lösung wurde auf- 5°C gekühlt, mit einer Lösung von Trimethylacetylchlorid (12, 5 ml, 101, 8 mmol) in DCM (65 ml) tropfenweise versetzt und weitere 15 min im Eisbad gerührt. Anschließend wurde das Gemisch 2 h bei RT gerührt. Die Suspension wurde mit $H_2O$ (150 ml), dann mit verdünnter $NaHCO_3$- Lösung gewaschen und die organische Phase über $MgSO_4$ getrocknet. Nach Filtration des Trockenmittels und Entfernung des Lösungsmittels am Rotationsverdampfer wurde der Rückstand kristallisiert. Es wurde N- (5- (trifluoromethyl) pyridin- 2-yl) pivalamid (20, 8 g, 91 ) erhalten.

**N-(3-Iodo-5-(trifluoromethyl)pyridin-2-yl)pivalamid**

N- (5- (trifluoromethyl) pyridin- 2- yl) pivalamid (22 g, 89, 4 mmol) und TMEDA (33, 3 ml, 223, 4 mmol) wurden in THF (400 ml) unter Stickstoff gelöst und bei- 75°C tropfenweise mit n- BuLi (139, 6 ml, 223, 4 mmol; 1, 6 M Lösung in *n*- Hexan) versetzt. Das Gemisch wurde 2 h bei- 75°C gerührt. Unter Beibehaltung der Temperatur wurde eine Lösung von Iod (56, 7 g, 223, 4 mmol) in THF (280 ml) zugetropft und das Reaktionsgemisch für 2 h gerührt. Es wurde auf 0°C erwärmt und mit gesättigter wässriger Natriumthiosulfat- Lösung gequencht. Die wässrige Phase wurde mit DCM (2x 150 ml) extrahiert und die vereinigten organischen Phasen über $MgSO_4$ getrocknet. Nach Filtration des Trockenmittels und Entfernung des Lösungsmittels am Rotationsverdampfer wurde der Rückstand mittels Säulenchromatographie (Ether: Cyclohexan= 1: 1) gereinigt und so N- (3- Iodo- 5- (trifluoromethyl) pyridin- 2- yl) pivalamid (13 g, 39, 1 %) erhalten.

**3-Iod-5-(trifluoromethyl)pyridin-2-amin**

N- (3- Iod- 5- (trifluoromethyl) pyridin- 2- yl) pivalamid (16, 8 g, 45, 1 mmol) wurde in $H_2SO_4$ (24 gew%, 392 ml) aufgenommen und das Gemisch 60 min am Rückfluss gerührt. Nach Abkühlen auf RT wurde mit 4 N NaOH und festem $NaHCO_3$ neutralisiert, die wässrige Phase mit DCM (3x 200 ml ) extrahiert und die vereinigten organischen Phasen über $MgSO_4$ getrocknet. Nach Filtration wurde das Lösungsmittel am Rotationsverdampfer entfernt. Es resultierte 3- Iodo- 5- (trifluoromethyl) pyridin- 2- amin (13 g, 100 %) als cremefarbener Feststoff.

**2-(2-(Triethylsilyl)-5-(trifluoromethyl)-1H-pyrrolo[2,3-b]pyridin-3-yl)ethanol**

Unter Stickstoff wurde eine Mischung aus 3- Iodo- 5- (trifluoromethyl) pyridin- 2- amin (13 g, 45, 1 mmol), 4- (Triethylsilyl) but- 3- in- 1- ol (12, 4 g, 67, 7 mmol), 1, 1'- Bis (diphenylphosphino) ferrocen- palladium (II) dichlorid-dichloromethan (1, 78 g, 2, 26 mmol), Lithiumchlorid (1, 86 g, 45, 1 mmol) und Natriumcarbonat (9, 54 g, 90, 3 mmol) in DMF (460 ml) 15 h bei 100°C gerührt. Das Reaktionsgemisch wurde auf RT abgekühlt, mit 2L EtOAc/ Ether (1: 1) versetzt und in 2 L $H_2O$ gegeben. Die zweiphasige Suspension wurde über Filtererde filtriert. Nach Trennung der Phasen wurde die wässrige Phase mit EtOAc (2 x 1 L) extrahiert. Die organischen Phasen wurden vereinigt und mit $H_2O$ und mit gesättigter NaCl- Lösung gewaschen und über $MgSO_4$ getrocknet. Nach Filtration des Trockenmittels wurden die Lösungsmittel am Rotationsverdampfer entfernt. Säulenchromatographie des Rückstands (tert.- Butylmethylether/n-Hexan = 2: 3) Anschließend wurde die erhaltene Mischfraktion 4 x in jeweils 20 ml Dichlormethan aufgenommen und vom weißen kristallinen Feststoff abgesaugt. Die ersten 3 fraktionierten Niederschläge wurden vereinigt 2- (2- (Triethylsilyl)- 5- (trifluoromethyl)- 1H- pyrrolo [2, 3- b] pyridin- 3- yl) ethanol (3, 8 g, 24, 4 %) .

**2-(5-(trifluoromethyl)-1H-pyrrolo[2,3-b]pyridin-3-yl)ethanol (Ind-3)**

2- (2- (Triethylsilyl)- 5- (trifluoromethyl)- 1H- pyrrolo [2, 3- b] pyridin- 3- yl) ethanol (3, 8 g, 11, 0 mmol) wurde mit TBAF (33, 1 ml, 33, 1 mmol; 1M- Lösung in THF) 6h bei 50°C und anschließend 10h bei RT gerührt. Das Lösungsmittel wurde am Rotationsverdampfer entfernt. Es resultierte 2- (5- (Trifluoromethyl)- 1H- pyrrolo [2, 3- b] pyridin- 3- yl) ethanol (2, 4 g, 94, 5 %) .

<u>**Baustein Ind-4**</u>

**S-2-(1H-Pyrrolo[2,3-b]pyridin-3-yl)ethyl ethanthioat**

Unter Schutzgas wurde Triphenylphosphin (3, 56 g, 13, 57 mmol) in absolutem THF (20 ml) gelöst. Die klare Lösung wurde auf- 5 °C abgekühlt. Unter Rühren wurde das in THF (20 ml) gelöste Diisopropyl- azodicarboxylat (2, 75 g, 13, 6 mmol) innerhalb von 15 min zugetropft. Dabei fiel ein weißer Niederschlag aus. Die Suspension wurde 30 min bei- 5 °C gerührt. Danach wurde eine Mischung aus dem **Ind- 1** (1100 mg, 6, 78 mmol) und Thioessigsäure (965 µl, 13, 57 mmol), gelöst in THF (20 ml), innerhalb von 30 min zugetropft. Die Reaktion war leicht exotherm. Die Temperatur wurde anschließend noch eine Stunde bei- 5 °C gehalten. Beim langsamen Erwärmen auf Raumtemperatur wurde aus der Suspension eine klare Lösung. Nach 18 h Rühren bei 23 °C wurde THF weitgehend abdestilliert. Das erhaltene Substanzgemisch (8, 5 g, braunes Öl) wurde mit Ethylacetat (30 ml) verdünnt und mit 1 N Salzäure (1 × 10 ml, 3 × 5 ml) extrahiert. Zu den vereinigten wässrigen Phasen wurde vorsichtig gesättigte Natriumhydrogencarbonat Lösung (25 ml) zugegeben. Die Mischung wurde mit Dichlormethan (3 × 10 ml) extrahiert. Die vereinigten organischen Phasen wurden über Natriumsulfat getrocknet, filtriert, und das Lösungsmittel im Vakuum abdestilliert. S- 2- (1 H- Pyrrolo [2, 3- b] pyridin- 3- yl) ethyl ethanthioat wurde als fast weißer Feststoff (1, 12 g, 75 %) erhalten.

**2-(1*H*-Pyrrolo[2,3-b]pyridin-3-yl)ethanthiol-Hydrochlorid (Ind-4)**

Unter Argon wurde Methanol (20 ml) auf unter 0 °C abgekühlt. Dann wurde Acetylchlorid (3 ml, 42 mmol) langsam zugetropft. Die Reaktion war exotherm. Die Temperatur wurde beim Zutropfen unter 15 °C gehalten. Das Reaktionsgemisch wurde 1 h bei Raumtemperatur gerührt. S- 2- (1 H- Pyrrolo [2, 3- b] pyridin- 3- yl) ethyl ethanthioat (600 mg, 2, 724 mmol) wurde in Methanol/ Dichlormethan 1 : 1 (10 ml) gelöst und dann innerhalb von 10 min zugetropft. Die Reaktionsmischung wurde 18 h bei 23 °C gerührt. Das Lösungsmittel wurde abdestilliert. Der orange Rückstand wurde in Cyclohexan (5 ml) suspendiert, filtriert und mit Cyclohexan (3 × 1 ml) gewaschen. Das beigefarbene **Ind- 4** wurde vermutlich als Hydrochlorid erhalten (578 mg, 99 %, Schmelzpunkt 138- 150 °C) .

<u>**Baustein Ind-5**</u>

**S-2-(1H-Pyrrolo[3,2-c]pyridin-3-yl)ethyl ethanthioat**

Unter Schutzgas wurde Triphenylphosphin (3, 83 g, 14, 6 mmol) in absolutem THF (20 ml) gelöst. Die klare Lösung wurde auf- 5°C abgekühlt. Unter Rühren wurde das in THF (20 ml) gelöste Diisopropyl- azodicarboxylat (2, 18 g, 10, 78 mmol) innerhalb von 15 min zugetropft. Dabei fiel ein weißer Niederschlag aus. Die Suspension wurde 30 min bei- 5°C gerührt. Danach wurde eine Mischung aus dem **Ind- 2** (1700 mg, 5, 38 mmol, Reinheit ca. 51 % (g/g) ) und Thioessigsäure (765 µl, 10, 75 mmol), gelöst in THF (20 ml), innerhalb von 30 min zugetropft. Die Reaktion war leicht exotherm. Die Temperatur wurde anschließend noch eine Stunde bei- 5 °C gehalten. Beim langsamen Erwärmen auf

Raumtemperatur wurde aus der Suspension eine klare Lösung. Nach 65 h Rühren bei 23°C wurde THF weitgehend abdestilliert. Das erhaltene Substanzgemisch (7, 3 g, braunes Öl) wurde mit Ethylacetat (30 ml) verdünnt und mit 1 N Salzäure (1 × 10 ml, 3 × 5 ml) extrahiert. Zu den vereinigten wäßrigen Phasen wurde vorsichtig gesättigte Natriumhydrogencarbonat Lösung (60 ml) zugegeben. Die Mischung wurde mit Dichlormethan (3 × 10 ml) extrahiert. Die vereinigten organischen Phasen wurden über Natriumsulfat getrocknet. Nach Filtration wurde das Lösungsmittel im Vakuum abdestilliert. S- 2- (1H- Pyrrolo [3, 2- c] pyridin- 3- yl) ethyl ethanthioat wurde als braunes Öl (1, 05 g, 82 %) erhalten.

**2-(1*H*-Pyrrolo[3,2-c]pyridin-3-yl)ethanthiol-Hydrochlorid (Ind-5)**

Unter Argon wurde Methanol (30 ml) auf unter 0 °C abgekühlt. Dann wurde Acetylchlorid (3 ml, 42 mmol) langsam zugetropft. Die Reaktion war exotherm. Die Temperatur wurde beim Zutropfen unter 15°C gehalten. Das Reaktionsgemisch wurde 1 h bei Raumtemperatur gerührt. S- 2- (1H- Pyrrolo [3, 2- c] pyridin- 3- yl) ethyl ethanthioat (1, 05 g, 4, 77 mmol) wurde in Methanol (10 ml) gelöst und dann innerhalb von 10 min zugetropft. Die Reaktionsmischung wurde 18 h bei 23 °C gerührt. Das Lösungsmittel wurde abdestilliert. Der orange Rückstand wurde in Diethylether (10 ml) suspendiert, filtriert und mit Diethylether (3 × 1 ml) gewaschen. Das beigefarbene **Ind- 5** wurde vermutlich als Hydrochlorid erhalten (975 mg, 95 %, Schmelzpunkt 182- 195 °C) .

<u>**Beispiele**</u>

<u>**Beispiel 1:**</u>

4-(Dimethylamino)-4-phenyl-spiro[cyclohexan-1,8'-(5,6,8,9-Tetrahydro-pyrano[3,4-b]-7-aza-indol)], 1 Diastereomer

Keton **Ket-1** (0,13 g, 0,62 mmol) wurde zusammen mit **Ind-1** (0,10 g, 0,62 mmol) in Dichlormethan (4 ml) ultratrocken vorgelegt. Das Mikrowellengefäß wurde mittels eines Septums verschlossen und mit Stickstoff gespült. Anschließend erfolgte eine schnelle Zugabe von Trimethylsilyltrifluormethansulfonat (0,47 ml, 2,47 mmol). Das Gemisch wurde 2x20 min bei 120°C in der Mikrowelle erhitzt. Anschließend wurde das Reaktionsgemisch mit 2N NaOH versetzt und 20 min gerührt. Die organische Phase wurde abgetrennt und die wässrige Phase mit Dichlormethan (3x) extrahiert. Die vereinigten organischen Extrakte wurden mit Wasser gewaschen und über $MgSO_4$ getrocknet. Nach Filtration des Trockenmittels wurde das Lösungsmittel am Rotationsverdampfer entfernt. Der erhaltene Feststoff (0,71 g) wurde mit Methanol (7 ml) versetzt und das Gemisch für 2 h bei Raumtemperatur gerührt. Der Feststoff wurde abgesaugt, mit wenig Methanol nachgewaschen und getrocknet.
**Ausbeute (Bsp. 1)**: 0,17 g (78 %), cremefarbener Feststoff.
$^1$H NMR (600 MHz, $D_6$-DMSO)$\delta$ ppm: 1.65-2.8 (m, 16H), 3.17 (s, 1 H), 3.96 (m, 2H), 6.93-6.8 (m, 1 H), 7.40-7.70 (bm, 4H), 7.76 (d, 1H), 8.06 (d, 1 H), 11.40 (s, 1 H)

<u>**Beispiel 2:**</u>

4-(Dimethylamino)-4-phenyl-spiro[cyclohexan-1,8'-(5,6,8,9-Tetrahydro-pyrano[3,4-b]-7-aza-indol)]; Methansulfonat, Diastereomerengemisch

Keton **Ket-1** (0,13 g, 0,62 mmol) wurde zusammen mit **Ind-1** (0,10 g, 0,62 mmol) in Dichlormethan (4 ml, ultratrocken) vorgelegt. Das Mikrowellengefäß wurde mittels eines Septums verschlossen und mit Stickstoff gespült. Anschließend erfolgte eine schnelle Zugabe von Trimethylsilyltrifluormethansulfonat (0,26 ml, 1,36 mmol). Das Gemisch wurde 2x10 min bei 90°C in der Mikrowelle erhitzt. Anschließend wurde erneut Trimethylsilyltrifluormethansulfonat (0,26 ml, 1,36 mmol) hinzugefügt und das Gemisch 1 x10 min, dann 1 x20 min bei 120°C in der Mikrowelle erhitzt.
Das Reaktionsgemisch wurde schließlich mit 2N NaOH versetzt und 20 min gerührt. Die organische Phase wurde abgetrennt und die wässrige Phase mit Dichlormethan (3x) extrahiert. Die vereinigten organischen Extrakte wurden mit Wasser gewaschen und über $MgSO_4$ getrocknet. Nach Filtration des Trockenmittels wurde das Lösungsmittel am Rotationsverdampfer entfernt. Der erhaltene Feststoff wurde mit Methanol (7 ml) versetzt und das Gemisch für 2 h bei Raumtemperatur gerührt. Der Feststoff wurde abgesaugt, mit wenig Methanol gewaschen, getrocknet (0,14 g, 63%).Zur Fällung des Methansulfonats wurde der Feststoff (0,14 g, 0,39 mmol) in Dichlormethan (2 ml) aufgeschlämmt und mit Methansulfonsäure (0,028 ml) versetzt. Der nun klaren Lösung wurde nach 1 min Aceton (0,5 ml) und tropfenweise Ether hinzugefügt bis ein rührfähiges Gemisch resultierte. Es wurde 30 min bei Raumtemperatur gerührt. Anschließend wurde der Niederschlag unter Luftausschluss abgesaugt, portionsweise mit Ether gewaschen und am Hochvakuum bei 50°C getrocknet.
**Ausbeute (Bsp. 2):** 0,16 g (90 %), Diastereomerengemisch (ca. 6:1).

**Beispiel 3:**

**4-(Dimethylamino)-4-phenyl-spiro[cyclohexan-1,6'-(5,6,8,9-Tetrahydro-pyrano[3,4-b]-5-aza-indol)], Diastereomerengemisch**

Keton **Ket-1** (0,13 g, 0,62 mmol) wurde zusammen mit **Ind-2** (0,10 g, 0,62 mmol) in Dichlormethan (4 ml) ultratrocken vorgelegt. Das Mikrowellengefäß wurde mittels eines Septums verschlossen und mit Stickstoff gespült. Anschließend erfolgte eine schnelle Zugabe von Trimethylsilyltrifluormethansulfonat (0,47 ml, 2,47 mmol). Das Gemisch wurde 40 min bei 120°C in der Mikrowelle erhitzt. Anschließend wurde das Reaktionsgemisch mit 2N NaOH versetzt und 20 min gerührt. Die organische Phase wurde abgetrennt und die wässrige Phase mit Dichlormethan (3x) extrahiert. Die vereinigten organischen Extrakte wurden mit Wasser gewaschen und über $MgSO_4$ getrocknet. Nach Filtration des Trockenmittels wurde das Lösungsmittel am Rotationsverdampfer entfernt. Der erhaltene Feststoff wurde mit Methanol (7 ml) versetzt und das Gemisch für 48 h bei Raumtemperatur gerührt. Der Feststoff wurde abgesaugt, mit wenig Methanol nachgewaschen und getrocknet.
**Ausbeute (Bsp. 3):** 0,06 g (28 %), cremefarbener Feststoff (Diastereomerengemisch: ca. 5:1).
$^1$H NMR (600 MHz, $D_6$-DMSO) $\delta$ ppm: Diastereomer 1:1.63 (t, 2H), 2.13 (bd, 2H), 2.16 (t, 2H), 2.27 (s, 6H) 2.11 (bd, 2H), 2.77 (m, 2H), 3.98 (m,2H), 7.26 (d, 1 H), 7.37 (d, 1H), 7.5 (m, 1 H) 7.56 (t, 2H), 7.63 (m. 2H), 8.12 (d,1 H), 8.76 (s, 1 H) 11.33 (bs, 1 H),
Zusätzliche Peaks Diastereomer 2: 1.74-1.80 (bt, 4H), 2.06 (s, 6H), 3.91 (m, 2H), 7.43-7.48 (m, 1 H), 8,19 (d, 1H), 8.84 (s, 1 H), 11.8 (bs, 1 H)

**Beispiel 4:**

**4-(Dimethylamino)-4-thiophen-2-yl-spiro[cyclohexan-1,8'-(5,6,8,9-Tetrahydro-pyrano[3,4-b]-7-aza-indol)]; Methansulfonat, 1 Diastereomer**

Keton **Ket- 2** (0, 13 g, 0, 62 mmol) und 2- (1H- pyrrolo [2, 3- b] pyridin- 3- yl) ethanol **Ind- 1** (0, 10 g, 0, 62 mmol) wurden unter Stickstoff in Dichlormethan (4 ml, ultratrocken) vorgelegt. Anschließend erfolgte eine schnelle Zugabe von Trimethylsilyltrifluormethansulfonat (0, 48 ml, 2, 47 mmol) . Das Gemisch wurde 5 Tage bei Raumtemperatur gerührt. Nach Zugabe von Tetrahydrofuran wurde das Gemisch mit 1 M wässriger $Na_2CO_3$- Lösung basisch gestellt (pH= 11) und 20 min bei Raumtemperatur gerührt. Die organische Phase wurde abgtrennt und die wässrige Phase mit Tetrahydrofuran (2x) extrahiert. Die vereinigten organischen Phasen wurden mit gesättigter NaCl- Lösung gewaschen und über $MgSO_4$ getrocknet. Nach Filtration des Trockenmittels wurden die Lösungsmittel am Rotationsverdampfer entfernt. Der erhaltene Feststoff wurde mit Methanol (3 ml) versetzt und 18 h bei Raumtemperatur gerührt. Der Feststoff wurde abgesaugt, mit wenig Methanol gewaschen und am Ölpumpenvakuum bei 50°C getrocknet (0, 09 g, 43% ) .Zur Fällung des Methansulfonats wurde der Feststoff (0, 07 g, 0, 19 mmol) in Dichlormethan (2 ml) aufgeschlämmt und bei Raumtemperatur mit Methansulfonsäure (0, 014 ml) versetzt. Die Suspension wurde nach 5 min mit Ether (10 ml) versetzt. Nach 30 min Rühren bei Raumtemperatur wurde der Feststoff unter Luftausschluss abgesaugt, mit Ether gewaschen und bei 50°C am Ölpumpenvakuum getrocknet.**Ausbeute (Bsp. 4) :** 0, 07 g (79 %)
$^1$H NMR (600 MHz, $D_6$- DMSO) $\delta$ ppm: 1.87- 1.98 (m, 4H), 2.26- 2.35 (m, 5H), 2.59- 2.65 (m, 8H), 2.71 (t, 2H), 3.97 (t, 2H), 6.98- 7.04 (m, 1 H), 7.32 (t, 1 H), 7.55 (d, 1 H), 7.83 (d, 1 H), 7.92 (d, 1 H), 8.11 (d, 1 H), 9.55 (bs, 1 H), 11.60 (s, 1 H)

**Beispiel 5:**

**4-(Dimethylamino)-4-thiophen-2-yl-spiro[cyclohexan-1,6'-(5,6,8,9-Tetrahydro-pyrano[3,4-b]-5-aza-indol)], 1 Diastereomer**

Keton **Ket- 2** (0, 13 g, 0, 62 mmol) und **Ind- 2** (0, 10 g, 0, 62 mmol) wurden unter Stickstoff in Dichlormethan (4 ml, ultratrocken) vorgelegt. Anschließend erfolgte eine schnelle Zugabe von Trimethylsilyltrifluormethansulfonat (0, 48 ml, 2, 47 mmol) . Das Gemisch wurde 6 Tage bei Raumtemperatur gerührt. Nach Zugabe von Tetrahydrofuran wurde das Gemisch mit 1M wässriger $Na_2CO_3$- Lösung basisch gestellt (pH= 11) und 20 min bei Raumtemperatur gerührt. Die organische Phase wurde abgetrennt und die wässrige Phase mit Essigsäureethylester (2x) extrahiert. Die vereinigten organischen Phasen wurden mit gesättigter NaCl- Lösung gewaschen und über $MgSO_4$ getrocknet. Nach Filtration des Trockenmittels wurden die Lösungsmittel am Rotationsverdampfer entfernt. Säulenchromatographische Reinigung des Rückstandes (Dichlormethan : Methanol= 19: 1) lieferte **Bsp. 5** (0, 05 g, 22% ) .
$^1$H NMR (600 MHz, $D_6$- DMSO) $\delta$ ppm: 1.84 (bt, 2H), 2.01 (bd, 2H), 2.29 (bt, 2H), 2.5 (s, 6H überlagert von DMSO), 2.60 (bd, 2H), 2.85 (t, 2H), 4.01 (t, 2H), 7.30- 7.34 (m, 1 H), 7.48- 7.53 (m, 1 H), 7.63 (d, 1H), 7.86- 7.91 (m, 1 H), 8.29 (d, 1H), 9.09 (s, 1H), 12.37 (bs, 1 H)

**Beispiel 6:**

**4-(Dimethylamino)-4-phenyl-spiro[cyclohexan-1,8'-(5,6,8,9-Tetrahydro-pyrano[3,4-b]-7-aza-indol)]; Citrat (4:3), 1 Diastereomer**

**Ket- 1** (0, 27 g, 1, 23 mmol) wurde zusammen mit **Ind- 1** (0, 20 g, 1, 23 mmol) in Dichlormethan (4 ml, ultratrocken) vorgelegt. Das Mikrowellengefäß wurde mittels eines Septums verschlossen und mit Stickstoff gespült. Anschließend erfolgte eine schnelle Zugabe von Trimethylsilyltrifluormethansulfonat (1, 1 ml, 4, 9 mmol) . Das Gemisch wurde 40 min bei 120°C in der Mikrowelle erhitzt. Das Reaktionsgemisch wurde schließlich mit 1 M $Na_2CO_3$- Lösung versetzt (pH = 11) und 20 min gerührt. Die organische Phase wurde abgetrennt und die wässrige Phase mit Dichlormethan (3x) extrahiert. Die vereinigten organischen Extrakte wurden mit Wasser gewaschen und über $MgSO_4$ getrocknet. Nach Filtration des Trockenmittels wurde das Lösungsmittel am Rotationsverdampfer entfernt. Der erhaltene Feststoff wurde mit Methanol (7 ml) versetzt und das Gemisch für 2 h bei Raumtemperatur gerührt. Der Feststoff wurde abgesaugt, mit wenig Methanol gewaschen, getrocknet (0, 257 g, 57%) .

Zur Fällung des Citrates wurde der Feststoff (0,1 g, 0,27 mmol) in heißem Ethanol (6 ml) aufgeschlämmt und mit einer ebenfalls heißen Lösung von Citronensäure (0,053 g) in Ethanol (1,4 ml) versetzt. Die Lösung wurde 3 Tage bei Raumtemperatur gerührt. Anschließend wurde der Niederschlag abgesaugt, portionsweise mit Ether gewaschen und am Hochvakuum bei 60°C getrocknet.
**Ausbeute (Bsp. 6):** 0,109 g (71 %).
$^1H$ NMR (600 MHz, $D_6$-DMSO) $\delta$ ppm: 1.68-1.89 (m, 4H), 2.1-2.3 (m, 8H), 2.50-2.58 (q, 3H), 2.68 (m, 4H), 3.96 (m, 2H), 6.93-6.99 (m, 1 H), 7.43-7.64 (m, 5H), 7.75 (d, 1 H), 8.06 (d, 1 H), 11.40 (s, 1 H)

**Beispiel 7:**

**4-(Methylamino)-4-phenyl-spiro[cyclohexan-1,8'-(5,6,8,9-Tetrahydro-pyrano[3,4-b]-7-aza-indol)]; Citrat (1:1), 1 Diastereomer**

Keton **Ket- 3** (0, 125 g, 0, 62 mmol) wurde zusammen mit **Ind- 1** (0, 10 g, 0, 62 mmol) in Dichlormethan (4 ml, ultratrocken) vorgelegt. Das Mikrowellengefäß wurde mittels eines Septums verschlossen und mit Stickstoff gespült. Anschließend erfolgte eine schnelle Zugabe von Trimethylsilyltrifluormethansulfonat (0, 48 ml, 2, 5 mmol) . Das Gemisch wurde 30 min bei 120°C in der Mikrowelle erhitzt. Das Reaktionsgemisch wurde schließlich mit 2N NaOH- Lösung versetzt und 20 min gerührt. Die organische Phase wurde abgetrennt und die wässrige Phase mit Dichlormethan (3x) extrahiert. Die vereinigten organischen Extrakte wurden mit Wasser gewaschen und über $MgSO_4$ getrocknet. Nach Filtration des Trockenmittels wurde das Lösungsmittel am Rotationsverdampfer entfernt. Säulenchromatographische Reinigung des Rückstandes (Dichlormethan : Methanol = 4: 1) lieferte 4- (Methylamino)- 4- phenyl- spiro [cyclohexan- 1, 8'- (5, 6, 8, 9- Tetrahydro- pyrano [3, 4- b]- 7- aza- indol) ] (0, 06 g, 28%) .

Zur Fällung des Citrates wurde 4- (Methylamino)- 4- phenyl- spiro [cyclohexan- 1, 8'- (5, 6, 8, 9- Tetrahydro- pyrano [3, 4- b]- 7- aza- indol) ] (0, 053 g, 0, 15 mmol) in heißem Ethanol (4 ml) aufgeschlämmt und mit einer ebenfalls heißen Lösung von Citronensäure (0, 032 g) in Ethanol (1 ml) versetzt. Die Lösung wurde 3 h bei Raumtemperatur gerührt. Anschließend wurde der Niederschlag abgesaugt, portionsweise mit Ether gewaschen und am Hochvakuum bei 60°C getrocknet.
**Ausbeute (Bsp. 7) :** 0, 07 g (85 %) .
$^1H$ NMR (600 MHz, $D_6$- DMSO) $\delta$ ppm: 1.76- 1.83 (bt, 2H), 1.87- 1.93 (bd, 2H), 2.08 (s, 3H), 2.16- 2.25 (bt, 2H), 2.47 (d, 2H), 2.54 (d, 2H), 2.55- 2.60 (m, 2H), 2.70 (bt, 2H), 3.98 (bt, 2H), 6.95- 6.99 (m, 1 H), 7.49 (t, 1 H), 7.56 (t, 2H), 7.66 (d, 2H), 7.77 (d, 1 H) 8.08 (d, 1 H), 11.46 (s, 1 H) Breites Signal bei 8.5- 12.0

**Beispiel 8:**

4-(Methylamino)-4-tiophen-2-yl-spiro[cyclohexan-1,8'-(5,6,8,9-Tetrahydro-pyrano[3,4-b]-7-aza-indol)]; Citrat (4:3), 1 Diastereomer

Keton **Ket- 4** (0, 13 g, 0, 62 mmol) wurde zusammen mit **Ind- 1** (0, 10 g, 0, 62 mmol) unter Stickstoff in Dichlormethan (4 ml, ultratrocken) vorgelegt. Anschließend erfolgte eine schnelle Zugabe von Trimethylsilyltrifluormethansulfonat (0, 48 ml, 2, 5 mmol) . Das Gemisch wurde 15 Tage bei Raumtemperatur gerührt. Nach Zugabe von Tetrahydrofuran wurde das Gemisch mit 1 M $Na_2CO_3$- Lösung basisch gestellt und 20 min gerührt. Die organische Phase wurde abgetrennt und die wässrige Phase mit Tetrahydrofuran (2x) extrahiert. Die vereinigten organischen Extrakte wurden mit gesättigter NaCl- Lösung gewaschen und über $MgSO_4$ getrocknet. Nach Filtration des Trockenmittels wurde das Lösungsmittel am Rotationsverdampfer entfernt. Der erhaltene Feststoff wurde mit Methanol (5 ml) versetzt

und das Gemisch für 18 h bei Raumtemperatur gerührt. Der Feststoff wurde abgesaugt, mit wenig Methanol gewaschen, im Ölpumpenvakuum getrocknet (0, 118 g, 54%) . Zur Fällung des Citrates wurde der Feststoff (0, 112 g, 0, 32 mmol) in heißem Ethanol (4 ml) aufgeschlämmt und mit einer ebenfalls heißen Lösung von Citronensäure (0, 066 g) in Ethanol (1, 5 ml) versetzt. Die Lösung wurde 3 h bei Raumtemperatur gerührt. Anschließend wurde der Niederschlag abgesaugt, portionsweise mit Ether gewaschen und am Hochvakuum bei 60°C getrocknet.
**Ausbeute (Bsp. 8) :** 0, 117 g (68 %) .
$^1$H NMR (600 MHz, $D_6$- DMSO) δ ppm: 1.86- 1.99 (m, 4H), 2.14 (s, 3H) 2.18 (bd, 2H), 2.31 (bd, 2H), 2.47 (d, 1.4 H), 2.54 (d, 1.4 H), 2.69 (t, 2H), 3.95 (t, 2H), 6.96- 6.99 (m, 1 H), 7.16 (m, 1 H), 7.30 (m, 1 H), 7.66 (d, 1 H), 7.77 (d, 1 H), 8.09 (d, 1H), 11.51 (s, 1 H) Breites Signal bei 8.0- 12.0

**Beispiel 9:**

**4-(Dimethylamino)-4-benzo[1,3-dioxol]-spiro[cyclohexan-1,8'-(5,6,8,9-Tetrahydro-pyrano[3,4-b]-7-aza-indol)]; Citrat (1:1), 1 Diastereomer**

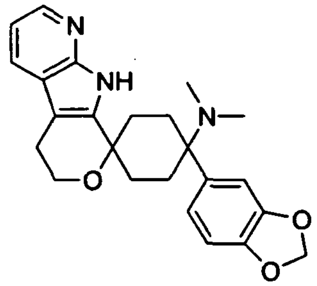

Keton **Ket- 5** (0, 16 g, 0, 62 mmol) wurde zusammen mit **Ind- 1** (0, 10 g, 0, 62 mmol) unter Stickstoff in Dichlormethan (4 ml) ultratrocken vorgelegt. Anschließend erfolgte eine schnelle Zugabe von Trimethylsilyltrifluormethansulfonat (0, 48 ml, 2, 5 mmol) . Das Gemisch wurde 15 Tage bei Raumtemperatur gerührt. Nach Zugabe von Tetrahydrofuran wurde das Gemisch mit 1 M $Na_2CO_3$- Lösung basisch gestellt und 20 min gerührt. Die organische Phase wurde abgetrennt und die wässrige Phase mit Tetrahydrofuran (2x) extrahiert. Die vereinigten organischen Extrakte wurden mit gesättigter NaCl- Lösung gewaschen und über $MgSO_4$ getrocknet. Nach Filtration des Trockenmittels wurde das Lösungsmittel am Rotationsverdampfer entfernt. Der erhaltene Feststoff wurde mit Methanol (5 ml) versetzt und das Gemisch für 2 h bei Raumtemperatur gerührt. Der Feststoff wurde abgesaugt, mit wenig Methanol gewaschen und am Ölpumpenvakuum bei 50°C getrocknet (0, 052 g, 21%) . Zur Fällung des Citrates wurde der Feststoff (0, 047 g, 0.11 mmol) in heißem Ethanol (4 ml) aufgeschlämmt und mit einer ebenfalls heißen Lösung von Citronensäure (0, 024 g) in Ethanol (1 ml) versetzt. Die Lösung wurde 3 h bei Raumtemperatur gerührt. Anschließend wurde der Niederschlag abgesaugt, portionsweise mit Ether gewaschen und am Hochvakuum bei 60°C getrocknet.
**Ausbeute (Bsp. 9) :** 0, 061 g (88 %) .
$^1$H NMR (600 MHz, $D_6$- DMSO) δ ppm: 1.7- 1.78 (t, 2H), 1.83- 1.90 (d, 2H), 2.09- 2.17 (m, 2H), 2, 32- 2.41 (m, 6H) 2.53 (d, 2H), 2.58 (d, 2H), 2.68 (t, 4H), 3.96 (t, 2H), 6.13 (s, 2H), 6.95- 6.99 (m, 1H), 7.05- 7.10 (m, 1 H), 7.10- 7.17 (m, 1 H), 7.25 (m, 1 H), 7.76 (d, 1 H), 8.08 (d, 1 H), 11.42 (bs, 1 H)

**Beispiel 10:**

**4-(Dimethylamino)-4-(Benzothiophen-2-yl)-spiro[cyclohexan-1,8'-(5,6,8,9-Tetrahydro-pyrano[3,4-b]-7-aza-indol)]; Citrat (1:1), 1 Diastereomer**

Keton **Ket- 6** (0, 17 g, 0, 62 mmol) wurde zusammen mit **Ind- 1** (0, 10 g, 0, 62 mmol) unter Stickstoff in Dichlormethan (4 ml) ultratrocken vorgelegt. Anschließend erfolgte eine schnelle Zugabe von Trimethylsilyltrifluormethansulfonat (0, 48 ml, 2, 5 mmol) . Das Gemisch wurde 7 Tage bei Raumtemperatur gerührt. Nach Zugabe von Tetrahydrofuran wurde das Gemisch mit 1M $Na_2CO_3$- Lösung basisch gestellt und 20 min gerührt. Die organische Phase wurde abgetrennt und die wässrige Phase mit Dichlormethan (3x) extrahiert. Die vereinigten organischen Extrakte wurden mit gesättigter NaCl- Lösung gewaschen und über $MgSO_4$ getrocknet. Nach Filtration des Trockenmittels wurde das Lösungsmittel am Rotationsverdampfer entfernt. Der erhaltene Feststoff wurde mit Methanol (5 ml) versetzt und das Gemisch für 2 h bei Raumtemperatur gerührt. Der Feststoff wurde abgesaugt, mit wenig Methanol gewaschen und

am Ölpumpenvakuum bei 50°C getrocknet (0, 158 g, 61%) . Zur Fällung des Citrates wurde der Feststoff (0, 154 g, 0, 37 mmol) in heißem Ethanol (4 ml) aufgeschlämmt und mit einer ebenfalls heißen Lösung von Citronensäure (0, 071 g) in Ethanol (2, 5 ml) versetzt. Die Lösung wurde 3 h bei Raumtemperatur gerührt. Anschließend wurde der Niederschlag abgesaugt, portionsweise mit Ether gewaschen und am Hochvakuum bei 60°C getrocknet.
**Ausbeute (Bsp. 10) :** 0, 193 g (85 %) .
$^1$H NMR (600 MHz, $D_6$- DMSO) δ ppm: 1.89- 2.03 (m, 4H), 2.19- 2.28 (m, 2H), 2.39 (bs, 6H), 2.56- 2.62 (m, 3H), 2.65- 2.71 (m, 5H) ; 3.96 (m, 2H), 6.94- 6.98 (m, 1H), 7.38- 7.46 (m, 2H), 7.63 (bs, 1H), 7.76 (d, 1 H), 7.93 (d, 1H), 8.02 (d, 1 H), 8.05 (d, 1 H), 11.43 (bs, 1 H)

**Beispiel 12:**

4-(Dimethylamino)-4-(3-fluorophenyl)-spiro[cyclohexan-1,8'-(5,6,8,9-Tetrahydro-pyrano[3,4-b]-7-aza-indol)]; Citrat (4: 3), 1 Diastereomer

Keton **Ket- 7** (0, 145 g, 0, 62 mmol) wurde zusammen mit **Ind- 1** (0, 10 g, 0, 62 mmol) unter Stickstoff in Dichlormethan (4 ml) ultratrocken vorgelegt. Anschließend erfolgte eine schnelle Zugabe von Trimethylsilyltrifluormethansulfonat (0, 48 ml, 2, 5 mmol) . Das Gemisch wurde 7 Tage bei Raumtemperatur gerührt. Nach Zugabe von Dichlormethan wurde das Gemisch mit 1M $Na_2CO_3$- Lösung basisch gestellt und 20 min gerührt. Die organische Phase wurde abgetrennt und die wässrige Phase mit Dichlormethan (3x) extrahiert. Die vereinigten organischen Extrakte wurden mit gesättigter NaCl- Lösung gewaschen und über $MgSO_4$ getrocknet. Nach Filtration des Trockenmittels wurde das Lösungsmittel am Rotationsverdampfer entfernt. Der erhaltene Feststoff wurde mit Methanol (5 ml) versetzt und das Gemisch für 2 h bei Raumtemperatur gerührt. Der Feststoff wurde abgesaugt, mit wenig Methanol gewaschen und am Ölpumpenvakuum bei 50°C getrocknet (0, 054 g, 23%) . Zur Fällung des Citrates wurde der Feststoff (0, 054 g, 0, 14 mmol) in heißem Ethanol (3 ml) aufgeschlämmt und mit einer ebenfalls heißen Lösung von Citronensäure (0, 027 g) in Ethanol (1 ml) versetzt. Die Lösung wurde 3 h bei Raumtemperatur gerührt. Anschließend wurde der Niederschlag abgesaugt, portionsweise mit Ether gewaschen und am Hochvakuum bei 60°C getrocknet.
**Ausbeute (Bsp. 12) :** 0, 048 g (59 %) .
$^1$H NMR (600 MHz, $D_6$- DMSO) δ ppm: 1.69 (bt, 2H), 1.85 (bd, 2H), 2.09 (bt, 2H), 2.20 (bs, 6H), 2.51- 2.55 (d, 1.5H), 2.56- 2.62 (d, 1.5H), 2.63 (m, 2H) 2.67 (t, 2H), 3.95 (t, 2H), 6.94- 6.97 (m, 1 H), 7.25- 7.31 (m, 1 H), 7.35- 7.42 (m, 2H), 7.52- 7.58 (m, 1 H), 7.76 (d, 1 H), 8.07 (d, 1H), 11.37 (s, 1 H)

**Beispiel 13:**

4-(Dimethylamino)-4-(3-methylphenyl)-spiro[cyclohexan-1,8'-(5,6,8,9-Tetrahydro-pyrano[3,4-b]-7-aza-indol)]; Citrat (1: 1), 1 Diastereomer

Keton **Ket- 8** (0, 142 g, 0, 62 mmol) wurde zusammen mit **Ind- 1** (0, 10 g, 0, 62 mmol) unter Stickstoff in Dichlormethan (4 ml) ultratrocken vorgelegt. Anschließend erfolgte eine schnelle Zugabe von Trimethylsilyltrifluormethansulfonat (0, 48 ml, 2, 5 mmol) . Das Gemisch wurde 7 Tage bei Raumtemperatur gerührt. Nach Zugabe von Dichlormethan wurde das Gemisch mit 1M $Na_2CO_3$- Lösung basisch gestellt und 20 min gerührt. Die organische Phase wurde abgetrennt und die wässrige Phase mit Dichlormethan (3x) extrahiert. Die vereinigten organischen Extrakte wurden mit gesättigter NaCl- Lösung gewaschen und über $MgSO_4$ getrocknet. Nach Filtration des Trockenmittels wurde das Lösungsmittel am Rotationsverdampfer entfernt. Der erhaltene Feststoff wurde mit Methanol (5 ml) versetzt und das Gemisch für 2 h bei Raumtemperatur gerührt. Der Feststoff wurde abgesaugt, mit wenig Methanol gewaschen und am Ölpumpenvakuum bei 50°C getrocknet (0, 117 g, 50%) . Zur Fällung des Citrates wurde der Feststoff (0, 112 g, 0, 30 mmol) in heißem Ethanol (4 ml) aufgeschlämmt und mit einer ebenfalls heißen Lösung von Citronensäure (0, 057 g) in Ethanol (2 ml) versetzt. Die Lösung wurde 3 h bei Raumtemperatur gerührt. Anschließend wurde der Niederschlag abgesaugt, portionsweise mit Ether gewaschen und am Hochvakuum bei 60°C getrocknet.
**Ausbeute (Bsp. 13) :** 0, 118 g (70 %) .
$^1$H NMR (600 MHz, $D_6$- DMSO) δ ppm: 1.73 (m, 2H), 2.15 (m, 2H), 2.3 (bs, 6 H), 2.43 (s, 3H), 2.56- 2.58 (m, 3H), 2.67- 2.69 (m, 4H), 3.96 (m, 2H), 6.95- 6.97 (m, 1 H), 7. 32 (bm, 1), 7.42- 7.44 (m, 3H), 7.77 (d, 1 H), 8.07 (d, 1 H), 11.41 (s, 1 H) .

**Beispiel 14:**

**4-(Dimethylamino)-4-(but-1-yl)-spiro[cyclohexan-1,8'-(5,6,8,9-Tetrahydro-pyrano[3,4-b]-7-aza-indol)], Citrat (1: 1), 1 Diastereomer**

Keton **Ket- 9** (0, 3 g, 0, 1, 85 mmol) wurde zusammen mit **Ind- 1** (0, 10 g, 0, 62 mmol) unter Stickstoff in

Dichlormethan (4 ml) ultratrocken vorgelegt. Anschließend erfolgte eine schnelle Zugabe von Trimethylsilyltrifluormethansulfonat (0, 48 ml, 2, 5 mmol) . Das Gemisch wurde 5 Tage bei Raumtemperatur gerührt. Nach Zugabe von Dichlormethan wurde das Gemisch mit 1 M $Na_2CO_3$- Lösung basisch gestellt und 20 min gerührt. Die organische Phase wurde abgetrennt und die wässrige Phase mit Dichlormethan (3x) extrahiert. Die vereinigten organischen Extrakte wurden mit gesättigter NaCl- Lösung gewaschen und über $MgSO_4$ getrocknet. Nach Filtration des Trockenmittels wurde das Lösungsmittel am Rotationsverdampfer entfernt. Säulenchromatographische Reinigung des Rückstandes (Dichlormethan : Methanol = 4: 1) lieferte 4- (Dimethylamino)- 4- (but- 1- yl)- spiro [cyclohexan- 1, 8'- (5, 6, 8, 9- Tetrahydro-pyrano [3, 4- b]- 7- aza- indol) ] (0, 026 g, 12%) .

Zur Fällung des Citrates wurde der Feststoff (0,02 g, 0,06 mmol) in heißem Ethanol (2 ml) aufgeschlämmt und mit einer ebenfalls heißen Lösung von Citronensäure (0,012 g) in Ethanol (1 ml) versetzt. Die Lösung wurde 3 h bei Raumtemperatur gerührt. Anschließend wurde der Niederschlag abgesaugt, portionsweise mit Ether gewaschen und am Hochvakuum bei 60°C getrocknet.
**Ausbeute (Bsp. 14):** 0,021 g (67 %).

__Beispiel 15:__

4-(Dimethylamino)-4-Phenylethyl-spiro[cyclohexan-1,8'-(3-trifluoromethyl-5,6,8,9-Tetrahydro-pyrano[3,4-b]-7-aza-indol)]; Citrat (1:1), 1 Diastereomer

Keton **Ket- 10** (0, 319 g, 1, 30 mmol) wurde zusammen mit **Ind- 3** (0, 3 g, 1, 30 mmol) in Dichlormethan (3, 4 ml) ultratrocken vorgelegt. Das Mikrowellengefäß wurde mittels eines Septums verschlossen und mit Stickstoff gespült. Anschließend erfolgte eine schnelle Zugabe von Trimethylsilyltrifluormethansulfonat (1, 01 ml, 5, 2 mmol) . Der Ansatz wurde 10 min bei 120°C in der Mikrowelle erhitzt. Anschließend wurde das Reaktionsgemisch mit 1 M wässriger $Na_2CO_3$-Lösung versetzt und 20 min gerührt. Die organische Phase wurde abgetrennt und die wässrige Phase mit Dichlormethan (3x) extrahiert. Die vereinigten organischen Extrakte wurden mit Wasser und gesättigter NaCl- Lösung gewaschen und über $MgSO_4$ getrocknet. Nach Filtration des Trockenmittels wurde das Lösungsmittel am Rotationsverdampfer entfernt. Der erhaltene schaumige Feststoff wurde mittels Säulenchromatographie (Kieselgel KG 60; Dichlormethan : Methanol 9: 1) gereinigt. Es resultierte 4- (Dimethylamino)- 4- Phenylethyl- spiro [cyclohexan- 1, 8'- (3- trifluoromethyl- 5, 6, 8, 9- Tetrahydro- pyrano [3, 4- b]- 7- aza- indol) ] (0, 14 g, 23, 5 %) .

Zur Fällung des Citrates wurde der Feststoff (0,140 g, 0,306 mmol) in heißem Ethanol (2 ml) gelöst und mit Citronensäure (0,058 g) und mit Diethylether (10 ml) versetzt. Die Lösung wurde bei Raumtemperatur gerührt. Der Feststoff wurde abgesaugt und am Hochvakuum bei 60°C getrocknet.
**Ausbeute (Bsp. 15):** 0,173 g (87 %).

__Beispiel 17:__

**4-(Dimethylamino)-4-Ethyl-spiro[cyclohexan-1,8'-(5,6,8,9-Tetrahydro-pyrano[3,4-b]-7-aza-indol)]; Citrat (2:3), Diastereomerengemisch**

**Ket- 12** (0, 25 g, 1, 48 mmol) wurde zusammen mit **Ind- 1** (0, 24 g, 1, 48 mmol) in Dichlormethan (3, 3 ml) ultratrocken vorgelegt. Das Mikrowellengefäß wurde mittels eines Septums verschlossen und mit Stickstoff gespült. Anschließend erfolgte eine schnelle Zugabe von Trimethylsilyltrifluormethansulfonat (1, 14 ml, 5, 92 mmol) . Der Ansatz wird bei 120°C 20 min in der Mikrowelle erhitzt. Anschließend wurde das Reaktionsgemisch mit 1 M wässriger $Na_2CO_3$-Lösung versetzt und 20 min gerührt. Die organische Phase wurde abgetrennt und die wässrige Phase mit Dichlormethan (3x) extrahiert. Die vereinigten organischen Extrakte wurden mit Wasser und ges. NaCl- Lösung gewaschen und über $MgSO_4$ getrocknet. Nach Filtration des Trockenmittels wurde das Lösungsmittel am Rotationsverdampfer entfernt. Der erhaltene Feststoff wurde mit Methanol (5 ml) versetzt und das Gemisch für 2 h bei Raumtemperatur gerührt. Der Feststoff wurde abgesaugt, mit wenig Methanol nachgewaschen und im Hochvakuum bei 50°C getrocknet. Es resultierte ein cremefarbener Feststoff (0, 07 g, 15, 1 %) .

Der Feststoff (0,07 g, 0,22 mmol) wurde in heißem Ethanol (2 ml) aufgeschlämmt und mit einer ebenfalls heißen Lösung von Citronensäure (0,043 g) in Ethanol (1 ml) versetzt. Die Lösung wurde 3 h bei Raumtemperatur gerührt. Die klare Lösung wurde zur Trockne eingeengt und am Hochvakuum bei 60°C getrocknet.
**Ausbeute (Bsp. 17):** 0,083 g (62 %).

**Beispiel 18:**

**4-(Dimethylamino)-4-Phenylethyl-spiro[cyclohexan-1,8'-(5,6,8,9-Tetrahydro-pyrano[3,4-b]-7-aza-indol)]; Citrat (2:3), 1 Diastereomer**

**Ket- 10** (0, 227 g, 0, 925 mmol) wurde zusammen mit **Ind- 1** (0, 15 g, 0, 925 mmol) in Dichlormethan (3, 6 ml) ultratrocken vorgelegt. Das Mikrowellengefäß wurde mittels eines Septums verschlossen und mit Stickstoff gespült. Anschließend erfolgte eine schnelle Zugabe von Trimethylsilyltrifluormethansulfonat (0, 715 ml, 3, 70 mmol) . Der Ansatz wird 10 d bei RT gerührt. Wegen unvollständiger Umsetzung wird das Gemisch wurde 2 x 15 min bei 120°C in der Mikrowelle erhitzt. Anschließend wurde das Reaktionsgemisch mit 1 M wässriger $Na_2CO_3$- Lösung versetzt und 20 min gerührt. Die organische Phase wurde abgetrennt und die wässrige Phase mit Dichlormethan (3x) extrahiert. Die vereinigten organischen Extrakte wurden mit Wasser gewaschen und über $MgSO_4$ getrocknet. Nach Filtration des Trockenmittels wurde das Lösungsmittel am Rotationsverdampfer entfernt. Der erhaltene Feststoff wurde mit Methanol (7 ml) versetzt und das Gemisch für 1 h bei Raumtemperatur gerührt. Der Feststoff wurde abgesaugt, mit wenig Methanol nachgewaschen und getrocknet. Es resultierte ein cremefarbener Feststoff (0, 15 g, 41, 4 %) . Der Feststoff (0, 144 g, 0, 37 mmol) wurde in heißem Ethanol (3 ml) aufgeschlämmt und mit einer ebenfalls heißen Lösung von Citronensäure (0, 071 g) in Ethanol (2 ml) versetzt. Die Lösung wurde 3 h bei Raumtemperatur gerührt. Anschließend wurde der Niederschlag abgesaugt, mit wenig Ethanol und 2x mit Ether gewaschen und am Hochvakuum bei 60°C getrocknet.
**Ausbeute (Bsp. 18) :** 0, 184 g (73 %) .
$^1$H NMR (600 MHz, $D_6$- DMSO) δ ppm: 1.74- 1.83 (m, 2H), 1.90- 1.96 (m, 2H), 2.02- 2.10 (m, 4H), 2.22- 2.30 (m, 2H), 2.52- 2.58 (d, 3H), 2.58- 2.65 (d, 3H), 2.65- 2.75 (m, 5H), 2.79 (bs, 5H), 3.94 (t, 2H), 6.99- 7.04 (m, 1 H), 7.18- 7.32 (m, 2H), 7.34- 7.43 (m, 3H), 7.82 (d, 1 H), 8.14 (d, 1 H), 11.70 (s, 1 H)

**Beispiel 19:**

**4-Benzyl-4-morpholino-spiro[cyclohexan-1,8'-(5,6,8,9-Tetrahydro-pyrano[3,4-b]-7-aza-indol)]; Citrat (1:1), 1 Diastereomer**

**Ket- 13** (0, 252 g, 0, 925 mmol) wurde zusammen mit **Ind- 1** (0, 15 g, 0, 925 mmol) in Dichlormethan (3, 6 ml) ultratrocken vorgelegt. Das Mikrowellengefäß wurde mittels eines Septums verschlossen und mit Stickstoff gespült. Anschließend erfolgte eine schnelle Zugabe von Trimethylsilyltrifluormethansulfonat (0, 715 ml, 3, 70 mmol) . Der Ansatz wurde 10 d bei RT gerührt. Anschließend wurde das Reaktionsgemisch mit 1 M wässriger $Na_2CO_3$- Lösung versetzt und 20 min gerührt. Die organische Phase wurde abgetrennt und die wässrige Phase mit Dichlormethan (3x) extrahiert. Die vereinigten organischen Extrakte wurden mit Wasser gewaschen und über $MgSO_4$ getrocknet. Nach Filtration des Trockenmittels wurde das Lösungsmittel am Rotationsverdampfer entfernt. Der erhaltene Feststoff wurde mit Methanol (7 ml) versetzt und das Gemisch 2 h bei Raumtemperatur gerührt. Der Feststoff wurde abgesaugt, mit wenig Methanol nachgewaschen und im Hochvakuum bei 50°C getrocknet (0, 248 g, 64, 2 %, cremefarbener Feststoff) .

Der Feststoff (0,234 g, 0,56 mmol) wurde in heißem Ethanol (5 ml) aufgeschlämmt und mit einer ebenfalls heißen Lösung von Citronensäure (0,108 g) in Ethanol (3 ml) versetzt. Die Lösung wurde über Nacht bei Raumtemperatur gerührt. Anschließend wurde der Niederschlag abgesaugt, mit wenig Ethanol und 2x mit Ether gewaschen und am Hochvakuum bei 60°C getrocknet.
**Ausbeute (Bsp. 19):** 0,280 g (82 %).
$^1$H NMR (600 MHz, $D_6$-DMSO) δ ppm: 1.35-1.55 (bs, 2H), 1.87 (bm, 4 H), 2. 20 (m, 2H), 2.62-2.74 (m, 10 H), 3.13 (bm, 2 H), 3.58 (bm, 4H), 3.92 (m, 2H), 7.00 (m, 1 H), 7.17 (m, 1 H), 7.32 (m, 1 H), 7.46 (d, 1 H), 7.80 (d, 1 H), 8.16 (d, 1 H), 11.66 (s, 1 H).

**Beispiel 20:**

**4-(Dimethylamino)-4-(3-methoxypropyl)-spiro[cyclohexan-1,8'-(5,6,8,9-Tetrahydro-pyrano[3,4-b]-7-aza-indol)]; Citrat (1:1), 1 Diastereomer**

**Ket- 14** (0, 197 g, 0, 925 mmol) wurde zusammen mit **Ind- 1** (0, 15 g, 0, 925 mmol) in Dichlormethan (3, 6 ml) ultratrocken vorgelegt. Das Mikrowellengefäß wurde mittels eines Septums verschlossen und mit Stickstoff gespült. Anschließend erfolgte eine schnelle Zugabe von Trimethylsilyltrifluormethansulfonat (0, 715 ml, 3, 70 mmol) . Der Ansatz wurde 13 d bei RT gerührt. Der Ansatz wurde 2 x 15 min bei 120°C in der Mikrowelle erhitzt. Anschließend wurde das Reaktionsgemisch mit 1 M wässriger $Na_2CO_3$- Lösung versetzt und 20 min gerührt. Die organische Phase wurde abgetrennt und die wässrige Phase mit Dichlormethan (3x) extrahiert. Die vereinigten organischen Extrakte wurden mit Wasser gewaschen und über $MgSO_4$ getrocknet. Nach Filtration des Trockenmittels wurde das Lösungsmittel am Rotationsverdampfer entfernt. Das erhaltene Öl wurde mittels Säulenchromatographie (Kieselgel KG 60; Dichlormethan : Methanol 4: 1) gereinigt (0, 1 g, 30, 2 %, cremefarbener Feststoff) .

Zur Fällung des Citrates wurde der Feststoff (0,095 g, 0,27 mmol) in heißem Ethanol (2 ml) suspendiert und mit einer ebenfalls heißen Lösung von Citronensäure (0,051 g) in Ethanol (2 ml) versetzt. Die Lösung wurde über Nacht bei Raumtemperatur gerührt. Anschließend wurde der Niederschlag abgesaugt, mit wenig Ethanol und 2x mit Ether gewaschen und am Hochvakuum bei 60°C getrocknet.

**Ausbeute (Bsp. 20):** 0,111 g (76 %).

__Beispiel 21:__

**4-(Dimethylamino)-4-phenyl-spiro[cyclohexan-1,8'-(5,6,8,9-Tetrahydro-pyrano[3,4-b]-7-aza-indol)]; Citrat (1:1), 1 Diastereomer**

Das Keton **Ket-1** (247 mg, 1,1 mmol) wurde zusammen mit **Ind-1** (184 mg, 1,1 mmol) in Dichlormethan (20 ml) gelöst. Anschließend erfolgte die Zugabe von Trifluormethansulfonsäure (363 mg, 0,215 ml, 2,42 mmol), wobei sich der Ansatz dunkel färbte. Es wurde 20 h bei RT gerührt. Zur Aufarbeitung wurde das Reaktionsgemisch mit 1 N NaOH (8 ml) versetzt und 10 min gerührt. Die Farbe wechselte dabei von dunkelrot nach hellbraun. Es fiel ein farbloser Feststoff aus. Der Feststoff (126 mg) wurde abgesaugt und mit Dichlormethan (5 ml) gewaschen. Die Phasen des Filtrats wurden getrennt. Die wäßrige Phase wurde mit Dichlormethan (2 × 10 ml) extrahiert. Die vereinigten organischen Phasen wurden mit Natriumsulfat getrocknet, filtriert und im Vakuum eingeengt. Es wurde ein hellbrauner Feststoff (491 mg) erhalten. Das Rohprodukt wurde mit Ethylacetat (10 ml) versetzt, dabei fiel ein farbloser Feststoff aus. Dieser wurde abgesaugt (119 mg) und mit Ethylacetat (10 ml) gewaschen. Die beiden Feststoffe waren identisch und wurden vereinigt (245 mg, 62 %) mit einem Schmelzpunkt von 266-274 °C erhalten.

Der Feststoff (215 mg, 0,59 mmol) wurde in Ethanol (70 ml) zum Sieden erhitzt. Zu der trüben Lösung wurde Citronensäure (274 mg, 1,43 mmol), gelöst in heißem Ethanol (5 ml), gegeben. Die Lösung wurde klar. Der nach 20-stündigem Rühren bei RT ausgefallene farblose Niederschlag wurde abgesaugt und mit Ethanol (10 ml) gewaschen. **Bsp. 21** wurde so in einer Ausbeute von 88 % (288 mg)

$^1$H NMR (400 MHz, $CD_3OD$) δ ppm: 1.01 (t, J = 7.01 Hz, 1.5H), 1.53-1.78 (m, 2H), 1.78-1.93 (m, 2H), 2.00-2.41 (m, 8H), 2.42-2.61 (m, 5H), 2.61-2.82 (m, 3H), 3.40 (d, J = 6.83 Hz, 1H), 3.85-4.02 (m, 2H), 6.87-7.02 (m, 1 H), 7.41-7.58 (m, 3H), 7.58-7.69 (m, 2H), 7.73 (d, J = 7.74 Hz, 1 H), 8.03 (d, J = 4.57 Hz, 1 H), 11.40 (s, 1 H)

$^{13}$C NMR (101 MHz, $CD_3OD$) δ ppm: 18.5, 21.6, 26.2, 31.1, 37.4, 44.0, 56.0, 59.0, 70.8, 71.3, 104.3, 114.9, 118.6, 125.5, 128.6, 128.8, 129.1, 138.7, 141.8, 148.5, 171.1, 176.4

__Beispiel 22:__

**4-(Dimethylamino)-4-thiophen-2-yl-spiro[cyclohexan-1,8'-(5,6,8,9-Tetrahydro-pyrano[3,4-b]-7-aza-indol)];Citrat (1:1), 1 Diastereomer**

Das Keton **Ket-2** (268 mg, 1,2 mmol) wurde zusammen mit dem Azatryptophol **Ind-1** (195 mg, 1,2 mmol) in 1,2-Dichlorethan (20 ml) gelöst. Anschließend erfolgte die Zugabe von Trifluormethansulfonsäure (397 mg, 0,235 ml, 2,64 mmol), wobei sich der Ansatz dunkellila färbte. Es wurde insgesamt 40 h bei RT gerührt. Zur Aufarbeitung wurde das Reaktionsgemisch mit 1 N NaOH (8 ml) auf pH 11 eingestellt und 15 min gerührt. Die Farbe wechselte dabei nach hellbraun, und es fiel ein hellbrauner Feststoff aus. Der Feststoff (140 mg) wurde abgesaugt und mit Methanol (15 ml) gewaschen. Dabei entfärbte sich das Produkt. Die Phasen des Filtrats wurden getrennt. Die wäßrige Phase wurde mit Dichlormethan (2 × 10 ml) extrahiert. Die vereinigten organischen Phasen wurden mit Natriumsulfat getrocknet, filtriert und im Vakuum eingeengt. Der feste braune Rückstand wurde mit Methanol (10 ml) 10 min gerührt, dann abgesaugt (58 mg) und mit Methanol (10 ml) gewaschen. Die beiden Feststoffe waren identisch und wurden vereinigt (198 mg, 45 %).

Der Feststoff (123 mg, 0,33 mmol) wurde in Ethanol (90 ml) zum Sieden erhitzt. Zu der trüben Lösung wurde Citronensäure (152 mg, 0,79 mmol), gelöst in heißem Ethanol (5 ml), gegeben. Die Lösung wurde klar. Sie wurde weitere 5 min gekocht, dann auf Raumtemperatur abgekühlt und im Vakuum auf ca. die Hälfte ihres Volumens eingeengt. Anschließend wurde die Lösung 20 h bei Raumtemperatur gerührt, wobei ein farbloser Niederschlag ausfiel. Er wurde abgesaugt und mit Ethanol (10 ml) gewaschen. **Bsp. 22** wurde in einer Ausbeute von 54 % (100 mg) mit einem Schmelzpunkt von 278-283 °C gewonnen.

$^1$H NMR (400 MHz, $CD_3OD$) δ ppm: 1.75-1.99 (m, 4H), 2.03-2.36 (m, 8H), 2.36-2.78 (m, 8H), 3.85-4.05 (m, 2H), 6.91-7.04 (m, 1H), 7.16-7.34 (m, 2H), 7.63-7.73 (m, 1H), 7.73-7.84 (m, 1 H), 8.04-8.15 (m, 1 H), 11.51 (s, 1 H)

**Beispiel 23:**

**4-(Dimethylamino)-4-phenyl-spiro[cyclohexan-1,6'-(5,6,8,9-Tetrahydro-pyrano[3,4-b]-5-aza-indol)], Citrat (1:1), unpolares Diastereoemer**

**Beispiel 24:**

**4-(Dimethylamino)-4-phenyl-spiro[cyclohexan-1,6'-(5,6,8,9-Tetrahydro-pyrano[3,4-b]-5-aza-indol)], Citrat (1:1), polares Diastereomer**

**4-(Dimethylamino)-4-phenyl-spiro[cyclohexan-1,6'-(5,6,8,9-Tetrahydro-pyrano[3,4-b]-5-aza-indol)]** (unpolares und polares Diastereomer)

Das Keton **Ket- 1** (267 mg, 1, 23 mmol) und das 5- Azatryptophol (**Ind- 2**) (200 mg, 1, 23 mmol) wurden in abs. 1, 2- Dichlorethan (40 ml) gelöst, mit Trifluormethansulfonsäure (0, 12 ml, 204 mg, 1, 36 mmol) versetzt und 16 h bei Raumtemperatur gerührt. Im Reaktionskolben setzte sich ein helles Öl ab. Es wurde nochmals Trifluormethansulfonsäure (0, 12 ml, 204 mg, 1, 36 mmol) hinzugefügt und 5 h unter Rückfluß erwärmt. Der Ansatz wurde bei Raumtemperatur mit Wasser (30 ml) und 1 N Natronlauge (10 ml) versetzt und 30 min gerührt. Die Phasen wurden getrennt. Die wäßrige Phase wurde mit 1, 2- Dichlorethan (3 × 30 ml) extrahiert. Die organischen Phasen wurden vereinigt, mit Natriumsulfat getrocknet und eingeengt. Der Rückstand war ein beigefarbener Feststoff (455 mg), der chromatographisch aufgetrennt wurde [Kieselgel 60 (120 g) ; Ethylacetat/ Methanol 4 : 1 (500 ml), Ethylacetat/ Methanol 1 : 1 (500 ml), Methanol (300 ml) ] . 4- (Dimethylamino)- 4- phenyl- spiro [cyclohexan- 1, 6'- (5, 6, 8, 9- Tetrahydro- pyrano [3, 4- b]- 5- aza- indol) ] (unpolareres Diastereomer) wurde als farbloser Feststoff in einer Ausbeute von 30 % (133 mg) gewonnen. Das polarere Diastereomer war verunreinigt (158 mg, 35 %) .

**4-(Dimethylamino)-4-phenyl-spiro[cyclohexan-1,6'-(5,6,8,9-Tetrahydro-pyrano[3,4-b]-5-aza-indol)], Citrat (1:1), unpolares Diastereoemer (Bsp. 23)**

4- (Dimethylamino)- 4- phenyl- spiro [cyclohexan- 1, 6'- (5, 6, 8, 9- Tetrahydro- pyrano [3, 4- b]- 5- aza- indol) ] (unpolareres Diastereomer) (120 mg, 0, 332 mmol) wurde bei 60 °C in Ethanol (15 ml) gelöst und mit einer ethanolischen Lösung (2 ml) von Citronensäure (140 mg, 0, 73 mmol) versetzt. Nach 22 h wurde Diethylether (20 ml) hinzugegeben. Nach 30 min wurde der farblose Feststoff durch Filtration abgetrennt und mit Ethanot/ Ethylacetat 1 : 1 (3 ml) und Diethylether (2 ml) gewaschen. **Bsp. 23** wurde in einer Ausbeute von 56 % (102 mg) mit einem Schmelzpunkt von 273- 277 °C erhalten.
$^1$H NMR (300 MHz, DMSO- $d_6$) δ ppm: 1.80- 2.04 (m, 2H), 2.18- 2.4 (m, 4H), 2.48 (s, 6H), 2.57 (dd, 4H), 2.79 (t, 2H), 3.91 (t, 2H), 7.15- 7.3 (m, 6H), 8.24 (d, 1 H), 8.94 (s, 1 H) 12.17 (s, 1 H)

**4-(Dimethylamino)-4-phenyl-spiro[cyclohexan-1,6'-(5,6,8,9-Tetrahydro-pyrano[3,4-b]-5-aza-indol)], Citrat (1:1), polares Diastereomer (Bsp. 24)**

4- (Dimethylamino)- 4- phenyl- spiro [cyclohexan- 1, 6'- (5, 6, 8, 9- Tetrahydro- pyrano [3, 4- b]- 5- aza- indol) ] (polareres Diastereomer, verunreinigt (140 mg, 0, 38 mmol) wurde in Ethanol (7 ml) gelöst und mit Citronensäure (163 mg, 0, 85 mmol), gelöst in Ethanol (2 ml), versetzt. Eine Fällung setzte sofort ein. Nach 2 h wurde ein farbloser Feststoff (103 mg) durch Filtration abgetrennt und mit Ethanol (2 × 2 ml) und Diethylether (2 ml) gewaschen. Das Filtrat wurde mit Diethylether (10 ml) versetzt, der entstandene Niederschlag abgetrennt und mit Diethylether (2 × 2 ml) gewaschen (38 mg) . Die zwei Feststoffraktionen wurden vereinigt und mit Natronlauge und Trichlormethan versetzt (107 mg) Der resultierende Feststoff wurde in Ethanol (30 ml) mit Citronensäure (124 mg, 0, 65 mmol), gelöst in Ethanol (3 ml), versetzt. Nach 20 min wurde ein Feststoff abgetrennt und mit Ethanol (2 × 3 ml) und Diethylether (2 × 2 ml) gewaschen (59 mg) . Das Filtrat wurde mit Diethylether (30 ml) versetzt und 16 h bei Raumtemperatur gerührt. Durch Filtration und Waschen mit Diethylether (2 × 2 ml) wurde ein farbloser Feststoff isoliert.
**Ausbeute (Bsp. 24) :** 0, 040 g (25 %), Schmelzpunkt von 122- 127 °C.
$^1$H NMR (300 MHz, DMSO- $d_6$) δ ppm: 1.63 (t, 2H), 1.92 (d, 2H), 2.13- 2.27 (m, 2H), 2.36 (s, 6H), 2.57 (dd, 4H), 2.68- 2.83 (m, 4H), 3.99 (t, 2H), 7.32 (d, 1 H), 7.49- 7.63 (m, 3H), 7.66- 7.73 (m, 2H), 8.15 (d, 1 H), 8.83 (s, 1 H), 11.50 (s, 1 H)

**Beispiel 25:**

**4-Butyl-4-(dimethylamino)-spiro[cyclohexan-1,8'-(5,6,8,9-Tetrahydro-pyrano[3,4-b]-7-aza-indol)]; Citrat (1:1), unpolares Diastereomer**

**Beispiel 26:**

**4-Butyl-4-(dimethylamino)-spiro[cyclohexan-1,8'-(5,6,8,9-Tetrahydro-pyrano[3,4-b]-7-aza-indol)]; Citrat (1:1), polares Diastereomer**

**4-Butyl-4-(dimethylamino)-spiro[cyclohexan-1,8'-(5,6,8,9-Tetrahydro-pyrano[3,4-b]-7-aza-indol)]** (unpolares und polares Diastereomer)

Das Keton **Ket- 9** (462 mg, 2, 34 mmol) wurde zusammen mit dem Azatryptophol **Ind- 1** (380 mg, 2, 34 mmol) in 1, 2- Dichlorethan (40 ml) gelöst. Anschließend erfolgte die Zugabe von Trifluormethansulfonsäure (773 mg, 0, 457 ml, 5, 15 mmol), wobei sich der Ansatz verfärbte. Es wurde insgesamt 4 d bei RT gerührt. Zur Aufarbeitung wurde das Reaktionsgemisch mit Wasser (10 ml) versetzt, mit 1 N NaOH (10 ml) auf pH 11 eingestellt und 15 min gerührt. Anschließend wurden die Phasen getrennt. Die wäßrige Phase wurde mit Dichlormethan (2 × 10 ml) extrahiert. Die vereinigten organischen Extrakte wurden mit Natriumsulfat getrocknet, filtriert und im Vakuum eingeengt. Der braune ölige Rückstand wurde mit Methanol (10 ml) versetzt, wobei ein farbloser Feststoff ausfiel. Dieser wurde abgesaugt und mit Methanol (10 ml) gewaschen. Es handelte sich hierbei um 4- Butyl- 4- (dimethylamino)- spiro [cyclohexan- 1, 8'- (5, 6, 8, 9- Tetrahydro- pyrano [3, 4- b]- 7- aza- indol) ] (unpolareres Diastereoisomer) (39 mg, 5 %, geringfügig verunreinigt) . Die Mutterlauge wurde eingeengt und chromatographisch aufgetrennt [Kieselgel 60 (50 g) ; Ethylacetat (1200 ml), Ethylacetat/ Methanol 4 : 1 (500 ml), 1 : 1 (500 ml) ] . Es wurde ein gelber Feststoff (239 mg) erhalten, der mit Chloroform (25 ml), 1 N NaOH (5 ml) und Wasser (10 ml) 30 min kräftig gerührt wurde. Anschließend wurden die Phasen getrennt. Die wäßrige Phase wurde mit Chloroform (2 × 10 ml) extrahiert. Die vereinigten organischen Extrakte wurden mit Natriumsulfat getrocknet, filtriert und im Vakuum eingeengt. Der gelbe halbfeste Rückstand (230 mg, 30 %) war das polarere Diastereoisomer, das ca. 24 % des unpolareren Produktes enthielt. Die Trennung sollte über die Citratbildung erfolgen.

**4-Butyl-4-(dimethylamino)-spiro[cyclohexan-1,8'-(5,6,8,9-Tetrahydro-pyrano[3,4-b]-7-aza-indol)]; Citrat (1:1), unpolares Diastereomer (Bsp. 25)**

4- Butyl- 4- (dimethylamino)- spiro [cyclohexan- 1, 8'- (5, 6, 8, 9- Tetrahydro- pyrano [3, 4- b]- 7- aza- indol) ] (unpolareres Diastereoisomer) (27 mg, 0, 08 mmol) wurde in Ethanol (20 ml) unter leichtem Erwärmen gelöst und mit Citronensäure (37 mg, 0, 192 mmol), gelöst in heißem Ethanol (5 ml), versetzt. Nach 20- stündigem Rühren bei RT wurde die klare Lösung fast vollständig eingeengt und mit Diethylether (5 ml) versetzt. Es begann ein farbloser Feststoff auszufallen. Dieser wurde abgesaugt.
**Ausbeute (Bsp. 25) :** 38 mg (88 %) .
**Schmelzpunkt:** 86- 92 °C.

**4-Butyl-4-(dimethylamino)-spiro[cyclohexan-1,8'-(5,6,8,9-Tetrahydro-pyrano[3,4-b]-7-aza-indol)]; Citrat (1:1), polares Diastereomer (Bsp. 26)**

4- Butyl- 4- (dimethylamino)- spiro [cyclohexan- 1, 8'- (5, 6, 8, 9- Tetrahydro- pyrano [3, 4- b]- 7- aza- indol) ] (unpolareres Diastereoisomer) (verunreinigt mit unpolareren Diastereomer, 230 mg, 0, 67 mmol) wurde in Ethanol (20 ml) unter leichtem Erwärmen gelöst und mit Citronensäure (310 mg, 1, 62 mmol), gelöst in heißem Ethanol (5 ml), versetzt. Nach einstündigem Rühren bei RT wurde die klare Lösung auf ca. ein Drittel ihres Volumens eingeengt und mit Diethylether (10 ml) versetzt. Es begann ein farbloser Feststoff auszufallen. Dieser wurde abgesaugt.
**Ausbeute (Bsp. 26) :** 91 mg, (25 %)
**Schmelzpunkt:** 95- 103 °C.

**Beispiel 27:**

**4-Benzyl-4-morpholino-spiro[cyclohexan-1,8'-(3-trifluoromethyl-5,6,8,9-Tetrahydro-pyrano[3,4-b]-7-aza-indol)]; Citrat (1:1), 1 Diastereomer**

**Ket- 13** (0, 593 g, 2, 17 mmol) wurde zusammen mit **Ind- 3** (0, 5 g, 2, 17 mmol) in Dichlormethan (11 ml)

ultratrocken vorgelegt. Das Mikrowellengefäß wurde mittels eines Septums verschlossen und mit Stickstoff gespült. Anschließend erfolgte eine schnelle Zugabe von Trimethylsilyltrifluormethansulfonat (1, 68 ml, 8, 69 mmol) . Der Ansatz wurde 10 min bei 120°C in der Mikrowelle erhitzt. Anschließend wurde das Reaktionsgemisch mit 1 M wässriger $Na_2CO_3$-Lösung versetzt und 20 min gerührt. Die organische Phase wurde abgetrennt und die wässrige Phase mit Dichlormethan (3x) extrahiert. Die vereinigten organischen Extrakte wurden mit Wasser und gesättigter NaCl- Lösung gewaschen und über $MgSO_4$ getrocknet. Nach Filtration des Trockenmittels wurde das Lösungsmittel am Rotationsverdampfer entfernt. Der erhaltene schaumige Feststoff wurde mittels Säulenchromatographie (Kieselgel KG 60; Dichlormethan : Essigester 9: 1) gereinigt. Es resultierte 4- Benzyl- 4- morpholino- spiro [cyclohexan- 1, 8'- (3- trifluoromethyl- 5, 6, 8, 9- Tetrahydro-pyrano [3, 4- b]- 7- aza- indol) ] (0, 12 g, 8, 2 %) als cremefarbener Feststoff.

Der Feststoff (0,120 g, 0,25 mmol) wurde in heißem Ethanol (15 ml) gelöst und mit Citronensäure (0,051 g) und mit Diethylether (10 ml) versetzt. Die Lösung wurde über Nacht bei Raumtemperatur gerührt. Das Lösungsmittel wurde am Rotationsverdampfer entfernt. Der Rückstand wurde mit n-Hexan / Ethanol (10 ml; 9,5 : 0,5) kristallisiert. Der Feststoff wurde abgesaugt und am Hochvakuum bei 60°C getrocknet.
**Ausbeute (Bsp. 27):** 0,173 g (100 %).

**Beispiel 28:**

**4-(Dimethylamino)-4-phenyl-spiro[cyclohexan-1,8'-(3-trifluoromethyl-5,6,8,9-Tetrahydro-pyrano[3,4-b]-7-aza-indol)]; Citrat (1:1), 1 Diastereomer**

**Ket- 1** (0, 283 g, 1, 30 mmol) wurde zusammen mit **Ind- 3** (0, 3 g, 1, 30 mmol) in Dichlormethan (3, 4 ml) ultratrocken vorgelegt. Das Mikrowellengefäß wurde mittels eines Septums verschlossen und mit Stickstoff gespült. Anschließend erfolgte eine schnelle Zugabe von Trimethylsilyltrifluormethansulfonat (1, 01 ml, 5, 2 mmol) . Der Ansatz wurde 10 min bei 120°C in der Mikrowelle erhitzt. Anschließend wurde das Reaktionsgemisch mit 1 M wässriger $Na_2CO_3$-Lösung versetzt und 20 min gerührt. Die organische Phase wurde abgetrennt und die wässrige Phase mit Dichlormethan (3x) extrahiert. Die vereinigten organischen Extrakte wurden mit Wasser und gesättigter NaCl- Lösung gewaschen und über $MgSO_4$ getrocknet. Nach Filtration des Trockenmittels wurde das Lösungsmittel am Rotationsverdampfer entfernt. Der erhaltene schaumige Feststoff wurde mittels Säulenchromatographie (Kieselgel KG 60; Dichlormethan : Methanol 9: 1) gereinigt (0, 116 g, 20, 7 %) .

Zur Fällung des Citrates wurde der Feststoff (0,100 g, 0,23 mmol) in heißem Ethanol (2 ml) gelöst und mit Citronensäure (0,058 g) und Diethylether (45 ml) versetzt. Die wurde bei Raumtemperatur 1 nachgerührt. Der Feststoff wurde abgesaugt und 5 h am Hochvakuum bei 60°C getrocknet.
**Ausbeute (Bsp. 28):** 0,108 g (75 %).

**Beispiel 29:**

**4-(Azetidin-1-yl)-4-phenyl-spirofcyclohexan-1,8'-(5,6,8,9-Tetrahydro-pyrano[3,4-b]-7-aza-indol)], Citrat (1:1), 1 Diastereomer**

Das Keton **Ket-15** (275 mg, 1,2 mmol) wurde zusammen mit dem Azatryptophol **Ind-1** (195 mg, 1,2 mmol) in Dichlormethan (30 ml) gelöst. Anschließend erfolgte die Zugabe von Trifluormethansulfonsäure (397 mg, 0,235 ml, 2,64 mmol), wobei sich der Ansatz dunkel färbte. Es wurde 20 h bei RT gerührt. Zur Aufarbeitung wurde das Reaktionsgemisch mit 1 N NaOH (10 ml) und Wasser (10 ml) versetzt und 10 min gerührt. Die Farbe wechselte dabei von dunkelrot nach hellbraun. Die Phasen des Filtrats wurden getrennt. Die wäßrige Phase wurde mit Dichlormethan (2 $\times$ 10 ml) extrahiert. Die vereinigten organischen Phasen wurden mit Natriumsulfat getrocknet, filtriert und im Vakuum eingeengt. Es wurde ein gelber Feststoff erhalten, der mit Dichlormethan (5 ml) 10 min gerührt, dann abgesaugt und mit Dichlormethan (5 ml) gewaschen wurde(116 mg, 26 %, diastereoisomerenrein, Schmelzpunkt: 269-274 °C).

Der Feststoff (101 mg, 0,27 mmol) wurde in Ethanol (60 ml) zum Sieden erhitzt. Zu der trüben Lösung wurde Citronensäure (125 mg, 0,65 mmol), gelöst in heißem Ethanol (5 ml), gegeben. Die Lösung wurde klar. Nach 20-stündigem Rühren bei RT wurde die Lösung auf ca. 3 ml eingeengt und mit Diethylether (5 ml) bis zur Kristallisation versetzt. Der ausgefallene farblose Niederschlag wurde abgesaugt und mit Diethylether (5 ml) gewaschen.
**Ausbeute (Bsp. 29):** 80 mg, (53 %)
**Schmelzpunkt:** 208-212 °C.
$^1$H NMR (300 MHz, DMSO-$d_6$) $\delta$ ppm: 1.50-1.81 (m, 2H), 1.81-2.04 (m, 4H), 2.04-2.25 (m, 2H), 2.30-2.50 (m, 2H), 2.56 (dd, J = 26.01, 15.16 Hz, 4H), 2.64-2.77 (m, 2H), 3.47-3.77 (m, 4H), 3.97 (t, J = 5.08 Hz, 2H), 6.89-7.07 (m, 1 H), 7.46-7.89 (m, 6H), 8.00-8.16 (m, 1 H), 11.44 (s, 1 H)
$^{13}$C NMR (101 MHz, DMSO-$d_6$) $\delta$ ppm: 15.3, 21.6, 24.2, 30.6, 44.0, 47.6, 59.0, 62.8, 71.1, 71.3, 104.2, 114.9, 118.6, 125.5, 128.5, 128.9, 132.3, 138.8, 141.8, 148.5, 171.1, 176.5

**Beispiel 30:**

**4-Butyl-4-(pyrrolidin-1-yl)-spiro[cyclohexan-1,8'-(5,6,8,9-Tetrahydro-pyrano[3,4-b]-7-aza-indol)]; Citrat (2:1), 1 Diastereomer**

Das Keton **Ket- 16** (268 mg, 1, 2 mmol) und das 7- Azatryptophol (**Ind- 1**, 195 mg, 1, 2 mmol) wurden in abs. 1, 2- Dichlorethan (40 ml) gelöst und mit Trifluormethansulfonsäure (0, 117 ml, 198 mg, 1, 32 mmol) versetzt und 3 d bei Raumtemperatur gerührt. Es wurde 7 h auf 80 °C (Badtemeperatur) erhitzt, dann Trifluormethansulfonsäure (0, 117 ml, 198 mg, 1, 32 mmol) hinzugefügt und 8 h bei 80 °C gerührt.Das Gemisch wurde weitere 5 Tage bei Raumtemperatur gerührt. Der Ansatz wurde mit Wasser (15 ml) und 1 N Natronlauge (5 ml) versetzt und 15 min gerührt. Die Phasen wurden getrennt. Die wäßrige Phase wurde mit 1, 2- Dichlorethan (2 × 20 ml) extrahiert. Die organischen Phasen wurden vereinigt, mit Natriumsulfat getrocknet und eingeengt. Der Rückstand war ein beigefarbener Feststoff (424 mg), der in Ethylacetat/ Methanol 4 : 1 (3 ml) aufgenommen wurde. Dabei begann ein farbloser Feststoff auszufallen. Es wurde Ethylacetat (5 ml) zugestzt und der Feststoff durch Filtration abgetrennt. Er wurde 4- Butyl- 4- (pyrrolidin- 1- yl)- spiro [cyclohexan- 1, 8'- (5, 6, 8, 9- Tetrahydro- pyrano [3, 4- b]- 7- aza- indol) ] (diastereoisomerenrein) in einer Ausbeute von 12 % (52 mg) mit einem Schmelzpunkt von 246- 250 °C erhalten.

Das Filtrat wurde eingeengt und chromatographisch aufgetrennt [Kieselgel 60 (50 g); Ethylacetat/Methanol 4 : 1 (500 ml), Methanol (200 ml)] und auf diese Weise weiteres Produkt gewonnen (107 mg, 24 %).

Der Feststoff (91 mg, 0,247 mmol) wurde bei 30 °C in Ethanol (7 ml) gelöst und mit einer ethanolische Lösung von Citronensäure (105 mg, 0,545 mmol, 3 ml) versetzt. Nach 2 h wurde die Lösung mit Ethylether (50 ml) versetzt und 2 d bei Raumtemperatur gerührt. Dabei entstand ein hellbrauner Niederschlag, der durch Filtration abgetrennt wurde (50 mg). Das Filtrat wurde eingeengt, in Ethanol (1 ml) aufgenommen, mit Diethylether (20 ml) versetzt und 30 min gerührt. Dabei fiel ein farbloser Feststoff aus, der filtriert und mit Diethylether (2 × 2 ml) gewaschen wurde (50 mg). Beide Feststoffe waren identisch.

**Ausbeute (Bsp. 30):** 0,100 g (54 %).

$^{1}$H NMR (300 MHz, DMSO-$d_6$) $\delta$ ppm: 1.02 (t 3H), 1.25-1.53 (m, 5H), 1.63-1.75 (m, 2H), 1.79-2.08 (m 15H), 2.58-2.74 (m 6H), 3.93 (t 2H), 6.99-7.07 (m 1 H), 7.79-7.86 (m 1 H), 8.13-8.18 (m 1 H).

**Beispiel 31:**

**4-(Dimethylamino)-4-phenyl-spiro[cyclohexan-1,8'-(5,6,8,9-Tetrahydro-thiopyrano[3,4-b]-7-aza-indol)]; Citrat (1:1), 1 Diastereomer**

**Ind- 4** (258 mg, 1, 2 mmol) wurde zusammen mit dem Keton **Ket- 1** (261 mg, 1, 2 mmol) in abs. 1, 2- Dichlorethan (24 ml) unter Argon gelöst und mit Trifluormethansulfonsäure (0, 44 ml, 4, 96 mmol) versetzt. Der Ansatz wurde 1 h bei 150 °C in der Mikrowelle gerührt. Zur Aufarbeitung wurde die Reaktionslösung mit gesättigter Natriumhydrogencarbonat-Lösung (10 ml) versetzt. Das Gemisch wurde weitere 15 min gerührt. Nach Trennung der Phasen wurde die wäßrige Phase mit Dichlormethan (3 × 10 ml) extrahiert. Die vereinigten organischen Extrakte wurden über $Na_2SO_4$ getrocknet und anschließend eingeengt. Das erhaltene Rohprodukt (346 mg, gelber Schaum) wurde säulenchromatographisch gereinigt [Kieselgel 60 (20 g) ; Cyclohexan/ Ethylacetat 4 : 1 (500 ml), Cyclohexan/ Ethylacetat 1 : 1 (1000 ml) ] . 4-(Dimethylamino)- 4- phenyl- spiro [cyclohexan- 1, 8'- (5, 6, 8, 9- Tetrahydro- thiopyrano [3, 4- b]- 7- aza- indol) ] wurde als beigefarbener Feststoff (87 mg, 19 %, Schmp.: 246- 253 °C, diastereoisorenrein) erhalten Aus der wäßrigen Phase war über Nacht weiteres Produkt ausgefallen. Der Feststoff wurde filtriert und mit Wasser (3 × 0, 5 ml) und mit Methanol (2 × 0, 5 ml) gewaschen (51 mg, 11 % beigefarbener Feststoff, , Schmelzpunkt 262- 267 °C) .

4- (Dimethylamino)- 4- phenyl- spiro [cyclohexan- 1, 8'- (5, 6, 8, 9- Tetrahydro- thiopyrano [3, 4- b]- 7- aza-indol) ] (135 mg, 0, 358 mmol, diastereoisomerenrein) wurde in Dichlormethan (6 ml) gelöst und mit Citronensäure (78 mg, 0, 406 mmol), gelöst in Ethylacetat (12 ml), versetzt. Während der Zugabe der Säure fiel ein Niederschlag aus. Anschließend wurde die Mischung 17 h bei 23 °C gerührt, dann filtriert und der Niederschlag mit Ethylacetat (3 × 0, 5 ml) gewaschen.

**Ausbeute (Bsp. 31) :** 188 mg (92 %), weißer Feststof.

**Schmelzpunkt:** 130- 136 °C.

$^{1}$H NMR (400 MHz, $CD_3OD$) $\delta$ ppm: 2.01- 2.15 (m, 4H), 2.52- 2.71 (m, 7H), 2.71- 2.90 (m, 5H), 2.90- 3.05 (m, 6H), 7.02 (dd, J = 7.79, 4.86 Hz, 1H), 7.59- 7.72 (m, 3H), 7.74- 7.81 (m, 2H), 7.81- 7.88 (m, 1 H), 8.08 (d, J = 4.40 Hz, 1 H)

$^{13}$C NMR (101 MHz, $CD_3OD$) $\delta$ ppm: 24.4, 25.2, 28.4, 34.9, 38.4, 44.7, 45.4, 70.5, 74.2, 110.0, 116.4, 121.9, 127.9, 130.7, 130.8, 130.9, 131.5, 138.8, 143.1, 148.6, 174.7, 179.0

**Beispiel 32:**

**4-(Dimethylamino)-4-phenyl-spiro[cyclohexan-1,6'-(5,6,8,9-Tetrahydro-thiopyrano[3,4-b]-5-aza-indol)], Citrat (1:1), 1 Diastereomer**

**Ind- 5** (430 mg, 2, 0 mmol) wurde zusammen mit Keton **Ket- 1** (435 mg, 2, 0 mmol) in abs. 1, 2- Dichlorethan (25 ml) unter Argon gelöst und mit Trifluormethansulfonsäure (0, 533 ml, 6, 0 mmol) versetzt. Der Ansatz wurde 1 h bei 150 °C in der Mikrowelle gerührt. Zur Aufarbeitung wurde die Reaktionslösung mit 1 N Natriumhydroxyd- Lösung (20 ml) versetzt. Das Gemisch wurde weitere 15 min gerührt. Nach Trennung der Phasen wurde die wäßrige Phase mit Dichlormethan (3 × 20 ml) extrahiert. Die vereinigten organischen Extrakte wurden über $Na_2SO_4$ getrocknet und anschließend eingeengt. Das erhaltene Rohprodukt (715 mg, brauner Feststoff) wurde säulenchromatographisch gereinigt [Kieselgel 60 (30 g) ; Cyclohexan/ Ethylacetat 4 : 1 (500 ml), Cyclohexan/ Ethylacetat 2 : 1 (600 ml), Ethylacetat/ Methanol 100 : 1 (1000 ml), Ethylacetat/ Methanol 5 : 1 (600 ml) ] . 4- (Dimethylamino)- 4- phenyl- spiro [cyclohexan- 1, 6'- (5, 6, 8, 9- Tetrahydro- thiopyrano [3, 4- b]- 5- aza- indol) ] wurde als fast weißer Feststoff (320 mg, 42 %, Schmp.: 163- 168 °C, diastereoisomerenrein) erhalten.

Der Feststoff (233 mg, 0,617 mmol) wurde in heißem Isopropanol (10 ml) gelöst und mit Citronensäure (130 mg, 0,677 mmol), gelöst in heißem Isopropanol (10 ml), versetzt. Während der Zugabe der Säure fiel ein Niederschlag aus. Anschließend wurde die Mischung 2 h bei 5 °C gerührt, dann filtriert und der Niederschlag mit Isopropanol (2 × 0,5 ml) und Aceton (3 × 0,5 ml) gewaschen.

**Ausbeute (Bsp. 32):** 350 mg (97%), weißer Feststoff.

**Schmelzpunkt:** 132-140 °C.

$^1H$ NMR (400 MHz, DMSO-$d_6$) δ ppm: 1.04 (d, J = 5.87 Hz, 1.5H), 1.82-2.03 (m, 4H), 2.16-2.39 (m, 7H), 2.49-2.70 (m, 5H), 2.75-2.91 (m, 2H), 2.91-3.14 (m, 4H), 3.68-3.86 (m, 0.25H), 7.24-7.44 (m, 1H), 7.44-7.84 (m, 5H), 8.07-8.27 (m, 1H), 8.74-8.94 (m, 1H), 11.47 (s, broad, 1 H)

$^{13}C$ NMR (101 MHz, DMSO-$d_6$) δ ppm: 23.3, 23.5, 25.4, 27.4, 33.4, 37.5, 44.0, 44.6, 62.0, 65.3, 71.5, 107.1, 109.8, 123.8, 126.6, 127.5, 128.6, 128.7,131.9, 136.7, 138.1, 139.3,140.4, 171.3, 176.5

**Beispiel 33:**

**4-(1-Methyl-1H-1,2,4-triazol-5-yl)-4-(methylamino)-spiro[cyclohexan-1,8'-(5,6,8,9-Tetrahydro-pyrano[3,4-b]-7-aza-indol)], Citrat (1:1), polares Diastereomer**

**Beispiel 34:**

**4-(1-Methyl-1H-1,2,4-triazol-5-yl)-4-(methylamino)-spiro[cyclohexan-1,8'-(5,6,8,9-Tetrahydro-pyrano[3,4-b]-7-aza-indol)], Citrat (1:1), unpolares Diastereomer**

**4-(1-Methyl-1H-1,2,4-triazol-5-yl)-d-(methylamino)-spiro[cyclohexan-1,8'-(5,6,8,9-Tetrahydro-pyrano[3,4-b]-7-aza-indol)] (unpolares und polares Diastereomer)**

Das Keton **Ket-17** (250 mg, 1,2 mmol) und das 7-Azatryptophol (**Ind-1**, 195 mg, 1,2 mmol) wurden in abs.1,2-Dichlorethan (30 ml) gelöst und unter Argon mit Methansulfonsäure (0,467 ml, 692 mg, 7,2 mmol) versetzt. Nach 17 h wurde der Ansatz 7 h bei einer Badtemperatur von 90 °C und 15 h bei 75 °C gerührt. Das Reaktionsgemisch wurde mit 1 N Natronlauge (15 ml) und Wasser (10 ml) versetzt und 15 min gerührt. Die Phasen wurden getrennt. Die wäßrige Phase wurde mit 1,2-Dichlorethan (2 × 20 ml) extrahiert. Die organischen Phasen wurden vereinigt, mit Natriumsulfat getrocknet und eingeengt. Der Rückstand war ein beigefarbener Feststoff, der in Ethylacetat (5 ml) aufgenommen wurde. Dabei begann ein farbloser Feststoff auszufallen, der durch Filtration und Waschen mit Ethylacetat (2 ml) abgetrennt wurde.

Erhalten wurde 4- (1- Methyl- 1H- 1, 2, 4- triazol- 5- yl)- 4- (methylamino)- spiro [cyclohexan- 1, 8'- (5, 6, 8, 9- Tetrahydro- pyrano [3, 4- b]- 7- aza- indol) ] als Diastereoisomerengemisch (130 mg) . Das Filtrat wurde eingeengt und chromatographisch aufgetrennt [Kieselgel 60 (30 g) ; Ethylacetat/ Methanol 6 : 1 (700 ml) ] (220 mg, farbloser Feststoff, Diastereoisomerengemisch) .

Die beiden Fraktionen wurden erneut chromatographiert [Kieselgel 60 (20 g) ; Chloroform/ Methanol 30 : 1 (500 ml) ] und dabei 4- (1- Methyl- 1 H- 1, 2, 4- triazol- 5- yl)- 4- (methylamino)- spiro [cyclohexan- 1, 8'- (5, 6, 8, 9- Tetrahydro- pyrano [3, 4- b]- 7- aza- indol) ] (unpolareres Diastereoisomer) in einer Ausbeute von 40 % (164 mg) als farbloser Feststoff mit einem Schmelzpunkt von 282- 287 °C gewonnen. Das polarere Diastereoisomer wurde in einer Ausbeute von 11 % (48 mg) mit einem Schmelzpunkt von 285- 289 °C erhalten.

**4-(1-Methyl-1H-1,2,4-triazol-5-yl)-4-(methylamino)-spiro[cyclohexan-1,8'-(5,6,8,9-Tetrahydro-pyrano[3,4-b]-7-aza-indol)], Citrat (1:1), polares Diastereomer (Bsp. 33)**

4- (1- Methyl- 1 H- 1, 2, 4- triazol- 5- yl)- 4- (methylamino)- spiro [cyclohexan- 1, 8'- (5, 6, 8, 9- Tetrahydro-pyrano [3, 4- b]- 7- aza- indol) ] (polares Diastereomer) (48 mg, 0, 136 mmol) wurde unter Erwärmen in Ethanol (20 ml) gelöst und mit Citronensäure (58 mg, 0, 3 mmol), gelöst in Ethanol (2 ml), versetzt. Nach 3 Tagen Rühren bei Raumtemperatur wurde die klare Lösung fast vollständig eingeengt und der Rückstand mit Diethylether (5 ml) bis zur Kristallisation versetzt.
**Ausbeute (Bsp. 33) :** 71 mg (96 %), farbloser Feststoff.
**Schmelzpunkt:** 90- 93 °C.
$^1$H NMR (300 MHz, DMSO- $d_6$) δ ppm: 1.72- 1.92 (m, 2H), 1.92- 2.02 (m, 2H), 2.04 (s, 3H), 2.07- 2.19 (m, 4H), 2.56- 2.83 (m, 10H), 3.91 (t, J = 5.17 Hz, 2H), 4.10 (s, 3H), 6.94- 7.09 (m, 1 H), 7.78 (s, 1 H), 7.79- 7.86 (m, 1 H), 8.10- 8.22 (m, 1 H), 11.35 (s, 1 H)
$^{13}$C NMR (101 MHz, DMSO- $d_6$) δ ppm: 21.7, 27.0, 28.9, 29.0, 37.2, 42.8, 55.4, 59.1, 70.7, 72.3, 104.1, 115.0, 118.7, 125.7, 139.8, 141.8, 148.2, 148.9, 158.1, 171.2, 174.7

**4-(1-Methyl-1H-1,2,4-triazol-5-yl)-4-(methylamino)-spiro[cyclohexan-1,8'-(5,6,8,9-Tetrahydro-pyrano[3,4-b]-7-aza-indol)], unpolares Diastereomer (Bsp. 34)**

4- (1- Methyl- 1H- 1, 2, 4- triazol- 5- yl)- 4- (methylamino)- spiro [cyclohexan- 1, 8'- (5, 6, 8, 9- Tetrahydro-pyrano [3, 4- b]- 7- aza- indol) ] (unpolares Diastereomer) (149 mg, 0, 423 mmol) wurde in Ethanol (20 ml) gelöst und mit Citronensäure (179 mg, 0, 93 mmol), gelöst in Ethanol (2 ml), versetzt. Nach 3 d Rühren bei Raumtemperatur konnte das Citrat 8K/ 9K als farbloser Feststoff abgesaugt werden.
**Ausbeute (Bsp. 34) :** 74 mg, (91 %) .
**Schmelzpunkt:** 192- 193 °C.
$^1$H NMR (300 MHz, DMSO- $d_6$) δ ppm: 1.80- 2.10 (m, 6H), 2.11 (s, 3H), 2.52- 2.77 (m, 8H), 3.95 (t, J = 5.27 Hz, 2H), 4.08 (s, 3H), 6.93- 7.04 (m, 1 H), 7.73- 7.83 (m, 1 H), 7.99 (s, 1 H), 8.06- 8.16 (m, 1 H), 11.41 (s, 1 H)
$^{13}$C NMR (101 MHz, DMSO- $d_6$) δ ppm: 21.7, 27.7, 29.0, 31.2, 38.0, 38.9, 43.4, 57.0, 59.0, 71.2, 71.8, 104.1, 114.9, 118.6, 125.5, 139.2, 141.8, 148.5, 149.3, 152.4, 171.2, 175.7

**Untersuchungen zur Wirksamkeit der erfindungsgemäßen Verbindungen:**

**<u>Messung der ORL1-Bindung</u>**

Die Cyclohexan- Derivate der allgemeinen Formel I wurden in einem Rezeptorbindungsassay mit $^3$H- Nociceptin/ Orphanin FQ mit Membranen von rekombinanten CHO- ORL1 Zellen untersucht. Dieses Testsystem wurde gemäß der von Ardati et al. (Mol. Pharmacol., 51, 1997, S. 816- 824) vorgestellten Methode durchgeführt. Die Konzentration von $^3$H- Nociceptin/ Orphanin FQ betrug bei diesen Versuchen 0.5 nM. Die Bindungsassays wurden mit je 20 μg Membranprotein je 200 μl Ansatz in 50 mM Hepes, pH 7, 4, 10 mM $MgCl_2$ und 1 mM EDTA durchgeführt. Die Bindung an den ORL1- Rezeptor wurde unter Verwendung von je 1 mg WGA- SPA Beads (Amersham- Pharmacia, Freiburg), durch einstündige Inkubation des Ansatzes bei RT und anschliessende Messung im Szintillationscounter Trilux (Wallac, Finnland), bestimmt. Die Affinität wird in Tabelle 1 als nanomolarer $K_i$- Wert in oder % Inhibition bei c=1 μM angegeben.

**<u>Messung der μ-Bindung</u>**

Die Rezeptoraffinität zum humanen μ- Opiatrezeptor wurde in einem homogenen Ansatz in Mikrotiterplatten bestimmt. Hierzu wurden Verdünnungsreihen des jeweils zu prüfenden substituierten substituierten Cyclohexyl- 1, 4-diamin- Derivates mit einer Rezeptormembranpräparation (15- 40 μg Protein pro 250 μl Inkubationsansatz) von CHO-K1- Zellen, welche den humanen μ- Opiatrezeptor exprimieren (RB- HOM- RezeptormembranPräparation der Firma NEN, Zaventem, Belgien) in Gegenwart von 1 nmol/l des radioaktiven Liganden [$^3$H]- Naloxon (NET719, Firma NEN, Zaventem, Belgien) sowie von 1 mg WGA- SPA- Beads (Wheat germ agglutinin SPA Beads der Firma Amersham/ Pharmacia, Freiburg, Deutschland) in einem Gesamtvolumen von 250 μl für 90 Minuten bei Raumtemperatur inkubiert. Als Inkubationspuffer wurde 50 mmol/l Tris- HCl supplementiert mit 0, 05 Gew.- % Natriumazid und mit 0, 06 Gew.- % bovinem Serumalbumin verwendet. Zur Bestimmung der unspezifischen Bindung wurde zusätzlich 25 μmol/l Naloxon zugegeben. Nach Beendigung der neunzigminütigen Inkubationszeit wurden die Mikrotiterplatten für 20 Minuten bei 1000 g abzentrifugiert und die Radioaktivität in einem β- Counter (Microbeta- Trilux, Firma PerkinElmer Wallac, Freiburg, Deutschland) vermessen. Es wurde die prozentuale Verdrängung des radioaktiven Liganden aus seiner Bindung zum humanen μ- Opiatrezeptor bei einer Konzentration der Prüfsubstanzen von 1 μmol/l bestimmt und als prozentuale

Hemmung (%Hemmung) der spezifischen Bindung angegeben. Teilweise wurden ausgehend von der prozentualen Verdrängung durch unterschiedliche Konzentrationen der zu prüfenden Verbindungen der allgemeinen Formel I $IC_{50}$ Hemmkonzentrationen berechnet, die eine 50- prozentige Verdrängung des radioaktiven Liganden bewirken. Durch Umrechnung mittels der Cheng- Prusoff- Beziehung wurden Ki- Werte für die Prüfsubstanzen erhalten.

**Analgesieprüfung im Tall-Flick-Test an der Maus**

Die Mäuse wurden jeweils einzeln in einen Testkäfig gesetzt und die Schwanzbasis dem fokussierten Wärmestrahl einer elektrischen Lampe (Tail- flick- Typ 50/08/1.bc, Labtec, Dr. Hess) ausgesetzt. Die Lampenintensität wurde so eingestellt, daß die Zeit vom Einschalten der Lampe bis zum plötzlichen Wegzucken des Schwanzes (Schmerzlatenz) bei unbehandelten Mäusen 3 bis 5 Sekunden betrug. Vor der Applikation der Lösungen enthaltend die erfindungsgemäße Verbindung bzw. der jeweiligen Vergleichslösungen wurden die Mäuse innerhalb von fünf Minuten zweimal vorgetestet und der Mittelwert dieser Messungen als Vortestmittelwert berechnet.

Die Lösungen der erfindungsgemäßen Verbindung der allgemeinen Formel I sowie die Vergleichslösungen wurden dann intravenös appliziert. Die Schmerzmessung wurde jeweils 10, 20, 40 und 60 Minuten nach der intravenösen Applikation durchgeführt. Die analgetische Wirkung wurde als Zunahme der Schmerzlatenz (% des maximal möglichen antinociceptiven Effektes) nach der folgenden Formel bestimmt:

$$[(T_1-T_0)/(T_2-T_0)] \times 100$$

Hierbei ist die Zeit $T_0$ die Latenzzeit vor der Applikation, die Zeit $T_1$ die Latenzzeit nach der Applikation der Wirkstoffkombination und die Zeit $T_2$ die maximale Expositionsdauer (12 Sekunden).

**Analgesieprüfung im Tail-Flick-Test an der Ratte**

Die analgetische Wirksamkeit der Testverbindungen wurde im Brennstrahl (Tail- flick)- Test an der Ratte nach der Methode von D'Amour and Smith (J. Pharm. Exp. Ther. 72, 74 79 (1941) untersucht. Dazu wurden weibliche Sprague Dawley mit einem Gewicht zwischen 134 und 189 g verwendet. Die Tiere wurden einzeln in spezielle Testkäfige gesetzt und die Schwanzbasis einem focussierten Wärmestrahl einer Lampe (Tail- flick Typ 50/08/1.bc, Labtec, Dr. Hess) ausgesetzt. Die Lampenintensität wurde so eingestellt, daß die Zeit vom Einschalten der Lampe bis zum plötzlichen Wegzucken des Schwanzes (Schmerzlatenz) bei unbehandelten Tieren 2, 5- 5 Sekunden betrug. Vor Gabe einer Testverbindung wurden die Tiere innerhalb von 30 Minuten zweimal vorgetestet und der Mittelwert dieser Messungen als Vortestmittelwert berechnet. Die Schmerzmessung wurde 20, 40 und 60 min nach intravenöser Gabe durchgeführt. Die analgetische Wirkung wurde als Zunahme der Schmerzlatenz (% MPE) bestimmt nach folgender Formel:

$$[(T_1 - T_0)/(T_2 - T_0)] \times 100$$

Dabei ist die $T_0$ die Latenzzeit vor und $T_1$ die Latenzzeit nach Substanzapplikation, $T_2$ ist die maximale Expositionszeit (12 sec) .

Zur Bestimmung der Dosisabhängigkeit wurde die jeweilige Testverbindung in 3 - 5 logarithmisch ansteigenden Dosen, die jeweils die Schwellen- und die maximale Wirkdosis einschlossen, appliziert und die $ED_{50}$-Werte mit Hilfe der Regressionsanalyse bestimmt. Die $ED_{50}$-Berechnung erfolgte im Wirkmaximum, 20 Minuten nach intravenöser Substanzgabe.

Die folgende Tabelle bildet die Beispielverbindungen als Basen ohne Angabe, um welches Diastereomer es sich handelt, ab; in den Fällen, in denen die Beispielverbindung als Salz dargestellt wurde oder ein polareres und unpolareres Diasteremer isoliert wurde, kann dies der jeweiligen Synthesebeschreibung entnommen werden.

Die bestimmten Ki-Werte sind in der folgenden Tabelle angegeben. Es wurden nicht für alle Verbindungen Ki-Werte bestimmt. Beispielhaft wurden zwei Verbindungen im Tail-flick-Test untersucht.

| Beispiel | Struktur (ggf ohne Gegenion und Bezeichnung des Diastereomers) | .ORL1-Rezeptor, Ki-Wert [μM] | μ-Opioid-Rezeptor, Ki-Wert [μM] | Tail-flick |
|---|---|---|---|---|
| 1 | 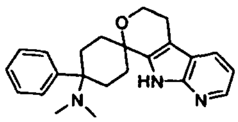 | 0.0147 | 0.0123 | |
| 2 | 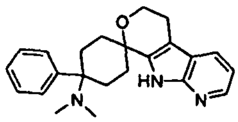 | 0.0053 | 0.0044 | Ratte, i. v. $ED_{50}$: 15 μg/kg |
| 3 | 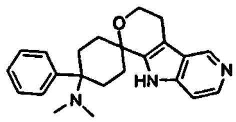 | 0.0197 | 0.0147 | |
| 4 | 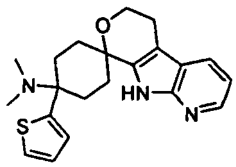 | 0.0060 | 0.0118 | Maus, i. v. 72% MPE bei 464μg/kg |
| 5 | 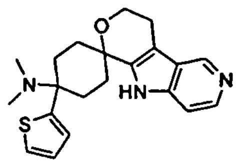 | 0.1800 | 0.3033 | |
| 6 | 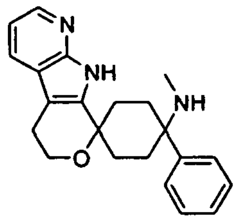 | 0.0237 | 0.0250 | |
| 7 | 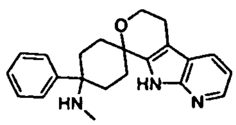 | 2.3950 | 0.6367 | |
| 8 | 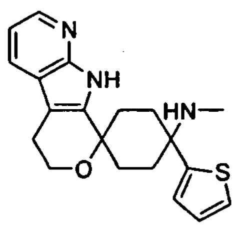 | 0.0657 | 0.0483 | |
| 9 | 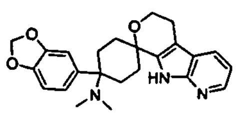 | 0.1533 | 0.0773 | |
| 10 | 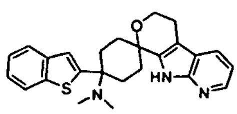 | | 0.0567 | |

(fortgesetzt)

| Beispiel | Struktur (ggf ohne Gegenion und Bezeichnung des Diastereomers) | .ORL1-Rezeptor, Ki-Wert [μM] | μ-Opioid-Rezeptor, Ki-Wert [μM] | Tail-flick |
|---|---|---|---|---|
| 12 | 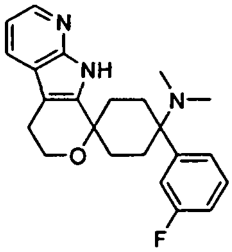 | 0.0683 | 0.0317 | |
| 13 | 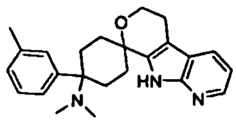 | 0.2800 | 0.0817 | |
| 14 | 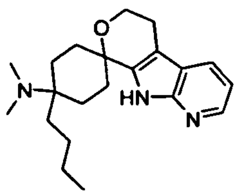 | | | |
| 15 | 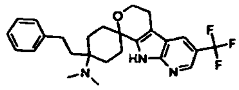 | | 0.0380 | |
| 17 | 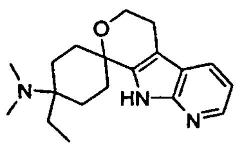 | 0.0870 | 0.0010 | |
| 18 | 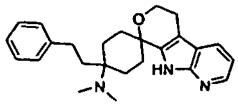 | 0.6633 | 0.0014 | |
| 19 | 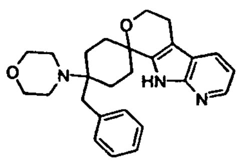 | | 2.9067 | |
| 20 | 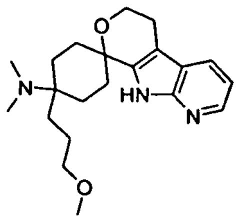 | | 0.0835 | |
| 21 | 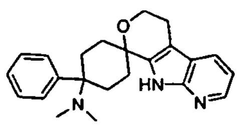 | 0.0280 | 0.0190 | |

(fortgesetzt)

| Beispiel | Struktur (ggf ohne Gegenion und Bezeichnung des Diastereomers) | .ORL1-Rezeptor, Ki-Wert [μM] | μ-Opioid-Rezeptor, Ki-Wert [μM] | Tail-flick |
|---|---|---|---|---|
| 22 | 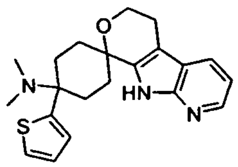 | 0.0167 | 0.0337 | |
| 23 | 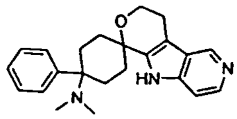 | 0.0023 | 0.0019 | |
| 24 | 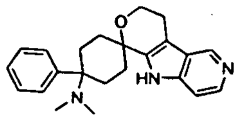 | | | |
| 25 | 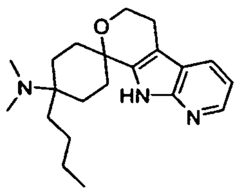 | | 0.0006 | |
| 26 | 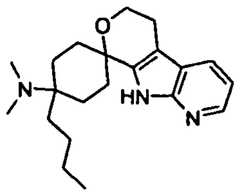 | | 0.0061 | |
| 27 | 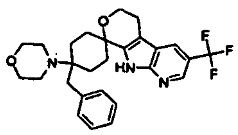 | | | |
| 28 | 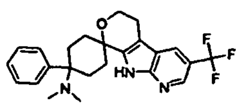 | 2.6367 | 0.6375 | |
| 29 | 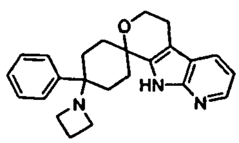 | | | |
| 30 | 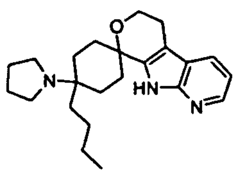 | | | |
| 31 | 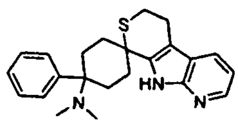 | | | |

(fortgesetzt)

| Beispiel | Struktur (ggf ohne Gegenion und Bezeichnung des Diastereomers) | .ORL1-Rezeptor, Ki-Wert [μM] | μ-Opioid-Rezeptor, Ki-Wert [μM] | Tail-flick |
|---|---|---|---|---|
| 32 | 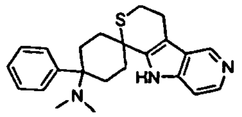 | | | |
| 33 | 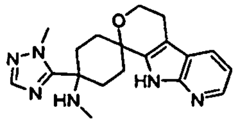 | | | |
| 34 | 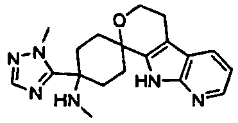 | | | |

**Parenterale Lösung eines erfindungsgemäßen spirocyclischen-Derivats**

3 g eines der erfindungsgemäßen spirocyclischen Azaindol-Derivate, hier Beispiel 1, wird in 1 l Wasser für Injektionszwecke bei Raumtemperatur gelöst und anschließend durch Zugabe von wasserfreier Glukose für Injektionszwecke auf isotone Bedingungen eingestellt.

**Patentansprüche**

**1.** Spirocyclische Azaindol-Derivate der allgemeinen Formel I,

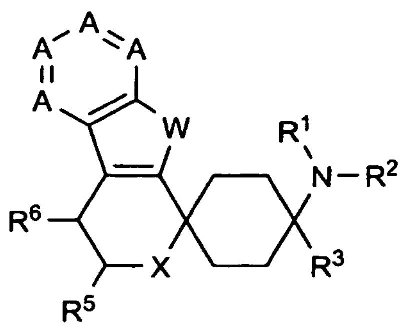

I

worin

A für N oder $CR^{7-10}$ steht, wobei A mindestens einmal und höchstens zweimal für N steht
W für $NR^4$ steht
X für $NR^{17}$, O oder S steht
$R^1$ und $R^2$, unabhängig voneinander für H; $C_{1-5}$- Alkyl jeweils gesättigt oder ungesättigt, verzweigt oder unverzweigt, einfach oder mehrfach substituiert oder unsubstituiert; $C_{38}$- Cycloalkyl, jeweils einfach oder mehrfach substituiert oder unsubstituiert; oder über $C_{1-3}$- Alkyl gebundenes Aryl, $C_{3-8}$- Cycloalkyl oder Heteroaryl, jeweils einfach oder mehrfach substituiert oder unsubstituiert, stehen;
oder die Reste $R^1$ und $R^2$ zusammen für $CH_2CH_2OCH_2CH_2$, $CH_2CH_2NR^{10}CH_2CH_2$ oder $(CH_2)_{3-6}$ stehen,

wobei $R^{10}$ H; $C_{1-5}$- Alkyl, jeweils gesättigt oder ungesättigt, verzweigt oder unverzweigt, einfach oder mehrfach substituiert oder unsubstituiert; $C_{3-8}$- Cycloalkyl, jeweils einfach oder mehrfach substituiert oder unsubstituiert; Aryl-, oder Heteroaryl, jeweils einfach oder mehrfach substituiert oder unsubstituiert; oder über $C_{1-3}$- Alkyl gebundenes Aryl, $C_{3-8}$- Cycloalkyl oder Heteroaryl, jeweils einfach oder mehrfach substituiert oder unsubstituiert; C (O) Phenyl, C (O) Heteroaryl, C (O) $C_{1-5}$- Alkyl, jeweils substituiert oder unsubstituiert, bedeutet;

$R^3$ für $C_{1-8}$- Alkyl, jeweils gesättigt oder ungesättigt, verzweigt oder unverzweigt, einfach oder mehrfach substituiert oder unsubstituiert; $C_{3-8}$- Cycloalkyl, jeweils einfach oder mehrfach substituiert oder unsubstituiert; Aryl oder Heteroaryl, jeweils unsubstituiert oder einfach oder mehrfach substituiert; über $C_{1-3}$- Alkyl- Gruppe gebundenes Aryl, Heteroaryl oder $C_{3-8}$- Cycloalkyl, jeweils unsubstituiert oder einfach oder mehrfach substituiert, steht;
$R^4$ für H; $C_{1-5}$- Alkyl, gesättigt oder ungesättigt, verzweigt oder unverzweigt, unsubstituiert oder einfach oder mehrfach substituiert; Aryl, oder Heteroaryl, jeweils substituiert oder unsubstituiert; über eine $C_{1-3}$- Alkyl- Gruppe gebundenes Aryl, Heteroaryl oder Cycloalkyl, jeweils einfach oder mehrfach substituiert oder unsubstituiert; $COR^{12}$; $SO_2R^{12}$ steht,

wobei $R^{12}$ H; $C_{1-5}$- Alkyl, jeweils gesättigt oder ungesättigt, verzweigt oder unverzweigt, einfach oder mehrfach substituiert oder unsubstituiert; $C_{3-8}$- Cycloalkyl, jeweils gesättigt oder ungesättigt, einfach oder mehrfach substituiert oder unsubstituiert; Aryl-, oder Heteroaryl, jeweils einfach oder mehrfach substituiert oder unsubstituiert; oder über $C_{13}$- Alkyl gebundenes Aryl, $C_{3-8}$- Cycloalkyl oder Heteroaryl, jeweils einfach oder mehrfach substituiert oder unsubstituiert; $OR^{13}$; $NR^{14}R^{15}$ bedeutet;

$R^5$ für =O; H; $COOR^{13}$, $CONR^{13}$, $OR^{13}$; $C_{1-5}$- Alkyl, gesättigt oder ungesättigt, verzweigt oder unverzweigt, unsubstituiert oder einfach oder mehrfach substituiert; $C_{3-8}$- Cycloalkyl, gesättigt oder ungesättigt, unsubstituiert oder einfach oder mehrfach substituiert; Aryl-, oder Heteroaryl, unsubstituiert oder einfach oder mehrfach substituiert; oder über $C_{1-3}$- Alkyl gebundenes Aryl, $C_{3-8}$- Cycloalkyl oder Heteroaryl, unsubstituiert oder einfach oder mehrfach substituiert, steht;
$R^6$ für H; F, Cl, $NO_2$, $CF_3$, $OR^{13}$, $SR^{13}$, $SO_2R^{13}$, $SO_2OR^{13}$, CN, $COOR^{13}$, $NR^{14}R^{15}$; $C_{1-5}$- Alkyl, gesättigt oder ungesättigt, verzweigt oder unverzweigt, unsubstituiert oder einfach oder mehrfach substituiert; $C_{3-8}$- Cycloalkyl, gesättigt oder ungesättigt, unsubstituiert oder einfach oder mehrfach substituiert; Aryl-, oder Heteroaryl, unsubstituiert oder einfach oder mehrfach substituiert; oder über $C_{1-3}$- Alkyl gebundenes Aryl, $C_{3-8}$- Cycloalkyl oder Heteroaryl, unsubstituiert oder einfach oder mehrfach substituiert, steht;
oder $R^5$ und $R^6$ gemeinsam $(CH_2)n$ mit n = 2, 3, 4, 5 oder 6 bedeuten, wobei einzelne Wasserstoffatome auch durch F, Cl, Br, I, $NO_2$, $CF_3$, $OR^{13}$, CN oder $C_{1-5}$-Alkyl ersetzt sein können;
$R^7$, $R^8$, $R^9$ und $R^{10}$ unabhängig voneinander für
H, F, Cl, Br, I, $NO_2$, $CF_3$, $OR^{13}$, $SR^{13}$, $SO_2R^{13}$, $SO_2OR^{13}$, CN, $COOR^{13}$, $NR^{14}R^{15}$; $C_{1-5}$- Alkyl, $C_{38}$- Cycloalkyl, unsubstituiert oder einfach oder mehrfach substituiert; Aryl-, oder Heteroaryl, unsubstituiert oder einfach oder mehrfach substituiert; oder über $C_{1-3}$- Alkyl gebundenes Aryl, $C_{3-8}$- Cycloalkyl oder Heteroaryl, unsubstituiert oder einfach oder mehrfach substituiert, stehen;

wobei $R^{13}$ H; $C_{1-5}$- Alkyl jeweils gesättigt oder ungesättigt, verzweigt oder unverzweigt, unsubstituiert oder einfach oder mehrfach substituiert; $C_{3-8}$- Cycloalkyl, jeweils gesättigt oder ungesättigt, unsubstituiert oder einfach oder mehrfach substituiert; Aryl-, oder Heteroaryl, unsubstituiert oder einfach oder mehrfach substituiert; oder über $C_{1-3}$- Alkyl gebundenes Aryl, $C_{3-8}$- Cycloalkyl oder Heteroaryl, unsubstituiert oder einfach oder mehrfach substituiert, bedeutet;

$R^{14}$ und $R^{15}$ unabhängig voneinander H; $C_{1-5}$- Alkyl , jeweils gesättigt oder ungesättigt, verzweigt oder unverzweigt, unsubstituiert oder einfach oder mehrfach substituiert; oder $C_{3-8}$- Cycloalkyl, jeweils gesättigt oder ungesättigt, unsubstituiert oder einfach oder mehrfach substituiert; Aryl-, oder Heteroaryl, unsubstituiert oder einfach oder mehrfach substituiert; oder über $C_{1-3}$- Alkyl gebundenes Aryl, $C_{3-8}$- Cycloalkyl oder Heteroaryl, unsubstituiert oder einfach oder mehrfach substituiert, bedeuten;
oder $R^{14}$ und $R^{15}$ zusammen $CH_2CH_2OCH_2CH_2$, $CH_2CH_2NR^{16}CH_2CH_2$ oder $(CH_2)_{3-6}$ bilden,

wobei $R^{16}$ H; $C_{1-5}$- Alkyl gesättigt oder ungesättigt, verzweigt oder unverzweigt, unsubstituiert oder einfach oder mehrfach substituiert, bedeutet;

$R^{17}$ für H; $C_{1-8}$- Alkyl, gesättigt oder ungesättigt, verzweigt oder unverzweigt; $COR^{12}$ oder $SO_2R^{12}$ steht

in Form des Razemats; der Enantiomere, Diastereomere, Mischungen der Enantiomere oder Diastereomere

oder eines einzelnen Enantiomers oder Diastereomers; der Basen und/ oder Salze physiologisch verträglicher Säuren.

**2.** Spirocyclische Azaindolderivate gemäß Anspruch 1, wobei die genannten $C_{1-8}$- Alkyle, $C_{1-5}$- Alkyle, $C_{1-3}$- Alkyle bzw. $C_{1-3}$- Alkylene und $C_{3-8}$- Cycloalkylreste jeweils einfach oder mehrfach mit F, Cl, Br, I, CN, $NH_2$, NH- $C_{1-6}$- Alkyl, NH- $C_{1-6}$- Alkyl- OH, N ($C_{1-6}$Alkyl)$_2$, N ($C_{1-6}$- Alkyl- OH)$_2$, $NO_2$, SH, S- $C_{1-6}$- Alkyl, OH, O- $C_{1-6}$- Alkyl, O- $C_{1-6}$Alkyl- OH, C (=O) $C_{1-6}$- Alkyl, $CO_2H$, $CO_2$- $C_{1-6}$- Alkyl, $CF_3$, $OCF_3$, $C_{1-6}$- Alkyl substituiert sein können, die genannten Aryl- oder Heteroaryl- Reste jeweils einfach oder mehrfach mit F, Cl, Br, I, CN, $NH_2$, NH- $C_{1-6}$- Alkyl, NH- $C_{1-6}$- Alkyl- OH, N ($C_{1-6}$Alkyl)$_2$, N ($C_{1-6}$- Alkyl- OH)$_2$, $NO_2$, SH, S- $C_{1-6}$- Alkyl, OH, O- $C_{1-6}$- Alkyl, O- $C_{1-6}$Alkyl- OH, C (=O) $C_{1-6}$- Alkyl, $CO_2H$, $CO_2$- $C_{1-6}$- Alkyl, $CF_3$, $OCF_3$, $C_{1-6}$- Alkyl,

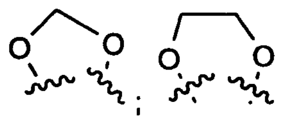

oder Phenoxy substituiert sein können.

**3.** Spirocyclische Azaindol- Derivate gemäß Anspruch 1 oder 2, worin $R^1$ und $R^2$ unabhängig voneinander für H; $C_{1-5}$- Alkyl, gesättigt oder ungesättigt, verzweigt oder unverzweigt, einfach oder mehrfach substituiert oder unsubstituiert, stehen; oder die Reste $R^1$ und $R^2$ zusammen einen Ring bilden und $CH_2CH_2OCH_2CH_2$, $CH_2CH_2NR^{11}CH_2CH_2$ oder $(CH_2)_{3-6}$ bedeuten,

wobei $R^{11}$ H; $C_{1-5}$- Alkyl, gesättigt oder ungesättigt, verzweigt oder unverzweigt, einfach oder mehrfach substituiert oder unsubstituiert, bedeutet.

**4.** Spirocyclische Azaindol-Derivate gemäß Anspruch 3, worin $R^1$ und $R^2$ für $CH_3$ stehen.

**5.** Spirocyclische Azaindol- Derivate gemäß Anspruch 1 oder 2, worin $R^3$ Propyl, Butyl, Pentyl, Hexyl, Phenyl, Furyl, Thiophenyl, Naphthyl, Benzyl, Benzofuranyl, Indolyl, Indanyl, Benzodioxanyl, Benzodioxolanyl, Pyridyl, Pyrimidyl, Pyrazinyl, Triazolyl oder Benzothiophenyl, jeweils unsubstituiert oder einfach oder mehrfach substituiert; über eine gesättigte, unverzweigte $C_{1-3}$- Alkyl- Gruppe gebundenen Phenyl, Furyl oder Thiophenyl, jeweils unsubstituiert oder einfach oder mehrfach substituiert, bedeutet.

**6.** Spirocyclische Azaindol- Derivate gemäß Anspruch 5, worin $R^3$ Butyl, Ethyl, 3- Methoxypropyl, Benzothiophenyl, Phenyl, 3- Methylphenyl, 3- Fluorphenyl, Benzo [1, 3]- dioxolyl, Benzyl, 1- Methyl- 1, 2, 4- triazolyl, Thienyl oder Phenethyl bedeutet.

**7.** Spirocyclische Azaindol- Derivate gemäß Anspruch 1 oder 2, worin $R^5$ für H, $C_{1-5}$- Alkyl, verzweigt oder unverzweigt, unsubstituiert oder einfach oder mehrfach substituiert, oder $COOR^{13}$ steht.

**8.** Spirocyclische Azaindol-Derivate gemäß Anspruch 7, worin $R^5$ H bedeutet.

**9.** Spirocyclische Azaindol- Derivate gemäß Anspruch 1 oder 2, worin $R^6$ für H, $C_{1-5}$- Alkyl, Aryl oder über eine $C_{1-3}$- Alkylgruppe verknüpftes Aryl steht.

**10.** Spirocyclische Azaindol-Derivate gemäß Anspruch 9, worin $R^6$ für H steht.

**11.** Spirocyclische Azaindol-Derivate gemäß Anspruch 1, worin $R^7$, $R^8$, $R^9$ und $R^{10}$ unabhängig voneinander für H; Methyl, Ethyl, Propyl, Butyl; Pyridyl, O-Benzyl, F, Cl, Br, I, $CF_3$, OH, $OCH_3$, $NH_2$, COOH, $COOCH_3$, $NHCH_3$ oder N $(CH_3)_2$ oder $NO_2$ stehen.

**12.** Spirocyclische Azaindol-Derivate gemäß Anspruch 11, worin $R^7$, $R^8$, $R^9$ und $R^{10}$ für H stehen.

**13.** Verbindungen gemäß einem der Ansprüche 1-12, worin die allgemeine Formel I die Bedeutung einer der allgemeinen

Formeln Ia oder Ib annimmt

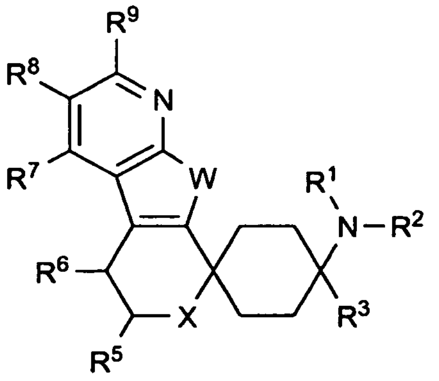

Ia

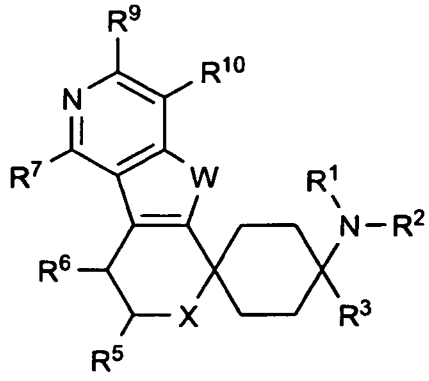

Ib

und die Gruppen X und W sowie die Reste $R^1$-$R^{10}$ die in den Ansprüchen 1-12 angegebenen Bedeutungen haben.

**14.** Spirocyclische Azaindol- Derivate gemäß Anspruch 1 aus der Gruppe

**(1)** 4- (Dimethylamino)- 4- phenyl- spiro [cyclohexan- 1, 8'- (5, 6, 8, 9- Tetrahydro- pyrano [3, 4- b]- 7- aza- indol) ]
**(2)** 4- (Dimethylamino)- 4- phenyl- spiro [cyclohexan- 1, 8'- (5, 6, 8, 9- Tetrahydro- pyrano [3, 4- b]- 7- aza- indol) ] ; Methansulfonat
**(3)** 4- (Dimethylamino)- 4- phenyl- spiro [cyclohexan- 1, 6'- (5, 6, 8, 9- Tetrahydro- pyrano [3, 4- b]- 5- aza- indol) ]
**(4)** 4- (Dimethylamino)- 4- thiophen- 2- yl- spiro [cyclohexan- 1, 8'- (5, 6, 8, 9- Tetrahydro- pyrano [3, 4- b]- 7- aza- indol) ] ; Methansulfonat
**(5)** 4- (Dimethylamino)- 4- thiophen- 2- yl- spiro [cyclohexan- 1, 6'- (5, 6, 8, 9- Tetrahydro- pyrano [3, 4- b]- 5- aza- indol) ]
**(6)** 4- (Dimethylamino)- 4- phenyl- spiro [cyclohexan- 1, 8'- (5, 6, 8, 9- Tetrahydro- pyrano [3, 4- b]- 7- aza- indol) ] ; Citrat (4: 3)
**(7)** 4- (Methylamino)- 4- phenyl- spiro [cyclohexan- 1, 8'- (5, 6, 8, 9- Tetrahydro- pyrano [3, 4- b]- 7- aza- indol) ] ; Citrat (1: 1)
**(8)** 4- (Methylamino)- 4- tiophen- 2- yl- spiro [cyclohexan- 1, 8'- (5, 6, 8, 9- Tetrahydro- pyrano [3, 4- b]- 7- aza- indol) ] ; Citrat (4: 3)
**(9)** 4- (Dimethylamino)- 4- benzo [1, 3- dioxol]- spiro [cyclohexan- 1, 8'- (5, 6, 8, 9- Tetrahydro- pyrano [3, 4- b]- 7- aza- indol) ] ; Citrat (1: 1)
**(10)** 4- (Dimethylamino)- 4- (Benzothiophen- 2- yl)- spiro [cyclohexan- 1, 8'- (5, 6, 8, 9- Tetrahydro- pyrano [3, 4- b]- 7- aza- indol) ] ; Citrat (1: 1)
**(12)** 4- (Dimethylamino)- 4- (3- fluorophenyl)- spiro [cyclohexan- 1, 8'- (5, 6, 8, 9- Tetrahydro- pyrano [3, 4- b]- 7- aza- indol) ] ; Citrat (4: 3)
**(13)** 4- (Dimethylamino)- 4- (3- methylphenyl)- spiro [cyclohexan- 1, 8'- (5, 6, 8, 9- Tetrahydro- pyrano [3, 4- b]- 7- aza- indol) ] ; Citrat (1: 1)
**(14)** 4- (Dimethylamino)- 4- (but- 1- yl)- spiro [cyclohexan- 1, 8'- (5, 6, 8, 9- Tetrahydro- pyrano [3, 4- b]- 7- aza- indol) ], Citrat (1: 1)
**(15)** 4- (Dimethylamino)- 4- Phenylethyl- spiro [cyclohexan- 1, 8'- (3- trifluoromethyl- 5, 6, 8, 9- Tetrahydro- pyrano [3, 4- b]- 7- aza- indol) ] ; Citrat (1: 1)
**(17)** 4- (Dimethylamino)- 4- Ethyl- spiro [cyclohexan- 1, 8'- (5, 6, 8, 9- Tetrahydro- pyrano [3, 4- b]- 7- aza- indol) ] ; Citrat (2: 3)
**(18)** 4- (Dimethylamino)- 4- Phenylethyl- spiro [cyclohexan- 1, 8'- (5, 6, 8, 9- Tetrahydro- pyrano [3, 4- b]- 7- aza- indol) ] ; Citrat (2: 3)
**(19)** 4- Benzyl- 4- morpholino- spiro [cyclohexan- 1, 8'- (5, 6, 8, 9- Tetrahydro- pyrano [3, 4- b]- 7- aza- indol) ] ; Citrat (1: 1)
**(20)** 4- (Dimethylamino)- 4- (3- methoxypropyl)- spiro [cyclohexan- 1, 8'- (5, 6, 8, 9- Tetrahydro- pyrano [3, 4- b]- 7- aza- indol) ] ; Citrat (1: 1)
**(21)** 4- (Dimethylamino)- 4- phenyl- spiro [cyclohexan- 1, 8'- (5, 6, 8, 9- Tetrahydro- pyrano [3, 4- b]- 7- aza-indol) ] ; Citrat (1: 1)
**(22)** 4- (Dimethylamino)- 4- thiophen- 2- yl- spiro [cyclohexan- 1, 8'- (5, 6, 8, 9- Tetrahydro- pyrano [3, 4- b]- 7- aza- indol) ] ; Citrat (1: 1)
**(23)** 4- (Dimethylamino)- 4- phenyl- spiro [cyclohexan- 1, 6'- (5, 6, 8, 9- Tetrahydro- pyrano [3, 4- b]- 5- aza- indol) ], Citrat (1: 1)
**(24)** 4- (Dimethylamino)- 4- phenyl- spiro [cyclohexan- 1, 6'- (5, 6, 8, 9- Tetrahydro- pyrano [3, 4- b]- 5- aza- indol) ], Citrat (1: 1)
**(25)** 4- Butyl- 4- (dimethylamino)- spiro [cyclohexan- 1, 8'- (5, 6, 8, 9- Tetrahydro- pyrano [3, 4- b]- 7- aza- indol) ] ; Citrat (1: 1)
**(26)** 4- Butyl- 4- (dimethylamino)- spiro [cyclohexan- 1, 8'- (5, 6, 8, 9- Tetrahydro- pyrano [3, 4- b]- 7- aza- indol) ] ; Citrat (1: 1)
**(27)** 4- Benzyl- 4- morpholino- spiro [cyclohexan- 1, 8'- (3- trifluoromethyl- 5, 6, 8, 9- Tetrahydro- pyrano [3, 4- b]- 7- aza- indol) ] ; Citrat (1: 1)
**(28)** 4- (Dimethylamino)- 4- phenyl- spiro [cyclohexan- 1, 8'- (3- trifluoromethyl- 5, 6, 8, 9- Tetrahydro- pyrano [3, 4- b]- 7- aza- indol) ] ; Citrat (1: 1)
**(29)** 4- (Azetidin- 1- yl)- 4- phenyl- spiro [cyclohexan- 1, 8'- (5, 6, 8, 9- Tetrahydro- pyrano [3, 4- b]- 7- aza- indol) ], Citrat (1: 1)
**(30)** 4- Butyl- 4- (pyrrolidin- 1- yl)- spiro [cyclohexan- 1, 8'- (5, 6, 8, 9- Tetrahydro- pyrano [3, 4- b]- 7- aza- indol) ] ; Citrat (2: 1)
**(31)** 4- (Dimethylamino)- 4- phenyl- spiro [cyclohexan- 1, 8'- (5, 6, 8, 9- Tetrahydro- thiopyrano [3, 4- b]- 7- aza- indol) ] ; Citrat (1: 1)
**(32)** 4- (Dimethylamino)- 4- phenyl- spiro [cyclohexan- 1, 6'- (5, 6, 8, 9- Tetrahydro- thiopyrano [3, 4- b]- 5- aza- indol) ], Citrat (1: 1)
**(33)** 4- (1- Methyl- 1H- 1, 2, 4- triazol- 5- yl)- 4- (methylamino)- spiro [cyclohexan- 1, 8'- (5, 6, 8, 9- Tetrahydro- pyrano [3, 4- b]- 7- aza- indol) ], Citrat (1: 1)
**(34)** 4- (1- Methyl- 1H- 1, 2, 4- triazol- 5- yl)- 4- (methylamino)- spiro [cyclohexan- 1, 8'- (5, 6, 8, 9- Tetrahydro- pyrano [3, 4- b]- 7- aza- indol) ], Citrat (1: 1)

in Form des Razemats; der Enantiomere, Diastereomere, Mischungen der Enantiomere oder Diastereomere oder eines einzelnen Enantiomers oder Diastereomers; der Basen und/ oder Salze physiologisch verträglicher Säuren.

**15.** Verfahren zur Herstellung von spirocyclischen Azaindol-Derivaten der allgemeinen Formel I, indem Verbindungen der allgemeinen Formel III in einem Reaktionsmedium in Gegenwart von Fluorid oder in Gegenwart einer organischen oder anorganischen Säure, vorzugsweise HCl, Essigsäure, Trifluoressigsäure, Bortrifluorid bei Temperaturen von vorzugsweise -70°C bis 300 °C, zu Verbindungen der allgemeinen Formel IV umgesetzt werden,

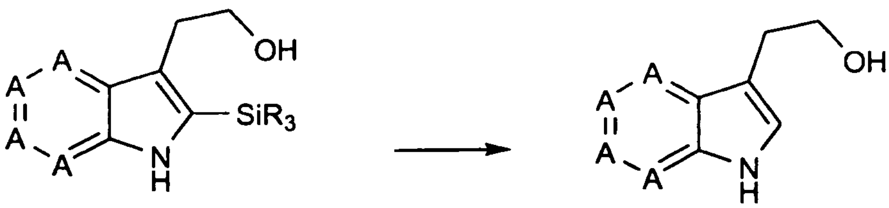

III IV

die anschließend unter Zusatz wenigstens einer organischen Säure oder deren Trimethylsilylester oder einer anorganischen Säure oder unter Zusatz wenigstens eines Übergangsmetallsalzes mit Ketonen der allgemeinen Formel VII,

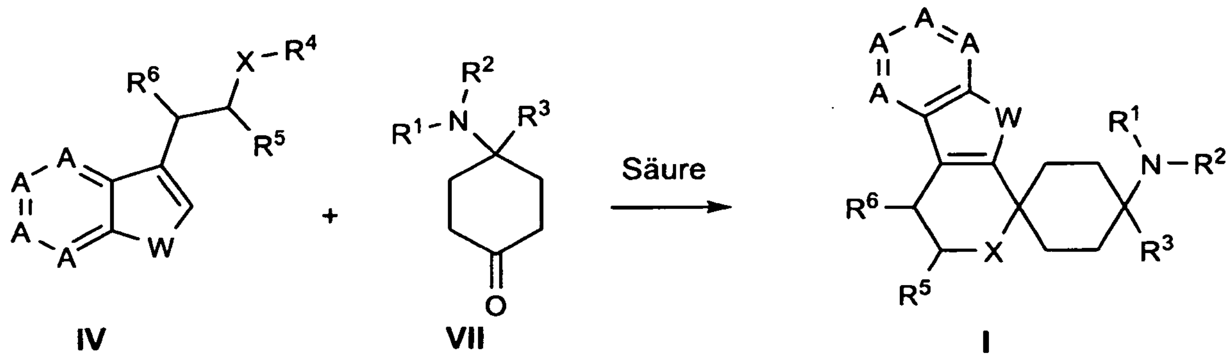


in einem geeigneten Lösungsmittel oder Lösungsmittelgemisch wie beispielsweise, Dichlormethan, Dichlorethan, Chloroform, Acetonitril, Diethylether oder Nitroethan, bei Temperaturen von 0 bis 150 °C ggf. unter Verwendung von Mikrowellen umgesetzt wird.

**16.** Arzneimittel enthaltend wenigstens ein substituiertes Azaindolderivat gemäß Anspruch 1, gegebenenfalls in Form seines Razemats, der reinen Stereoisomeren, insbesondere Enantiomeren und Diastereomeren, in einem beliebigen Mischungsverhältnis; in Form seiner Säuren oder seiner Basen oder in Form seiner Salze, insbesondere der physiologisch verträglichen Salze oder Salze physiologisch verträglicher Säuren oder Kationen; oder in Form seiner Solvate, insbesondere der Hydrate, sowie gegebenenfalls enthaltend geeignete Zusatz- und/oder Hilfsstoffe und/ oder gegebenenfalls weiterer Wirkstoffe.

**17.** Verwendung eines substituierten Azaindolderivat gemäß Anspruch 1, gegebenenfalls in Form seines Razemats, der reinen Stereoisomeren, insbesondere Enantiomeren und Diastereomeren, in einem beliebigen Mischungsverhältnis; in Form seiner Säuren oder seiner Basen oder in Form seiner Salze, insbesondere der physiologisch verträglichen Salze oder Salze physiologisch verträglicher Säuren oder Kationen; oder in Form seiner Solvate, insbesondere der Hydrate; zur Herstellung eines Arzneimittels zur Behandlung von Schmerz, insbesondere von akutem, neuropathischem oder chronischem Schmerz.

**18.** Verwendung eines substituierten Azaindolderivats gemäß Anspruch 1 zur Herstellung eines Arzneimittels zur Behandlung von Angstzuständen, von Stress und mit Stress verbundenen Syndromen, Depressionen, Epilepsie, Alzheimer Erkrankung, seniler Demenz, Katalepsie, allgemeinen kognitiven Dysfunktionen, Lern- und Gedächtnis-Störungen (als Nootropikum), Entzugserscheinungen, Alkohol- und/oder Drogen- und/oder Medikamentenmißbrauch und/oder -abhängigkeit, sexuellen Dysfunktionen, cardiovaskulären Erkrankungen, Hypotension, Hypertension, Tinitus, Pruritus, Migräne, Schwerhörigkeit, mangelnder Darmmotilität, gestörter Nahrungsaufnahme, Anorexie, Fettsucht, lokomotorischen Störungen, Diarrhoe, Kachexie, Harninkontinenz bzw. als Muskelrelaxanz, Antikonvulsivum oder Anesthetikum bzw. zur Coadministration bei Behandlung mit einem opioiden Analgetikum oder mit einem Anesthetikum, zur Diurese oder Antinatriurese, Anxiolyse, zur Behandlung von mit der Modulation der Neurotransmitter-Ausschüttung verbundenen neurodegenerativen Erkrankungen, zur Behandlung von Entzugserscheinungen und/oder zur Reduzierung des Suchtpotentials von Opioiden.

## Claims

**1.** Spirocyclic azaindole derivatives of the general Formula I,

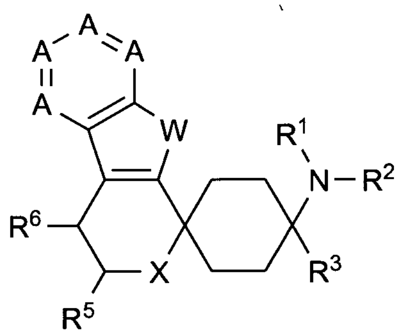

I

wherein

A denotes N or $CR^{7-10}$, wherein at least once and at most twice A denotes N
W denotes $NR^4$
X denotes $NR^{17}$, O or S
$R^1$ and $R^2$ independently of one another denote H; $C_{1-5}$- alkyl, in each case saturated or unsaturated, branched or unbranched, monosubstituted or polysubstituted or unsubstituted; $C_{3-8}$- cycloalkyl, in each case monosubstituted or polysubstituted or unsubstituted; or denote aryl, $C_{3-8}$- cycloalkyl or heteroaryl bonded via $C_{1-3}$- alkyl, in each case monosubstituted or polysubstituted or unsubstituted;
or the radicals $R^1$ and $R^2$ together denote $CH_2CH_2OCH_2CH_2$, $CH_2CH_2NR^{10}CH_2CH_2$ or $(CH_2)_{3-6}$,

wherein $R^{10}$ denotes H; $C_{1-5}$- alkyl, in each case saturated or unsaturated, branched or unbranched, monosubstituted or polysubstituted or unsubstituted; $C_{3-8}$- cycloalkyl, in each case monosubstituted or polysubstituted or unsubstituted; aryl or heteroaryl, in each case monosubstituted or polysubstituted or unsubstituted; or aryl, $C_{3-8}$- cycloalkyl or heteroaryl bonded via $C_{1-3}$- alkyl, in each case monosubstituted or polysubstituted or unsubstituted; C (O) phenyl, C (O) heteroaryl, C (O) $C_{1-5}$- alkyl, in each case substituted or unsubstituted;

$R^3$ denotes $C_{1-8}$- alkyl, in each case saturated or unsaturated, branched or unbranched, monosubstituted or polysubstituted or unsubstituted; $C_{3-8}$- cycloalkyl, in each case monosubstituted or polysubstituted or unsubstituted; aryl or heteroaryl, in each case unsubstituted or monosubstituted or polysubstituted; aryl, heteroaryl or $C_{3-8}$- cycloalkyl bonded via a $C_{1-3}$- alkyl group, in each case unsubstituted or monosubstituted or polysubstituted;
$R^4$ denotes H; $C_{1-5}$- alkyl, saturated or unsaturated, branched or unbranched, unsubstituted or monosubstituted or polysubstituted; aryl or heteroaryl, in each case substituted or unsubstituted; aryl, heteroaryl or cycloalkyl bonded via a $C_{1-3}$- alkyl group, in each case monosubstituted or polysubstituted or unsubstituted; $COR^{12}$; $SO_2R^{12}$,

wherein $R^{12}$ denotes H; $C_{1-5}$- alkyl, in each case saturated or unsaturated, branched or unbranched, monosubstituted or polysubstituted or unsubstituted; $C_{3-8}$- cycloalkyl, in each case saturated or unsaturated, monosubstituted or polysubstituted or unsubstituted; aryl or heteroaryl, in each case monosubstituted or polysubstituted or unsubstituted; or aryl, $C_{3-8}$- cycloalkyl or heteroaryl bonded via $C_{1-3}$- alkyl, in each case monosubstituted or polysubstituted or unsubstituted; $OR^{13}$; $NR^{14}R^{15}$;

$R^5$ denotes =O H; $COOR^{13}$, $CONR^{13}$, $OR^{13}$; $C_{1-5}$- alkyl, saturated or unsaturated, branched or unbranched, unsubstituted or monosubstituted or polysubstituted; $C_{3-8}$- cycloalkyl, saturated or unsaturated, unsubstituted or monosubstituted or polysubstituted; aryl or heteroaryl, unsubstituted or monosubstituted or polysubstituted; or aryl, $C_{3-8}$- cycloalkyl or heteroaryl bonded via $C_{1-3}$- alkyl, unsubstituted or monosubstituted or polysubstituted;
$R^6$ denotes H; F, Cl, $NO_2$, $CF_3$, $OR^{13}$, $SR^{13}$, $SO_2R^{13}$, $SO_2OR^{13}$, CN, $COOR^{13}$, $NR^{14}R^{15}$; $C_{1-5}$- alkyl, saturated or unsaturated, branched or unbranched, unsubstituted or monosubstituted or polysubstituted; $C_{3-8}$- cycloalkyl, saturated or unsaturated, unsubstituted or monosubstituted or polysubstituted; aryl or heteroaryl, unsubstituted or monosubstituted or polysubstituted; or aryl, $C_{3-8}$- cycloalkyl or heteroaryl bonded via $C_{1-3}$- alkyl, unsubstituted or monosubstituted or polysubstituted;
or $R^5$ and $R^6$ together denote $(CH_2)$ n where n = 2, 3, 4, 5 or 6, wherein individual hydrogen atoms can also be

replaced by F, Cl, Br, I, $NO_2$, $CF_3$, $OR^{13}$, CN or $C_{1-5}$- alkyl;
$R^7$, $R^8$, $R^9$ and $R^{10}$ independently of one another denote
H, F, Cl, Br, I, $NO_2$, $CF_3$, $OR^{13}$, $SR^{13}$, $SO_2R^{13}$, $SO_2OR^{13}$, CN, $COOR^{13}$, $NR^{14}R^{15}$; $C_{1-5}$- alkyl, $C_{3-8}$- cycloalkyl, unsubstituted or monosubstituted or polysubstituted; aryl or heteroaryl, unsubstituted or monosubstituted or polysubstituted; or aryl, $C_{3-8}$- cycloalkyl or heteroaryl bonded via $C_{1-3}$- alkyl, unsubstituted or monosubstituted or polysubstituted;

wherein $R^{13}$ denotes H; $C_{1-5}$- alkyl, in each case saturated or unsaturated, branched or unbranched, unsubstituted or monosubstituted or polysubstituted; $C_{3-8}$- cycloalkyl, in each case saturated or unsaturated, unsubstituted or monosubstituted or polysubstituted; aryl or heteroaryl, unsubstituted or monosubstituted or polysubstituted; or aryl, $C_{3-8}$- cycloalkyl or heteroaryl bonded via $C_{1-3}$- alkyl, unsubstituted or monosubstituted or polysubstituted;

$R^{14}$ and $R^{15}$ independently of one another denote H; $C_{1-5}$- alkyl, in each case saturated or unsaturated, branched or unbranched, unsubstituted or monosubstituted or polysubstituted; or $C_{3-8}$- cycloalkyl, in each case saturated or unsaturated, unsubstituted or monosubstituted or polysubstituted; aryl or heteroaryl, unsubstituted or monosubstituted or polysubstituted; or aryl, $C_{3-8}$- cycloalkyl or heteroaryl bonded via $C_{1-3}$- alkyl, unsubstituted or monosubstituted or polysubstituted;
or $R^{14}$ and $R^{15}$ together denote $CH_2CH_2OCH_2CH_2$, $CH_2CH_2NR^{16}CH_2CH_2$ or $(CH_2)_{3-6}$,

wherein $R^{16}$ denotes H; $C_{1-5}$- alkyl, saturated or unsaturated, branched or unbranched, unsubstituted or monosubstituted or polysubstituted;

$R^{17}$ denotes H; $C_{1-8}$- alkyl, saturated or unsaturated, branched or unbranched; $COR^{12}$ or $SO_2R^{12}$

in the form of the racemate; in the form of the enantiomers, diastereomers, mixtures of the enantiomers or diastereomers, or of an individual enantiomer or diastereomer; in the form of the bases and/or salts of physiologically compatible acids.

**2.** Spirocyclic azaindole derivatives according to claim 1,
wherein
the aforementioned $C_{1-8}$- alkyls, $C_{1-5}$- alkyls, $C_{1-3}$- alkyls and $C_{1-3}$- alkylenes and $C_{3-8}$- cycloalkyl radicals can in each case be monosubstituted or polysubstituted with F, Cl, Br, I, CN, $NH_2$, NH- $C_{1-6}$- alkyl, NH- $C_{1-6}$- alkyl- OH, N $(C_{1-6}alkyl)_2$, N $(C_{1-6}$- alkyl- $OH)_2$, $NO_2$, SH, S- $C_{1-6}$- alkyl, OH, O- $C_{1-6}$- alkyl, O- $C_{1-6}$alkyl- OH, C (=O) $C_{1-6}$- alkyl, $CO_2H$, $CO_2$- $C_{1-6}$- alkyl, $CF_3$, $OCF_3$, $C_{1-6}$- alkyl,
the aforementioned aryl or heteroaryl radicals can in each case be monosubstituted or polysubstituted with F, Cl, Br, I, CN, $NH_2$, NH- $C_{1-6}$- alkyl, NH- $C_{1-6}$- alkyl- OH, N $(C_{1-6}alkyl)_2$, N $(C_{1-6}$- alkyl- $OH)_2$, $NO_2$, SH, S- $C_{1-6}$- alkyl, OH, O- $C_{1-6}$- alkyl, O- $C_{1-6}$alkyl- OH, C (=O) $C_{1-6}$- alkyl, $CO_2H$, $CO_2$- $C_{1-6}$- alkyl, $CF_3$, $OCF_3$, $C_{1-6}$- alkyl,

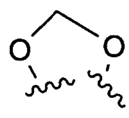 ; 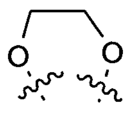

or phenoxy.

**3.** Spirocyclic azaindole derivatives according to claim 1 or 2, wherein $R^1$ and $R^2$ independently of one another denote H; $C_{1-5}$- alkyl, saturated or unsaturated, branched or unbranched, monosubstituted or polysubstituted or unsubstituted; or the radicals $R^1$ and $R^2$ together form a ring and denote $CH_2CH_2OCH_2CH_2$, $CH_2CH_2NR^{11}CH_2CH_2$ or $(CH_2)_{3-6}$,

wherein $R^{11}$ denotes H; $C_{1-5}$- alkyl, saturated or unsaturated, branched or unbranched, monosubstituted or polysubstituted or unsubstituted.

**4.** Spirocyclic azaindole derivatives according to claim 3, wherein $R^1$ and $R^2$ denote $CH_3$.

**5.** Spirocyclic azaindole derivatives according to claim 1 or 2, wherein $R^3$ denotes propyl, butyl, pentyl, hexyl, phenyl,

furyl, thiophenyl, naphthyl, benzyl, benzofuranyl, indolyl, indanyl, benzodioxanyl, benzodioxolanyl, pyridyl, pyrimidyl, pyrazinyl, triazolyl or benzothiophenyl, in each case unsubstituted or monosubstituted or polysubstituted; phenyl, furyl or thiophenyl bonded via a saturated, unbranched $C_{1-3}$-alkyl group, in each case unsubstituted or monosubstituted or polysubstituted.

**6.** Spirocyclic azaindole derivatives according to claim 5, wherein $R^3$ denotes butyl, ethyl, 3- methoxypropyl, benzothiophenyl, phenyl, 3- methylphenyl, 3- fluorophenyl, benzo [1, 3]- dioxolyl, benzyl, 1- methyl- 1, 2, 4- triazolyl, thienyl or phenethyl.

**7.** Spirocyclic azaindole derivatives according to claim 1 or 2, wherein $R^5$ denotes H, $C_{1-5}$- alkyl, branched or unbranched, unsubstituted or monosubstituted or polysubstituted, or denotes $COOR^{13}$.

**8.** Spirocyclic azaindole derivatives according to claim 7, wherein $R^5$ denotes H.

**9.** Spirocyclic azaindole derivatives according to claim 1 or 2, wherein $R^6$ denotes H, $C_{1-5}$- alkyl, aryl, or aryl coupled via a $C_{1-3}$- alkyl group.

**10.** Spirocyclic azaindole derivatives according to claim 9, wherein $R^6$ denotes H.

**11.** Spirocyclic azaindole derivatives according to claim 1, wherein $R^7$, $R^8$, $R^9$ and $R^{10}$ independently of one another denote H; methyl, ethyl, propyl, butyl; pyridyl, O-benzyl, F, Cl, Br, I, $CF_3$, OH, $OCH_3$, $NH_2$, COOH, $COOCH_3$, $NHCH_3$ or $N(CH_3)_2$ or $NO_2$.

**12.** Spirocyclic azaindole derivatives according to claim 11, wherein $R^7$, $R^8$, $R^9$ and $R^{10}$ denote H.

**13.** Compounds according to one of claims 1-12, wherein the general Formula I adopts the meaning of one of the general Formulae Ia or Ib

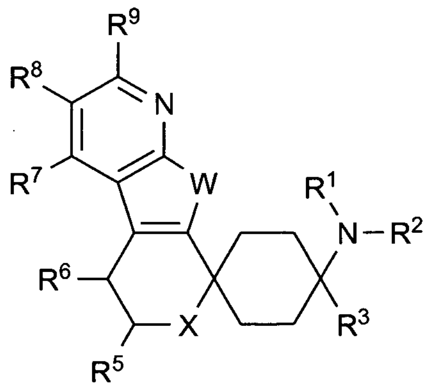

**Ia**

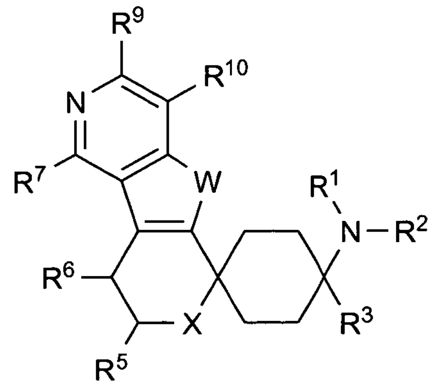

**Ib**

and the groups X and W as well as the radicals $R^1$-$R^{10}$ have the meanings given in claims 1-12.

**14.** Spirocyclic azaindole derivatives according to claim 1 from the following group

**(1)** 4- (dimethylamino)- 4- phenyl- spiro [cyclohexane- 1, 8'- (5, 6, 8, 9- tetrahydropyrano [3, 4- b]- 7- azaindole) ]
**(2)** 4- (dimethylamino)- 4- phenyl- spiro [cyclohexane- 1, 8'- (5, 6, 8, 9- tetrahydropyrano [3, 4- b]- 7- azaindole) ] ; methansulfonate
**(3)** 4- (dimethylamino)- 4- phenyl- spiro [cyclohexane- 1, 6'- (5, 6, 8, 9- tetrahydropyrano [3, 4- b]- 5- azaindole) ]
**(4)** 4- (dimethylamino)- 4- thiophen- 2- yl- spiro [cyclohexane- 1, 8'- (5, 6, 8, 9- tetrahydropyrano [3, 4- b]- 7- azaindole) ] ; methanesulfonate
**(5)** 4- (dimethylamino)- 4- thiophen- 2- yl- spiro [cyclohexane- 1, 6'- (5, 6, 8, 9- tetrahydropyrano [3, 4- b]- 5- azaindole) ]
**(6)** 4- (dimethylamino)- 4- phenyl- spiro [cyclohexane- 1, 8'- (5, 6, 8, 9- tetrahydropyrano [3, 4- b]- 7- azaindole)

] ; citrate (4: 3)
**(7)** 4- (methylamino)- 4- phenyl- spiro [cyclohexane- 1, 8'- (5, 6, 8, 9- tetrahydropyrano [3, 4- b]- 7- azaindole) ] ; citrate (1: 1)
**(8)** 4- (methylamino)- 4- thiophen- 2- yl- spiro [cyclohexane- 1, 8'- (5, 6, 8, 9- tetrahydropyrano [3, 4- b]- 7- azaindole) ] ; citrate (4: 3)
**(9)** 4- (dimethylamino)- 4- benzo [1, 3- dioxol]- spiro [cyclohexane- 1, 8'- (5, 6, 8, 9- tetrahydropyrano [3, 4- b]- 7- azaindole) ] ; citrate (1: 1)
**(10)** 4- (dimethylamino)- 4- (benzothiophen- 2- yl)- spiro [cyclohexane- 1, 8'- (5, 6, 8, 9- tetrahydropyrano [3, 4- b]- 7- azaindole) ] ; citrate (1: 1)
**(12)** 4- (dimethylamino)- 4- (3- fluorophenyl)- spiro [cyclohexane- 1, 8'- (5, 6, 8, 9- tetrahydropyrano [3, 4- b]- 7- azaindole) ] ; citrate (4: 3)
**(13)** 4- (dimethylamino)- 4- (3- methylphenyl)- spiro [cyclohexane- 1, 8'- (5, 6, 8, 9- tetrahydropyrano [3, 4- b]- 7- azaindole) ] ; citrate (1: 1)
**(14)** 4- (dimethylamino)- 4- (but- 1- yl)- spiro [cyclohexane- 1, 8'- (5, 6, 8, 9- tetrahydropyrano [3, 4- b]- 7- azaindole) ], citrate (1: 1)
**(15)** 4- (dimethylamino)- 4- phenylethyl- spiro [cyclohexane- 1, 8'- (3- trifluoromethyl- 5, 6, 8, 9- tetrahydropyrano [3, 4- b]- 7- azaindole) ] ; citrate (1: 1)
**(17)** 4- (dimethylamino)- 4- ethyl- spiro [cyclohexane- 1, 8'- (5, 6, 8, 9- tetrahydropyrano [3, 4- b]- 7- azaindole) ] ; citrate (2: 3)
**(18)** 4- (dimethylamino)- 4- phenylethyl- spiro [cyclohexane- 1, 8'- (5, 6, 8, 9- tetrahydropyrano [3, 4- b]- 7- azaindole) ] ; citrate (2: 3)
**(19)** 4- benzyl- 4- morpholino- spiro [cyclohexane- 1, 8'- (5, 6, 8, 9- tetrahydropyrano [3, 4- b]- 7- azaindole) ] ; citrate (1: 1)
**(20)** 4- (dimethylamino)- 4- (3- methoxypropyl)- spiro [cyclohexane- 1, 8'- (5, 6, 8, 9- tetrahydropyrano [3, 4- b]- 7- azaindole) ] ; citrate (1: 1)
**(21)** 4- (dimethylamino)- 4- phenyl- spiro [cyclohexane- 1, 8'- (5, 6, 8, 9- tetrahydropyrano [3, 4- b]- 7- azaindole) ] ; citrate (1: 1)
**(22)** 4- (dimethylamino)- 4- thiophen- 2- yl- spiro [cyclohexane- 1, 8'- (5, 6, 8, 9- tetrahydropyrano [3, 4- b]- 7- azaindole) ] ; citrate (1: 1)
**(23)** 4- (dimethylamino)- 4- phenyl- spiro [cyclohexane- 1, 6'- (5, 6, 8, 9- tetrahydropyrano [3, 4- b]- 5- azaindole) ], citrate (1: 1)
**(24)** 4- (dimethylamino)- 4- phenyl- spiro [cyclohexane- 1, 6'- (5, 6, 8, 9- tetrahydropyrano [3, 4- b]- 5- azaindole) ], citrate (1: 1)
**(25)** 4- butyl- 4- (dimethylamino)- spiro [cyclohexane- 1, 8'- (5, 6, 8, 9- tetrahydropyrano [3, 4- b]- 7- azaindole) ] ; citrate (1: 1)
**(26)** 4- butyl- 4- (dimethylamino)- spiro [cyclohexane- 1, 8'- (5, 6, 8, 9- tetrahydropyrano [3, 4- b]- 7- azaindole) ] ; citrate (1: 1)
**(27)** 4- benzyl- 4- morpholino- spiro [cyclohexane- 1, 8'- (3- trifluoromethyl- 5, 6, 8, 9- tetrahydropyrano [3, 4- b]- 7- azaindole) ] ; citrate (1: 1)
**(28)** 4- (dimethylamino)- 4- phenyl- spiro [cyclohexane- 1, 8'- (3- trifluoromethyl- 5, 6, 8, 9- tetrahydropyrano [3, 4- b]- 7- azaindole) ] ; citrate (1: 1)
**(29)** 4- (azetidin- 1- yl)- 4- phenyl- spiro [cyclohexane- 1, 8'- (5, 6, 8, 9- tetrahydropyrano [3, 4- b]- 7- azaindole) ], Citrate (1: 1)
**(30)** 4- butyl- 4- (pyrrolidin- 1- yl)- spiro [cyclohexane- 1, 8'- (5, 6, 8, 9- tetrahydropyrano [3, 4- b]- 7- azaindole) ] ; citrate (2: 1)
**(31)** 4- (dimethylamino)- 4- phenyl- spiro [cyclohexane- 1, 8'- (5, 6, 8, 9- tetrahydro- thiopyrano [3, 4- b]- 7- azaindole) ] ; citrate (1: 1)
**(32)** 4- (dimethylamino)- 4- phenyl- spiro [cyclohexane- 1, 6'- (5, 6, 8, 9- tetrahydro- thiopyrano [3, 4- b]- 5- azaindole) ], citrate (1: 1)
**(33)** 4- (1- methyl- 1H- 1, 2, 4- triazol- 5- yl)- 4- (methylamino)- spiro [cyclohexane- 1, 8'- (5, 6, 8, 9- tetrahydropyrano [3, 4- b]- 7- azaindole) ], citrate (1: 1)
**(34)** 4- (1- methyl- 1H- 1, 2, 4- triazol- 5- yl)- 4- (methylamino)- spiro [cyclohexane- 1, 8'- (5, 6, 8, 9- tetrahydropyrano [3, 4- b]- 7- azaindole) ], citrate (1: 1)

in the form of the racemate; in the form of the enantiomers, diastereomers, mixtures of the enantiomers or diastereomers, or of an individual enantiomer or diastereomer; in the form of the bases and/or salts of physiologically compatible acids.

**15.** Process for the preparation of spirocyclic azaindole derivatives of the general Formula I, wherein compounds of the general Formula III are reacted in a reaction medium in the presence of fluoride or in the presence of an organic or inorganic acid, preferably HCl, acetic acid, trifluoracetic acid, boron trifluoride, at temperatures of preferably -70°C to 300°C, to form compounds of the general Formula IV,

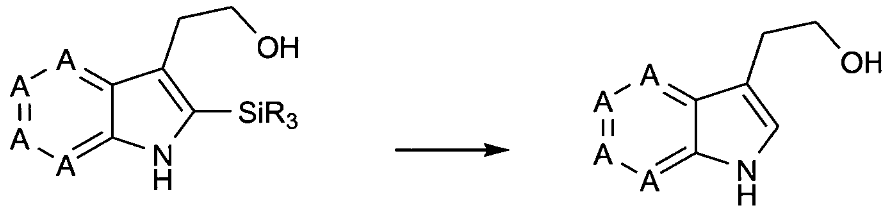

**III** **IV**

which are then reacted with the addition of at least one organic acid or its trimethylsilyl ester or an inorganic acid or with the addition of at least one transition metal salt, with ketones of the general Formula **VII**,

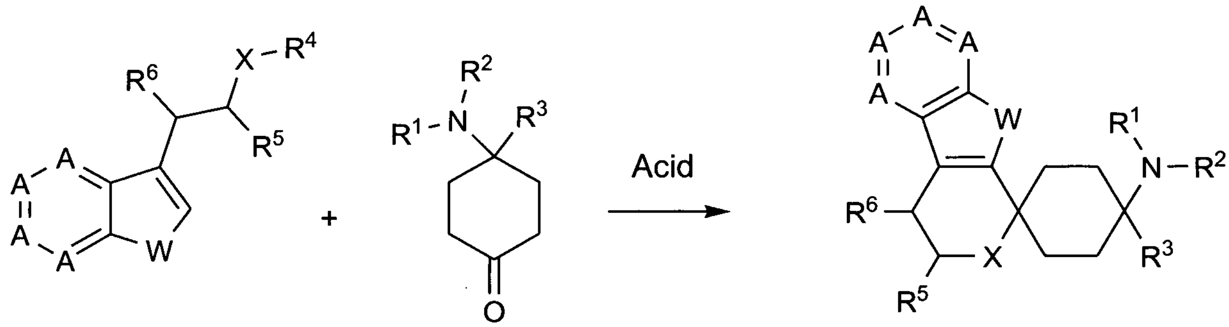


**IV** **VII** **I**

in a suitable solvent or solvent mixture, such as for example dichloromethane, dichloroethane, chloroform, acetonitrile, diethyl ether or nitroethane, at temperatures from 0° to 150°C, optionally with the use of microwaves.

**16.** Medicament containing at least one substituted azaindole derivative according to claim 1, optionally in the form of its racemate, in the form of the pure stereoisomers, in particular enantiomers and diastereomers, in an arbitrary mixture ratio; in the form of its acids or its bases or in the form of its salts, in particular the physiologically compatible salts or salts of physiologically compatible acids or cations; or in the form of its solvates, in particular the hydrates, and also optionally containing suitable additives and/or auxiliary substances and/or optionally further active substances.

**17.** Use of a substituted azaindole derivative according to claim 1, optionally in the form of its racemate, in the form of the pure stereoisomers, in particular enantiomers and diastereomers, in an arbitrary mixture ratio; in the form of its acids or its bases or in the form of its salts, in particular the physiologically compatible salts or salts of physiologically compatible acids or cations; or in the form of its solvates, in particular the hydrates, for the production of a medicament for the treatment of pain, in particular acute, neuropathic or chronic pain.

**18.** Use of a substituted azaindole derivative according to claim 1 for the production of a medicament for the treatment of anxiety states, stress and syndromes associated with stress, depression, epilepsy, Alzheimer's disease, senile dementia, catalepsy, general cognitive dysfunctions, learning and memory disorders (as a nootropic), withdrawal symptoms, alcohol and/or drug and/or medicament misuse and/or dependence, sexual dysfunctions, cardiovascular diseases, hypotension, hypertension, tinnitus, pruritus, migraine, impaired hearing, inadequate intestinal motility, eating disorders, anorexia, obesity, locomotor disorders, diarrhoea, cachexia, urinary incontinence, or as a muscle relaxant, anti-convulsant or anaesthetic, or for co-administration in treatment with an opioid analgesic or with an anaesthetic, for diuresis or anti-natriuresis, anxiolysis, for the treatment of neurodegenerative diseases associated with the modulation of neurotransmitter release, and for the treatment of withdrawal symptoms and/or for reducing the addiction potential of opioids.

## Revendications

1. Dérivés d'azaindole spirocycliques de formule générale I,

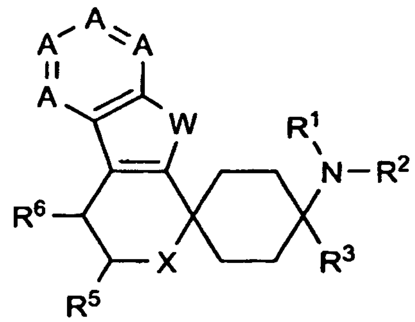

I

dans laquelle

A représente N ou $CR^{7-10}$, A représentant au moins une fois et au maximum deux fois N
W représente $NR^4$
X représente $NR^{17}$ O ou S
$R^1$ et $R^2$ représentent, indépendamment l'un de l'autre, H ; un groupe alkyle en $C_1$-$C_5$ dans chaque cas saturé ou insaturé, ramifié ou non ramifié, non substitué ou une ou plusieurs fois substitué ; un groupe cycloalkyle en $C_3$-$C_8$ chaque fois non substitué ou une ou plusieurs fois substitué ; ou un groupe aryle, cycloalkyle en $C_3$-$C_8$ ou hétéroaryle, chaque fois non substitué ou une ou plusieurs fois substitué, relié par l'intermédiaire d'un groupe alkyle en $C_1$-$C_3$ ;
ou les radicaux $R^1$ et $R^2$ représentent ensemble un groupe $CH_2CH_2OCH_2CH_2$, $CH_2CH_2NR^{10}CH_2CH_2$ ou $(CH_2)_{3-6}$,

$R^{10}$ représentant H ; un groupe alkyle en $C_1$- $C_5$ respectivement saturé ou insaturé, ramifié ou non ramifié, non substitué ou une ou plusieurs fois substitué ; un groupe cycloalkyle en $C_3$- $C_8$ respectivement non substitué ou une ou plusieurs fois substitué ; un groupe aryle ou hétéroaryle, chacun non substitué ou une ou plusieurs fois substitué ; ou un groupe aryle, cycloalkyle en $C_3$- $C_8$ ou hétéroaryle, chacun non substitué ou une ou plusieurs fois substitué, relié par l'intermédiaire d'un groupe alkyle en $C_1$- $C_3$ ; un groupe C (O) phényle, C (O) hétéroaryle, C (O)- alkyle ($C_1$- $C_5$), chacun substitué ou non substitué ;

$R^3$ représente un groupe alkyle en $C_1$-$C_8$ respectivement saturé ou insaturé, ramifié ou non ramifié, non substitué ou une ou plusieurs fois substitué ; un groupe cycloalkyle en $C_3$-$C_8$ respectivement non substitué ou une ou plusieurs fois substitué ; un groupe aryle ou hétéroaryle, chacun non substitué ou une ou plusieurs fois substitué ; un groupe aryle, hétéroaryle ou cycloalkyle en $C_3$-$C_8$, chacun non substitué ou une ou plusieurs fois substitué, relié par l'intermédiaire d'un groupe alkyle en $C_1$-$C_3$ ;
$R^4$ représente H ; un groupe alkyle en $C_1$- $C_5$, saturé ou insaturé, ramifié ou non ramifié, non substitué ou une ou plusieurs fois substitué ; un groupe aryle ou hétéroaryle, chacun substitué ou non substitué ; un groupe aryle, hétéroaryle ou cycloalkyle, chacun non substitué ou une ou plusieurs fois substitué, relié par l'intermédiaire d'un groupe alkyle en $C_1$- $C_3$ ; $COR^{12}$ ; $SO_2R^{12}$,

$R^{12}$ représentant H ; un groupe alkyle en $C_1$- $C_5$ respectivement saturé ou insaturé, ramifié ou non ramifié, non substitué ou une ou plusieurs fois substitué ; un groupe cycloalkyle en $C_3$- $C_8$ respectivement saturé ou insaturé, non substitué ou une ou plusieurs fois substitué ; un groupe aryle ou hétéroaryle, chacun non substitué ou une ou plusieurs fois substitué ; ou un groupe aryle, cycloalkyle en $C_3$- $C_8$ ou hétéroaryle, chacun non substitué ou une ou plusieurs fois substitué, relié par l'intermédiaire d'un groupe alkyle en $C_1$-$C_3$ : $OR^{13}$ ; $NR^{19}R^{15}$;

$R^5$ représente =O ; H ; $COOR^{13}$, $CONR^{13}$, $OR^{13}$ ; un groupe alkyle en $C_1$-$C_5$ saturé ou insaturé, ramifié ou non ramifié, non substitué ou une ou plusieurs fois substitué ; un groupe cycloalkyle en $C_3$-$C_8$ saturé ou insaturé, non substitué ou une ou plusieurs fois substitué ; un groupe aryle ou hétéroaryle, non substitué ou une ou

plusieurs fois substitué ; ou un groupe aryle, cycloalkyle en $C_3$-$C_8$ ou hétéroaryle, non substitué ou une ou plusieurs fois substitué, relié par l'intermédiaire d'un groupe alkyle en $C_1$-$C_3$ ;
$R^6$ représente H ; F, Cl, $NO_2$, $CF_3$, $OR^{13}$, $SR^{13}$, $SO_2R^{13}$, $SO_2OR^{13}$, CN, $COOR^{13}$, $NR^{14}R^{15}$ ; un groupe alkyle en $C_1$-$C_5$ saturé ou insaturé, ramifié ou non ramifié, non substitué ou une ou plusieurs fois substitué ; un groupe cycloalkyle en $C_3$-$C_8$ saturé ou insaturé, non substitué ou une ou plusieurs fois substitué ; un groupe aryle ou hétéroaryle, non substitué ou une ou plusieurs fois substitué ; ou un groupe aryle, cycloalkyle en $C_3$-$C_8$ ou hétéroaryle, non substitué ou une ou plusieurs fois substitué, relié par l'intermédiaire d'un groupe alkyle en $C_1$-$C_3$ ;
ou $R^5$ et $R^6$ forment ensemble un groupe $(CH_2)_n$, où n = 2, 3, 4, 5 ou 6, des atomes d'hydrogène individuels pouvant être remplacés par F, Cl, Br, I, $NO_2$, $CF_3$, $OR^{13}$, CN ou alkyle en $C_1$-$C_5$ ;
$R^7$, $R^8$, $R^9$ et $R^{10}$ représentent indépendamment les uns des autres
H, F, Cl, Br, I, $NO_2$, $CF_3$, $OR^{13}$, $SR^{13}$, $SO_2R^{13}$, $SO_2OR^{13}$, CN, $COOR^{13}$, $NR^{14}R^{15}$; un groupe alkyle en $C_1$- $C_5$, un groupe cycloalkyle en $C_3$- $C_8$ non substitué ou une ou plusieurs fois substitué ; un groupe aryle ou hétéroaryle, non substitué ou une ou plusieurs fois substitué ; ou un groupe aryle, cycloalkyle en $C_3$- $C_8$ ou hétéroaryle, non substitué ou une ou plusieurs fois substitué, relié par l'intermédiaire d'un groupe alkyle en $C_1$- $C_3$ ;

$R^{13}$ représentant H ; un groupe alkyle en $C_1$- $C_5$ respectivement saturé ou insaturé, ramifié ou non ramifié, non substitué ou une ou plusieurs fois substitué ; un groupe cycloalkyle en $C_3$- $C_8$ respectivement saturé ou insaturé, non substitué ou une ou plusieurs fois substitué ; un groupe aryle ou hétéroaryle, non substitué ou une ou plusieurs fois substitué ; ou un groupe aryle, cycloalkyle en $C_3$- $C_8$ ou hétéroaryle, non substitué ou une ou plusieurs fois substitué, relié par l'intermédiaire d'un groupe alkyle en $C_1$- $C_3$ ;

$R^{14}$ et $R^{15}$ représentent, indépendamment l'un de l'autre, H ; un groupe alkyle en $C_1$-$C_5$ dans chaque cas saturé ou insaturé, ramifié ou non ramifié, non substitué ou une ou plusieurs fois substitué ; ou un groupe cycloalkyle en $C_3$-$C_8$ chaque fois saturé ou insaturé, non substitué ou une ou plusieurs fois substitué ; un groupe aryle ou hétéroaryle, non substitué ou une ou plusieurs fois substitué ; ou un groupe aryle, cycloalkyle en $C_3$-$C_8$ ou hétéroaryle, non substitué ou une ou plusieurs fois substitué, relié par l'intermédiaire d'un groupe alkyle en $C_1$-$C_3$ ;
ou les radicaux $R^{14}$ et $R^{15}$ représentent ensemble un groupe $CH_2CH_2OCH_2CH_2$, $CH_2CH_2NR^{16}CH_2CH_2$ ou $(CH_2)_{3-6}$

$R^{16}$ représentant H ; un groupe alkyle en $C_1$- $C_5$ saturé ou insaturé, ramifié ou non ramifié, non substitué ou une ou plusieurs fois substitué ;

$R^{17}$ représente H ; un groupe alkyle en $C_1$-$C_8$, saturé ou insaturé, ramifié ou non ramifié ; $COR^{12}$ ou $SO_2R^{12}$ sous forme du racémate ; des énantiomères, des diastéréoisomères, de mélanges des énantiomères ou diastéréoisomères ou d'un énantiomère ou diastéréoisomère individuel ; des bases et/ou des sels avec des acides physiologiquement acceptables.

**2.** Dérivés d'azaindole spirocycliques selon la revendication 1,
dans lesquels
lesdits radicaux alkyle en $C_1$- $C_8$, alkyle en $C_1$- $C_5$, alkyle en $C_1$- $C_3$ ou alkylène en $C_1$- $C_3$ et cycloalkyle en $C_3$- $C_8$ peuvent être substitués chacun une ou plusieurs fois par F, Cl, Br, I, CN, $NH_2$, NH- alkyle ($C_1$- $C_6$), NH- alkyl ($C_1$- $C_6$)- OH, N [alkyle ($C_1$- $C_6$) $]_2$, N [alkyl [($C_1$- $C_6$)- $OH_2$, $NO_2$, SH, S- alkyle ($C_1$- $C_6$), OH, O- alkyle ($C_1$- $C_6$), O- alkyl ($C_1$- $C_6$)- OH, C (=O)- alkyle ($C_1$- $C_6$), $CO_2H$, $CO_2$- alkyle ($C_1$- $C_6$), $CF_3$, $OCF_3$, alkyle en $C_1$- $C_6$,
lesdits radicaux aryle ou hétéroaryle peuvent être substitués chacun une ou plusieurs fois par F, Cl, Br, I, CN, $NH_2$, NH- alkyle ($C_1$- $C_6$), NH- alkyl ($C_1$- $C_6$)- OH, N [alkyle ($C_1$- $C_6$) $]_2$, N [alkyl ($C_1$- $C_6$)- OH$]_2$, $NO_2$, SH, S- alkyle ($C_1$- $C_6$), OH, O- alkyle ($C_1$- $C_6$), O- alkyl ($C_1$- $C_6$)- OH, C (=O)- alkyle ($C_1$- $C_6$), $CO_2H$, $CO_2$- alkyle ($C_1$- $C_6$), $CF_3$, $OCF_3$, alkyle en $C_1$- $C_6$,

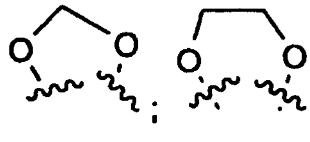

ou phénoxy.

**3.** Dérivés d'azaindole spirocycliques selon la revendication 1 ou 2, dans lesquels $R^1$ et $R^2$ représentent indépendamment l'un de l'autre H ; un groupe alkyle en $C_1$- $C_5$ saturé ou insaturé, ramifié ou non ramifié, non substitué ou une ou plusieurs fois substitué ; ou les radicaux $R^1$ et $R^2$ forment ensemble un cycle et représentent $CH_2CH_2OCH_2CH_2$, $CH_2CH_2NR^{11}CH_2CH_2$ ou $(CH_2)_{3-6}$;

$R^{11}$ représentant H ; un groupe alkyle en $C_1$- $C_5$ saturé ou insaturé, ramifié ou non ramifié, non substitué ou une ou plusieurs fois substitué.

**4.** Dérivés d'azaindole spirocycliques selon la revendication 3, dans lesquels $R^1$ et $R^2$ représentent $CH_3$.

**5.** Dérivés d'azaindole spirocycliques selon la revendication 1 ou 2, dans lesquels $R^3$ représente le groupe propyle, butyle, pentyle, hexyle, phényle, furyle, thiophényle, naphtyle, benzyle, benzofuranyle, indolyle, indanyle, benzodioxanyle, benzodioxolanyle, pyridyle, pyrimidyle, pyrazinyle, triazolyle ou benzothiophényle, chacun non substitué ou une ou plusieurs fois substitué ; un radical phényle, furyle ou thiophényle, chacun non substitué ou une ou plusieurs fois substitué, relié par l'intermédiaire d'un groupe alkyle en $C_1$-$C_3$ saturé, non ramifié.

**6.** Dérivés d'azaindole spirocycliques selon la revendication 5, dans lesquels $R^3$ représente le groupe butyle, éthyle, 3- méthoxypropyle, benzothiophényle, phényle, 3- méthylphényle, 3- fluorophényle, benzo [1, 3]- dioxolyle, benzyle, 1- méthyl- 1, 2, 4- triazolyle, thiényle ou phénéthyle.

**7.** Dérivés d'azaindole spirocycliques selon la revendication 1 ou 2, dans lesquels $R^5$ représente H, un groupe alkyle en $C_1$-$C_5$ ramifié ou non ramifié, non substitué ou une ou plusieurs fois substitué, ou $COOR^{13}$.

**8.** Dérivés d'azaindole spirocycliques selon la revendication 7, dans lesquels $R^5$ représente H.

**9.** Dérivés d'azaindole spirocycliques selon la revendication 1 ou 2, dans lesquels $R^6$ représente H, un groupe alkyle en $C_1$-$C_5$, aryle ou aryle relié par l'intermédiaire d'un groupe alkyle en $C_1$-$C_3$.

**10.** Dérivés d'azaindole spirocycliques selon la revendication 9, dans lesquels $R^6$ représente H.

**11.** Dérivés d'azaindole spirocycliques selon la revendication 1, dans lesquels $R^7$, $R^8$, $R^9$ et $R^{10}$ représentent indépendamment les uns des autres H ; un groupe méthyle, éthyle, propyle, butyle ; pyridyle, O-benzyle, F, Cl, Br, I, $CF_3$, OH, $OCH_3$, $NH_2$, COOH, $COOCH_3$, $NHCH_3$ ou $N(CH_3)_2$ ou $NO_2$.

**12.** Dérivés d'azaindole spirocycliques selon la revendication 11, dans lesquels $R^7$, $R^8$, $R^9$ et $R^{10}$ représentent H.

**13.** Composés selon l'une quelconque des revendications 1 à 12, dans lesquels la formule générale I a la signification de l'une des formules générales **Ia** ou **Ib**

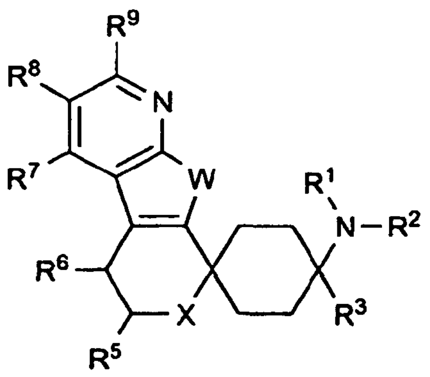

Ia

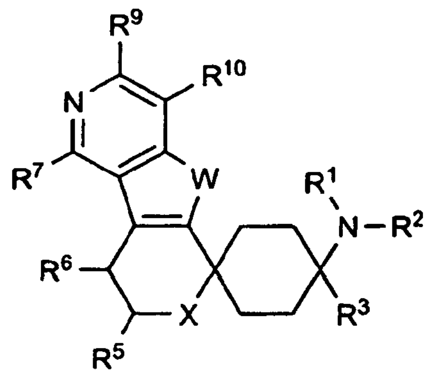

Ib

et les groupes X et W ainsi que les radicaux $R^1$-$R^{10}$ ont les significations indiquées dans les revendications 1 à 12.

**14.** Dérivés d'azaindole spirocycliques selon la revendication 1, choisis dans le groupe

(1) 4- (diméthylamino)- 4- phényl- spiro [cyclohexane- 1, 8'- (5, 6, 8, 9- tétrahydro- pyrano [3, 4- b]- 7- azaindole) ]
(2) 4- (diméthylamino)- 4- phényl- spiro [cyclohexane- 1, 8'- (5, 6, 8, 9- tétrahydro- pyrano [3, 4- b]- 7- azaindole) ] ; méthanesulfonate
(3) 4- (diméthylamino)- 4- phényl- spiro [cyclohexane- 1, 6'- (5, 6, 8, 9- tétrahydro- pyrano [3, 4- b]- 5- azaindole) ]
(4) 4- (diméthylamino)- 4- thiophén- 2- yl- spiro- [cyclohexane- 1, 8'- (5, 6, 8, 9- tétrahydro- pyrano [3, 4- b]- 7- azaindole) ] ; méthanesulfonate
(5) 4- (diméthylamino)- 4- thiophén- 2- yl- spiro- [cyclohexane- 1, 6'- (5, 6, 8, 9- tétrahydro- pyrano [3, 4- b]- 5- azaindole) ]
(6) 4- (diméthylamino)- 4- phényl- spiro [cyclohexane- 1, 8'- (5, 6, 8, 9- tétrahydro- pyrano [3, 4- b]- 7- azaindole) ] ; citrate (4: 3)
(7) 4- (méthylamino)- 4- phényl- spiro [cyclohexane- 1, 8'- (5, 6, 8, 9- tétrahydro- pyrano [3, 4- b]- 7- azaindole) ] ; citrate (1: 1)
(8) 4- (méthylamino)- 4- thiophén- 2- yl- spiro [cyclohexane- 1, 8'- (5, 6, 8, 9- tétrahydro- pyrano [3, 4- b]- 7- azaindole) ] ; citrate (4: 3)
(9) 4- (diméthylamino)- 4- benzo [1, 3- dioxol]- spiro- [cyclohexane- 1, 8'- (5, 6, 8, 9- tétrahydro- pyrano [3, 4- b]- 7- azaindole) ] ; citrate (1: 1)
(10) 4- (diméthylamino)- 4- (benzothiophén- 2- yl)- spiro- [cyclohexane- 1, 8'- (5, 6, 8, 9- tétrahydro- pyrano [3, 4- b]- 7- azaindole) ] ; citrate (1: 1)
(12) 4- (diméthylamino)- 4- (3- fluorophényl)- spiro [cyclohexane- 1, 8'- (5, 6, 8, 9- tétrahydro- pyrano [3, 4- 1a- 7- azaindole) ] ; citrate (4: 3)
(13) 4- (diméthylamino)- 4- (3- méthylphényl)- spiro [cyclohexane- 1, 8'- (5, 6, 8, 9- tétrahydro- pyrano [3, 4- b]- 7- azaindole) ] ; citrate (1: 1)
(14) 4- (diméthylamino)- 4- (but- 1- yl)- spiro [cyclohexane- 1, 8'- (5, 6, 8, 9- tétrahydro- pyrano [3, 4- b]- 7- azaindole) ] ; citrate (1: 1)
(15) 4- (diméthylamino)- 4- phényléthyl- spiro [cyclohexane- 1, 8'- (3- trifluorométhyl- 5, 6, 8, 9- tétrahydro- pyrano [3, 4- b]- 7- azaindole) ] ; citrate (1: 1)
(17) 4- (diméthylamino)- 4- éthyl- spiro [cyclohexane- 1, 8'- 5, 6, 8, 9- tétrahydro- pyrano [3, 4- b]- 7- azaindole) ] ; citrate (2: 3)
(18) 4- (diméthylamino)- 4- phényléthyl- spiro [cyclohexane- 1, 8'- (5, 6, 8, 9- tétrahydro- pyrano [3, 4- b]- 7- azaindole) ] ; citrate (2: 3)
(19) 4- benzyl- 4- morpholino- spiro [cyclohexane- 1, 8'- (5, 6, 8, 9- tétrahydro- pyrano [3, 4- b]- 7- azaindole) ] ; citrate (1: 1)
(20) 4- (diméthylamino)- 4- (3- méthoxypropyl)- spiro- [cyclohexane- 1, 8'- (5, 6, 8, 9- tétrahydro- pyrano [3, 4- b]- 7- azaindole) ] ; citrate (1: 1)
(21) 4- (diméthylamino)- 4- phényl- spiro [cyclohexane- 1, 8'- (5, 6, 8, 9- tétrahydro- pyrano [3, 4- b]- 7- azaindole) ] ; citrate (1: 1)
(22) 4- (diméthylamino)- 4- thiophén- 2- yl- spiro- [cyclohexane- 1, 8'- (5, 6, 8, 9- tétrahydro- pyrano [3, 4- b]- 7- azaindole) ] ; citrate (1: 1)
(23) 4- (diméthylamino)- 4- phényl- spiro [cyclohexane- 1, 6'- (5, 6, 8, 9- tétrahydro- pyrano [3, 4- b]- 5- azaindole) ] ; citrate (1: 1)
(24) 4- (diméthylamino)- 4- phényl- spiro [cyclohexane- 1, 6'- (5, 6, 8, 9- tétrahydro- pyrano [3, 4- b]- 5- azaindole) ] ; citrate (1: 1)
(25) 4- butyl- 4- (diméthylamino)- spiro [cyclohexane- 1, 8'- (5, 6, 8, 9- tétrahydro- pyrano [3, 4- b]- 7- azaindole) ) ; citrate (1: 1)
(26) 4- butyl- 4- (diméthylamino)- spiro [cyclohexane- 1, 8'- (5, 6, 8, 9- tétrahydro- pyrano [3, 4- b) ]- 7- azaindole) ] ; citrate (1: 1)
(27) 4- benzyl- 4- morpholino- spiro [cyclohexane- 1, 8'- (3- trifluorométhyl- 5, 6, 8, 9- tétrahydro- pyrano [3, 4- b]- 7- aza- indole) ] ; citrate (1: 1)
(28) 4- (diméthylamino)- 4- phényl- spiro [cyclohexane- 1, 8'- (3- trifluorométhyl- 5, 6, 8, 9- tétrahydro- pyrano [3, 4- b]- 7- azaindole) ] ; citrate (1: 1)
(29) 4- (azétidin- 1- yl)- 4- phényl- spiro [cyclohexane- 1, 8'- 5, 6, 8, 9- tétrahydro- pyrano [3, 4- b]- 7- azaindole) ] ; citrate (1: 1)
(30) 4- butyl- 4- (pyrrolidin- 1- yl)- spiro [cyclohexane- 1, 8'- (5, 6, 8, 9- tétrahydro- pyrano [3, 4- b]- 7- azaindole) ] ; citrate (2: 1)
(31) 4- (diméthylamino)- 4- phényl- spiro [cyclohexane- 1, 8'- (5, 6, 8, 9- tétrahydro- thiopyrano [3, 4- b)- 7- azaindole) ] ; citrate (1: 1)
(32) 4- (diméthylamino)- 4- phényl- spiro [cyclohexane- 1, 6'- (5, 6, 8, 9- tétrahydrothiopyrano [3, 4- b]- 5- azaindole) ] citrate (1: 1)

(33) 4- (1- méthyl- 1H- 1, 2, 4- triazol- 5- yl)- 4- (méthylamino)- spiro [cyclohexane- 1, 8'- (5, 6, 8, 9- tétrahydro-pyrano [3, 4- b]- 7- aza- indole) ], citrate (1: 1)
(34) 4- (1- méthyl- 1H- 1, 2, 4- triazol- 5- yl)- 4- (méthylamino)- spiro [cyclohexane- 1, 8'- (5, 6, 8, 9- tétrahydro-pyrano [3, 4- b]- 7- azaindole) ], citrate (1: 1)

sous forme du racémate ; des énantiomères, des diastéréoisomères, de mélanges des énantiomères ou diastéréoisomères ou d'un énantiomère ou diastéréoisomère individuel ; des bases et/ou des sels avec des acides physiologiquement acceptables.

**15.** Procédé pour la préparation de dérivés d'azaindole spirocycliques de formule générale I, par mise en réaction de composés de formule générale III dans un milieu réactionnel en présence de fluorure ou en présence d'un acide organique ou inorganique, de préférence HCl, acide acétique, acide trifluoroacétique, trifluorure de bore, à des températures de préférence de -70°C à 300 °C, pour l'obtention de composés de formule générale IV,

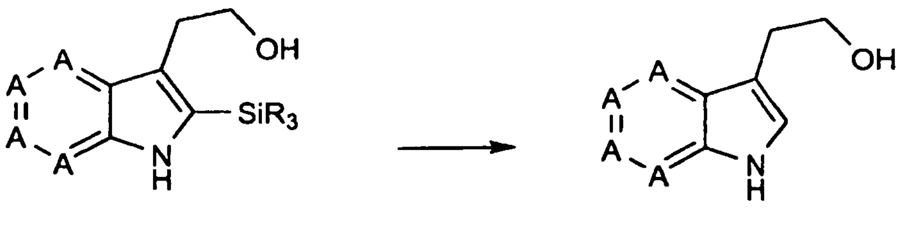

III IV

qu'on fait ensuite réagir, en ajoutant au moins un acide organique ou son ester de triméthylsilyle ou un acide inorganique ou en ajoutant au moins un sel d'un métal de transition, avec des cétones de formule générale VII,

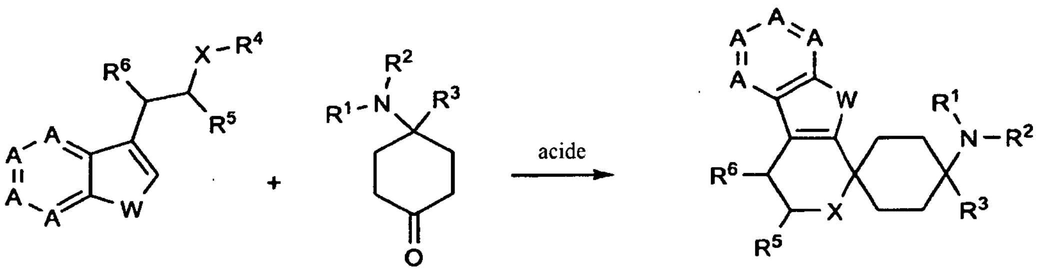


IV VII I

dans un solvant ou mélange de solvants convenable, comme par exemple le dichlorométhane, le dichloréthane, le chloroforme, l'acétonitrile, l'oxyde d'éthyle ou le nitroéthane, à des températures de 0 à 150 °C, en utilisant des micro-ondes.

**16.** Médicament contenant au moins un dérivé d'azaindole substitué selon la revendication 1, éventuellement sous forme de son racémate, des stéréoisomères purs, en particulier énantiomères et diastéréoisomères, en un rapport de mélange quelconque ; sous forme de ses acides ou de ses bases ou sous forme de ses sels, en particulier des sels physiologiquement acceptables ou des sels avec des cations ou acides physiologiquement acceptables ; ou sous forme de ses produits de solvatation, en particulier des hydrates ; ainsi qu'éventuellement contenant des additifs et/ou adjuvants convenables et/ou éventuellement d'autres substances actives.

**17.** Utilisation d'un dérivé d'azaindole substitué selon la revendication 1, éventuellement sous forme de son racémate, des stéréoisomères purs, en particulier énantiomères et diastéréoisomères, en un rapport de mélange quelconque ; sous forme des ses acides ou de ses bases ou sous forme de ses sels, en particulier des sels physiologiquement acceptables ou des sels avec des cations ou acides physiologiquement acceptables ; ou sous forme de ses produits de solvatation, en particulier des hydrates ; pour la fabrication d'un médicament destiné au traitement de la douleur, en particulier de la douleur aiguë, névropathique ou chronique.

**18.** Utilisation d'un dérivé d'azaindole substitué selon la revendication 1, pour la fabrication d'un médicament destiné

au traitement d'états anxieux, du stress et de syndromes liés au stress, de dépressions, de l'épilepsie, de la maladie d'Alzheimer, de la démence sénile, de la catalepsie, de dysfonctionnements cognitifs généraux, de troubles de la mémoire et de l'apprentissage (en tant qu'agent nootrope), de manifestations inflammatoires, d'abus d'alcool et/ou de drogues et/ou de médicaments et/ou de la dépendance à ceux-ci, de dysfonctionnements sexuels, de maladies cardiovasculaires, de l'hypotension, de l'hypertension, de l'acouphène, du prurit, de la migraine, de troubles de l'audition, de la motilité intestinale insuffisante, de l'alimentation perturbée, de l'anorexie, de l'adiposité, de troubles locomoteurs, de la diarrhée, de la cachexie, de l'incontinence urinaire ou en tant que myorelaxant, anticonvulsivant ou anesthésique, ou à l'administration conjointe dans le traitement par un analgésique opioïde ou par un anesthésique, à la diurèse ou à l'antinatriurèse, à l'anxiolyse, au traitement de maladies neurodégénératives liées à la modulation de la sécrétion de neurotransmetteurs, au traitement de manifestations inflammatoires et/ou à la réduction du potentiel de dépendance aux opioïdes.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 04043967 A
- WO 0290317 A
- US 4065573 A
- US 4065573 A1, Upjohn_Lednicer


### In der Beschreibung aufgeführte Nicht-Patentliteratur


- Opioids: Introduction. Further Opioid Receptors, 455-476 in: Analgesics - From Chemistry and Pharmacology to Clinical Application. Wiley VCH, 2002, 127-150
- **MANABE et al.** *Nature,* 1997, vol. 394, 577-581
- **NISHI et al.** *EMBO J.,* 1997, vol. 16, 1858-1864
- **VON CALO et al.** *Br.J. Pharmacol.,* 2000, vol. 129, 1261-1283
- **LEDNICER et al.** *J.Med.Chem.,* 1980, vol. 23, 424-430
- **SCHNEIDER ; WOLDEMAR ; KROMBHOLZ ; GOTTFRIED ; ARPMAS.** *Arch.Pharm.(Weinheim Ger.,* 1980, vol. 313 (6), 487-498
- **LETSINGER ; SCHNIZER.** *J. Org. Chem.,* 1951, vol. 16, 704, 706
- **J. A. TURNER.** *J. Org. Chem.,* 1983, vol. 48, 3401
- **L. ESTEL ; F. MARSAIS ; G. QUÉGUINER.** *J. Org. Chem.,* 1988, vol. 53, 2740
- **J. MALM ; B. REHN ; A.-B. HÖRNFELDT ; S. GRONOWITZ.** *J. Het. Chem.,* 1994, vol. 31, 11
- **B. C. BISHOP ; I.F. COTTRELL ; D. HANDS.** *Synthesis,* 1997, 1315
- **C. CHENG ; D. R. LIEBERMAN ; R. D. LARSEN ; R. A. REAMER ; T. R. VERHOEVEN ; P. J. REIDER.** *Tet. Lett.,* 1994, 6981
- **F. UJJAINWALLA ; D. WARNER.** *Tet. Lett.,* 1998, 5355
- **VON ARDATI et al.** *Mol. Pharmacol.,* 1997, vol. 51, 816-824
- **VON D'AMOUR ; SMITH.** *J. Pharm. Exp. Ther.,* 1941, vol. 72, 74-79